# EUROPEAN PATENT SPECIFICATION

(11) **EP 4 311 850 B1**
(45) Date of publication and mention of the grant of the patent: **04.12.2024**
(21) Application number: 23187817.4
(22) Date of filing: 26.07.2023
(51) Int. Cl.: C09K 11/06, H01L 33/26, H10K 50/00, C07D 247/00, C07D 405/04, C07D 409/04, C07D 471/04, C07F 15/00, H10K 101/10, H10K 85/30

(54) **ORGANOMETALLIC COMPOUND, ORGANIC LIGHT-EMITTING DEVICE INCLUDING THE SAME, AND ELECTRONIC APPARATUS INCLUDING THE ORGANIC LIGHT-EMITTING DEVICE**
ORGANOMETALLISCHE VERBINDUNG, ORGANISCHE LICHTEMITTIERENDE VORRICHTUNG DAMIT UND ELEKTRONISCHE VORRICHTUNG MIT DER ORGANISCHEN LICHTEMITTIERENDEN VORRICHTUNG
COMPOSÉ ORGANOMÉTALLIQUE, DISPOSITIF ÉLECTROLUMINESCENT ORGANIQUE LE COMPRENANT ET APPAREIL ÉLECTRONIQUE COMPRENANT LE DISPOSITIF ÉLECTROLUMINESCENT ORGANIQUE

(30) Priority: 29.07.2022 KR 20220095043
(43) Date of publication of application: 31.01.2024
(73) Proprietor: Samsung Electronics Co., Ltd., Suwon-si, Gyeonggi-do 16677 (KR)
(72) Inventor: LEE, Banglin, 16678 Suwon-si (KR); KANG, Byungjoon, 16678 Suwon-si (KR); KIM, Sungmin, 16678 Suwon-si (KR); LEE, Kum Hee, 16678 Suwon-si (KR); LEE, Yong Joo, 16678 Suwon-si (KR); CHO, Hwayoung, 16678 Suwon-si (KR); CHOI, Byoungki, 16678 Suwon-si (KR); HWANG, Kyuyoung, 16678 Suwon-si (KR)
(74) Representative: Elkington and Fife LLP

(56) References cited:
- EP-A1- 3 623 443
- EP-A2- 3 444 258
- US-A1- 2010 219 397
- US-A1- 2010 270 916
- US-A1- 2012 228 583

## Description

### FIELD OF THE INVENTION

One or more embodiments relate to an organometallic compound, an organic light-emitting device including the same, and an electronic apparatus including the organic light-emitting device.

### BACKGROUND OF THE INVENTION

Organic light-emitting devices (OLEDs) are self-emissive devices, which have improved characteristics in terms of viewing angles, response time, brightness, driving voltage, and response speed. In addition, OLEDs can produce full-color images.

In a typical example, an organic light-emitting device includes an anode, a cathode, and an organic layer located between the anode and the cathode, wherein the organic layer includes an emission layer. A hole transport region may be located between the anode and the emission layer, and an electron transport region may be located between the emission layer and the cathode. Holes provided from the anode may move toward the emission layer through the hole transport region, and electrons provided from the cathode may move toward the emission layer through the electron transport region. The holes and the electrons may recombine in the emission layer to produce excitons. The excitons may transition from an excited state to a ground state, thus generating light. US2012/228583A1 (claim 16, compound 11) discloses an organometallic compound similar to Formula 1 of the invention.

### SUMMARY OF THE INVENTION

Provided are an organometallic compound, an organic light-emitting device including the same, and an electronic apparatus including the organic light-emitting device.

Additional aspects will be set forth in part in the detailed description that follows and, in part, will be apparent from the detailed description, or may be learned by practice of the presented exemplary embodiments.

According to an aspect, an organometallic compound represented by Formula 1 is provided:

Formula 1 M(L₁)₃

wherein, in Formula 1,
   M is iridium,
   L₁ is a ligand represented by Formula 2-1 or a ligand represented by Formula 2-2, 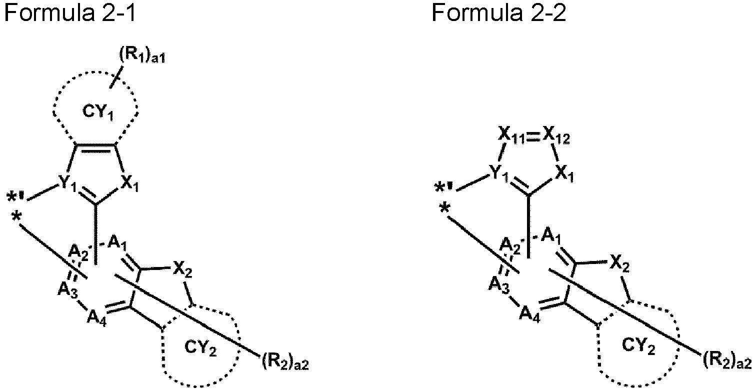
wherein, in Formulae 2-1 and 2-2,
   Y₁ is N,
   X₁₁ is C(R₁₁) or N, and X₁₂ may be C(R₁₂) or N,
   X₁ is O, S, or N-{(T₁)_{b1}-(Z₁)_{c1}},
   T₁ is a single bond, a C₁-C₂₀ alkylene group that is unsubstituted or substituted with at least one R₁₀ₐ, a C₅-C₃₀ carbocyclic group that is unsubstituted or substituted with at least one R₁₀ₐ, or a C₁-C₃₀ heterocyclic group that is unsubstituted or substituted with at least one R₁₀ₐ,
   b1 is an integer from 1 to 5,
   c1 is an integer from 0 to 20,
   A₁ to A₄ may each independently C or N, provided that one of A₁ to A₄ is C bonded to an adjacent 5-membered ring, and another of A₁ to A₄ is C bonded to M in Formula 1,
   X₂ is O, S, Se, Si(R₂₉ₐ)(R_{29b}), or Ge(R₂₉ₐ)(R_{29b}),
   ring CY₁ and ring CY₂ are each independently a C₅-C₃₀ carbocyclic group or a C₁-C₃₀ heterocyclic group,
   R₁, R₁₁, R₁₂, R₂, R₂₉ₐ, R_{29b}, and Z₁ are each independently hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₇-C₆₀ alkyl aryl group, a substituted or unsubstituted C₇-C₆₀ aryl alkyl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₂-C₆₀ alkyl heteroaryl group, a substituted or unsubstituted C₂-C₆₀ heteroaryl alkyl group, a substituted or unsubstituted C₁-C₆₀ heteroaryloxy group, a substituted or unsubstituted C₁-C₆₀ heteroarylthio group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), - B(Q₆)(Q₇), -P(=O)(Q₃)(Q₉), or -P(Q₈)(Q₉),
   a1 and a2 are each independently an integer from 0 to 20,
   two or more of a plurality of R₁ are optionally linked to each other to form a C₅-C₃₀ carbocyclic group that is unsubstituted or substituted with at least one R₁₀ₐ, or a C₁-C₃₀ heterocyclic group that is unsubstituted or substituted with at least one R₁₀ₐ,
   two or more of a plurality of R₂ are optionally linked to each other to form a C₅-C₃₀ carbocyclic group that is unsubstituted or substituted with at least one R₁₀ₐ, or a C₁-C₃₀ heterocyclic group that is unsubstituted or substituted with at least one R₁₀ₐ,
   R₁ and R₂ are optionally linked to each other to form a C₅-C₃₀ carbocyclic group that is unsubstituted or substituted with at least one R₁₀ₐ, or a C₁-C₃₀ heterocyclic group that is unsubstituted or substituted with at least one R₁₀ₐ,
   R₁₀ₐ is as described in connection with R₂,
   * and *' each indicates a binding site to M in Formula 1,
   at least one substituent of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₁-C₆₀ alkylthio group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₇-C₆₀ alkyl aryl group, the substituted C₇-C₆₀ aryl alkyl group, the substituted C₆-C₆₀ aryloxy group, the substituted C₆-C₆₀ arylthio group, the substituted C₁-C₆₀ heteroaryl group, the substituted C₂-C₆₀ alkyl heteroaryl group, the substituted C₂-C₆₀ heteroaryl alkyl group, the substituted C₁-C₆₀ heteroaryloxy group, the substituted C₁-C₆₀ heteroarylthio group, the substituted monovalent non-aromatic condensed polycyclic group, and the substituted monovalent non-aromatic condensed heteropolycyclic group is:
      deuterium, -F, -Cl, -Br, -I, -SF₅, -CD₃, -CD₂H, -CDH₂, -CF₃, -CF₂H, - CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, or a C₁-C₆₀ alkylthio group,
      a C₁-C₆₀ alkyl group, a C₂-C₆₀ alkenyl group, a C₂-C₆₀ alkynyl group, a C₁-C₆₀ alkoxy group, or a C₁-C₆₀ alkylthio group, each substituted with at least one of deuterium, -F, -Cl, -Br, -I, -SF₅, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇-C₆₀ alkyl aryl group, a C₆-C₆₀ aryloxy group, a C₆-C₆₀ arylthio group, a C₁-C₆₀ heteroaryl group, a C₂-C₆₀ alkyl heteroaryl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), -Ge(Q₁₃)(Q₁₄)(Q₁₅), -B(Q₁₆)(Q₁₇), - P(=O)(Q₁₈)(Q₁₉), -P(Q₁₈)(Q₁₉), or a combination thereof,
      a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇-C₆₀ alkyl aryl group, a C₆-C₆₀ aryloxy group, a C₆-C₆₀ arylthio group, a C₁-C₆₀ heteroaryl group, a C₂-C₆₀ alkyl heteroaryl group, a C₁-C₆₀ heteroaryloxy group, a C₁-C₆₀ heteroarylthio group, a monovalent non-aromatic condensed polycyclic group, or a monovalent non-aromatic condensed heteropolycyclic group, each unsubstituted or substituted with at least one of deuterium, -F, -Cl, -Br, -I, -SF₅, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₁-C₆₀ alkylthio group, C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇-C₆₀ alkyl aryl group, a C₇-C₆₀ aryl alkyl group, a C₆-C₆₀ aryloxy group, a C₆-C₆₀ arylthio group, a C₁-C₆₀ heteroaryl group, a C₂-C₆₀ alkyl heteroaryl group, a C₂-C₆₀ heteroaryl alkyl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), - Ge(Q₂₃)(Q₂₄)(Q₂₅), -B(Q₂₆)(Q₂₇), -P(=O)(Q₂₈)(Q₂₉), P(Q₂₈)(Q₂₉), or a combination thereof,
      -N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), - P(=O)(Q₃₈)(Q₃₉), or -P(Q₃₈)(Q₃₉), or
      a combination thereof,
      wherein Q₁ to Q₉, Q₁₁ to Q₁₉, Q₂₁ to Q₂₉, and Q₃₁ to Q₃₉ are each independently hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C ₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₇-C₆₀ alkyl aryl group, a substituted or unsubstituted C₇-C₆₀ aryl alkyl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₂-C₆₀ alkyl heteroaryl group, a substituted or unsubstituted C₂-C₆₀ heteroaryl alkyl group, a substituted or unsubstituted C₁-C₆₀ heteroaryloxy group, a substituted or unsubstituted C₁-C₆₀ heteroarylthio group, a substituted or unsubstituted monovalent non-aromatic condensed polycyclic group, or a substituted or unsubstituted monovalent non-aromatic condensed heteropolycyclic group.

Another aspect provides an organic light-emitting device including a first electrode, a second electrode, and an organic layer located between the first electrode and the second electrode, wherein the organic layer includes an emission layer, and wherein the organic layer further includes at least one of the organometallic compounds.

The organometallic compound may be included in the emission layer, and the organometallic compound included in the emission layer may act as a dopant.

Another aspect provides an electronic apparatus including the organic light-emitting device.

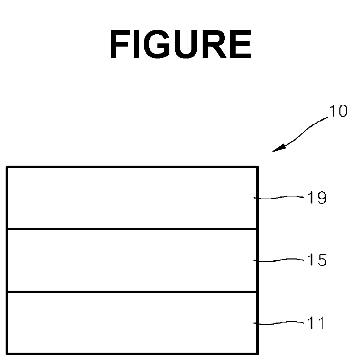

### BRIEF DESCRIPTION OF THE DRAWING

The above and other aspects, features, and advantages of certain exemplary embodiments will be more apparent from the following detailed description taken in conjunction with the FIGURE, which is a schematic cross-sectional view of an organic light-emitting device according to one or more embodiments.

### DETAILED DESCRIPTION OF THE EMBODIMENTS

Reference will now be made in further detail to exemplary embodiments, examples of which are illustrated in the accompanying drawing, wherein like reference numerals refer to like elements throughout. In this regard, the present exemplary embodiments may have different forms and should not be construed as being limited to the detailed descriptions set forth herein. Accordingly, the exemplary embodiments are merely described in further detail, and by referring to the figure, to explain certain aspects. As used herein, the term "and/or" includes any and all combinations of one or more of the associated listed items. Expressions such as "at least one of," when preceding a list of elements, modify the entire list of elements and do not modify the individual elements of the list.

The terminology used herein is for the purpose of describing one or more exemplary embodiments only and is not intended to be limiting. As used herein, the singular forms "a," "an," and "the" are intended to include the plural forms as well, unless the context clearly indicates otherwise. The term "or" means "and/or." It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

It will be understood that, although the terms first, second, third etc. may be used herein to describe various elements, components, regions, layers, and/or sections, these elements, components, regions, layers, and/or sections should not be limited by these terms. These terms are only used to distinguish one element, component, region, layer, or section from another element, component, region, layer, or section. Thus, a first element, component, region, layer, or section discussed below could be termed a second element, component, region, layer, or section without departing from the teachings of the present embodiments.

Exemplary embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims.

It will be understood that when an element is referred to as being "on" another element, it can be directly in contact with the other element or intervening elements may be present therebetween. In contrast, when an element is referred to as being "directly on" another element, there are no intervening elements present.

Unless otherwise defined, all terms (including technical and scientific terms) used herein have the same meaning as commonly understood by one of ordinary skill in the art to which this general inventive concept belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ± 30%, 20%, 10%, 5% of the stated value.

An organometallic compound according to an aspect is represented by Formula 1:

Formula 1 M(L₁)₃

wherein M in Formula 1 is iridium.

In Formula 1, L₁ is a ligand represented by Formula 2-1 or a ligand represented by Formula 2-2: 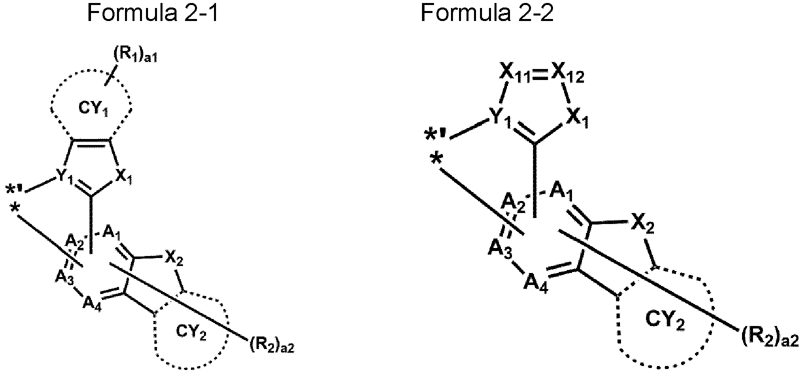 wherein Formulae 2-1 and 2-2 are each as described in further detail herein.

In one or more embodiments, L₁ may be a ligand represented by Formula 2-1.

The three ligands L₁ in Formula 1 may be identical to or different from each other. In one or more embodiments, the organometallic compound represented by Formula 1 may be a homoleptic complex.

In Formulae 2-1 and 2-2, Y₁ is N.

In Formula 2-2, X₁₁ is C(R₁₁) or N, and X₁₂ is C(R₁₂) or N. For example, X₁₁ may be C(R₁₁), and X₁₂ may be C(R₁₂). R₁₁ and R₁₂ are as described herein.

In Formulae 2-1 and 2-2, X₁ is O, S, or N-{(T₁)_{b1}-(Z₁)_{c1}}. For example, X₁ may be N-{(T₁)_{b1}-(Z₁)_{c1}}.

T₁ is a single bond, a C₁-C₂₀ alkylene group that is unsubstituted or substituted with at least one R₁₀ₐ, a C₅-C₃₀ carbocyclic group that is unsubstituted or substituted with at least one R₁₀ₐ, or a C₁-C₃₀ heterocyclic group that is unsubstituted or substituted with at least one R₁₀ₐ.

For example, T₁ may be:
a single bond; or
a C₁-C₂₀ alkylene group, a benzene group, a naphthalene group, an anthracene group, a phenanthrene group, a triphenylene group, a pyrene group, a chrysene group, a cyclopentadiene group, a furan group, a thiophene group, a silole group, an indene group, a fluorene group, an indole group, a carbazole group, a benzofuran group, a dibenzofuran group, a benzothiophene group, a dibenzothiophene group, a benzosilole group, a dibenzosilole group, an azafluorene group, an azacarbazole group, an azadibenzofuran group, an azadibenzothiophene group, an azadibenzosilole group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, or a benzothiadiazole group, each unsubstituted or substituted with at least one R₁₀ₐ.

In one or more embodiments, T₁ may be:
a single bond; or
a C₁-C₂₀ alkylene group or a benzene group, each unsubstituted or substituted with R₁₀ₐ.

In one or more embodiments, T₁ may be:
a single bond; or
a C₁-C₂₀ alkylene group, a benzene group, a naphthalene group, a dibenzofuran group, or a dibenzothiophene group, each unsubstituted or substituted with at least one of deuterium, -F, a cyano group, a C₁-C₂₀ alkyl group, a deuterated C₁-C₂₀ alkyl group, a fluorinated C₁-C₂₀ alkyl group, a C₃-C₁₀ cycloalkyl group, a deuterated C₃-C₁₀ cycloalkyl group, a fluorinated C₃-C₁₀ cycloalkyl group, a (C₁-C₂₀ alkyl)C₃-C₁₀ cycloalkyl group, a phenyl group, a deuterated phenyl group, a fluorinated phenyl group, a (C₁-C₂₀ alkyl)phenyl group, a naphthyl group, a pyridinyl group, a furanyl group, a thiophenyl group, a benzofuranyl group, a benzothiophenyl group, a dibenzofuranyl group, a dibenzothiophenyl group, or a combination thereof.

In the formula N-{(T₁)_{b1}-(Z₁)_{c1}}, b1 represents the number of T₁ groups, and is an integer from 1 to 5. When b1 is 2 or greater, two or more T₁ may be the same as or different from each other. For example, b1 may be 1, 2, or 3.

In the formula N-{(T₁)_{b1}-(Z₁)_{c1}}, c1 represents the number of Z₁ groups, and is an integer from 0 to 20. When c1 is 2 or greater, two or more Z₁ may be the same as or different from each other. For example, c1 may be 0, 1, 2, 3, or 4.

In one or more embodiments, X₁ in Formulae 2-1 and 2-2 may be N-{(T₁)_{b1}-(Z₁)_{c1}}, and T₁ may be a C₁-C₁₀ alkylene group that is unsubstituted or substituted with R₁₀ₐ, a C₅-C₂₀ carbocyclic group that is unsubstituted or substituted with R₁₀ₐ, or a C₁-C₂₀ heterocyclic group that is unsubstituted or substituted with R₁₀ₐ.

A₁ to A₄ in Formula 2-1 and 2-2 are each independently C or N, provided that one of A₁ to A₄ is C bonded to an adjacent 5-membered ring, and another of A₁ to A₄ is a C bonded to M in Formula 1.

For example, in Formulae 2-1 and 2-2,
i) A₁ may be C bonded to an adjacent 5-membered ring, A₂ may be C bonded to M in Formula 1, and A₃ and A₄ may each independently be C or N;
ii) A₂ may be C bonded to an adjacent 5-membered ring, A₃ may be C bonded to M in Formula 1, and A₁ and A₄ may each independently be C or N;
iii) A₃ may be C bonded to an adjacent 5-membered ring, A₄ may be C bonded to M in Formula 1, and A₁ and A₂ may each independently be C or N;
iv) A₂ may be C bonded to an adjacent 5-membered ring, A₁ may be C bonded to M in Formula 1, and A₃ and A₄ may each independently be C or N;
v) A₃ may be C bonded to an adjacent 5-membered ring, A₂ may be C bonded to M in Formula 1, and A₁ and A₄ may each independently be C or N; or
vi) A₄ may be C bonded to an adjacent 5-membered ring, A₃ may be C bonded to M in Formula 1, and A₁ and A₂ may each independently be C or N.

In Formulae 2-1 and 2-2, X₂ is O, S, Se, Si(R₂₉ₐ)(R_{29b}), or Ge(R₂₉ₐ)(R_{29b}). For example, X₂ may be O or S.

In Formulae 2-1 and 2-2, ring CY₁ and ring CY₂ are each independently a C₅-C₃₀ carbocyclic group or a C₁-C₃₀ heterocyclic group.

For example, ring CY₁ and ring CY₂ may each independently be i) a first ring, ii) a second ring, iii) a condensed ring group in which two or more first rings are condensed with each other, iv) a condensed ring group in which two or more second rings are condensed with each other, or v) a condensed ring group in which at least one first ring is condensed with at least one second ring,
the first ring may be a cyclopentane group, a cyclopentadiene group, a furan group, a thiophene group, a pyrrole group, a silole group, a germole group, a borole group, a selenophene group, a phosphole group, an oxazole group, an oxadiazole group, an oxatriazole group, a thiazole group, a thiadiazole group, a thiatriazole group, a pyrazole group, an imidazole group, a triazole group, a tetrazole group, an azasilole group, an azagermole group, an azaborole group, an azaselenophene group, or an azaphosphole group, and
the second ring may be an adamantane group, a norbornane group (a bicyclo[2.2.1]heptane group), a norbornene group, a bicyclo[1.1.1]pentane group, a bicyclo[2.1.1]hexane group, a bicyclo[2.2.2]octane group, a cyclohexane group, a cyclohexene group, a benzene group, a pyridine group, a pyrimidine group, a pyrazine group, a pyridazine group, or a triazine group.

In one or more embodiments, ring CY₁ and ring CY₂ may each independently be a cyclopentane group, a cyclohexane group, a cyclohexene group, a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, a benzocarbazole group, a benzofluorene group, a naphthobenzosilole group, a naphthobenzothiophene group, a naphthobenzofuran group, a naphthobenzoselenophene group, a dibenzocarbazole group, a dibenzofluorene group, a dinaphthosilole group, a dinaphthothiophene group, a dinaphthofuran group, a dinaphthoselenophene group, a naphthocarbazole group, a naphthofluorene group, a phenanthrobenzosilole group, a phenanthrobenzothiophene group, a phenanthrobenzofuran group, a phenanthrobenzoselenophene group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, an azacarbazole group, an azafluorene group, an azadibenzosilole group, an azadibenzothiophene group, an azadibenzofuran group, an azadibenzoselenophene group, an azabenzocarbazole group, an azabenzofluorene group, an azanaphthobenzosilole group, an azanaphthobenzothiophene group, an azanaphthobenzofuran group, an azanaphthobenzoselenophene group, an azadibenzocarbazole group, an azadibenzofluorene group, an azadinaphthosilole group, an azadinaphthothiophene group, an azadinaphthofuran group, an azadinaphthoselenophene group, an azanaphthocarbazole group, an azanaphthofluorene group, an azaphenanthrobenzosilole group, an azaphenanthrobenzothiophene group, an azaphenanthrobenzofuran group, an azaphenanthrobenzoselenophene group, a pyridine group, a pyrimidine group, a pyrazine group, a pyridazine group, a triazine group, a quinoline group, an isoquinoline group, a quinoxaline group, a quinazoline group, a phenanthridine group, a phenanthroline group, a benzoquinoline group, a benzoisoquinoline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, a 5,6,7,8-tetrahydroquinoline group, an adamantane group, a norbornane group, a norbornene group, a benzene group to which an adamantane group is condensed, a benzene group to which a norbornane group is condensed, a benzene group to which a norbornene group is condensed, a pyridine group to which an adamantane group is condensed, a pyridine group to which a norbornane group is condensed, or a pyridine group to which a norbornene group is condensed.

In one or more embodiments, ring CY₁ may be a benzene group, a naphthalene group, an anthracene group, a phenanthrene group, a pyridine group, a pyrimidine group, a pyridazine group, a pyrazine group, a quinoline group, an isoquinoline group, a quinoxaline group, a quinazoline group, a phenanthridine group, a phenanthroline group, a benzoquinoline group, or a benzoisoquinoline group.

In one or more embodiments, ring CY₂ may be a benzene group, a naphthalene group, an anthracene group, a phenanthrene group, a pyridine group, a pyrimidine group, a pyridazine group, a pyrazine group, a quinoline group, an isoquinoline group, a quinoxaline group, a quinazoline group, a phenanthridine group, a phenanthroline group, a benzoquinoline group, a benzoisoquinoline group, a benzene group to which a cyclohexane group is condensed, a benzene group to which an adamantane group is condensed, a benzene group to which a norbornane group is condensed, a pyridine group to which a cyclohexane group is condensed, a pyridine group to which an adamantane group is condensed, a pyridine group to which a norbornane group is condensed, a pyrrole group, a cyclopentadiene group, a silole group, a thiophene group, a furan group, a selenophene group, an indole group, an indene group, a benzosilole group, a benzothiophene group, a benzofuran group, a benzoselenophene group, a carbazole group, a fluorene group, a dibenzosilole group, a dibenzothiophene group, a dibenzofuran group, a dibenzoselenophene group, an azaindole group, an azaindene group, an azabenzosilole group, an azabenzothiophene group, an azabenzofuran group, an azabenzoselenophene group, an azacarbazole group, an azafluorene group, an azadibenzosilole group, an azadibenzothiophene group, an azadibenzofuran group, or an azadibenzoselenophene group.

In one or more embodiments, ring CY₁ may be a benzene group, a naphthalene group, or a pyridine group, and ring CY₂ may be a benzene group or a naphthalene group.

In one or more embodiments, ring CY₁ and ring CY₂ may each be a benzene group.

In Formulae 2-1 and 2-2, R₁, R₁₁, R₁₂, R₂, R₂₉ₐ, R_{29b}, and Z₁ are each independently hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₇-C₆₀ alkyl aryl group, a substituted or unsubstituted C₇-C₆₀ aryl alkyl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₂-C₆₀ alkyl heteroaryl group, a substituted or unsubstituted C₂-C₆₀ heteroaryl alkyl group, a substituted or unsubstituted C₁-C₆₀ heteroaryloxy group, a substituted or unsubstituted C₁-C₆₀ heteroarylthio group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), - Ge(Q₃)(Q₄)(Q₅), -B(Q₆)(Q₇), -P(=O)(Q₈)(Q₉), or -P(Q₈)(Q₉).

Q₁ toQ₉, Q₁₁ toQ₁₉, Q₂₁ to Q₂₉, and Q₃₁ to Q₃₉ are each independently hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₇-C₆₀ alkyl aryl group, a substituted or unsubstituted C₇-C₆₀ aryl alkyl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₂-C₆₀ alkyl heteroaryl group, a substituted or unsubstituted C₂-C₆₀ heteroaryl alkyl group, a substituted or unsubstituted C₁-C₆₀ heteroaryloxy group, a substituted or unsubstituted C₁-C₆₀ heteroarylthio group, a substituted or unsubstituted monovalent non-aromatic condensed polycyclic group, or a substituted or unsubstituted monovalent non-aromatic condensed heteropolycyclic group.

For example, R₁, R₁₁, R₁₂, R₂, R₂₉ₐ, R_{29b}, and Z₁ may each independently be:
hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, or a C₁-C₂₀ alkylthio group;
a C₁-C₂₀ alkyl group, a C₁-C₂₀ alkoxy group, or a C₁-C₂₀ alkylthio group, each substituted with at least one of deuterium, -F, -Cl, -Br, -I, -SF₅, -CD₃, - CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a deuterated C₁-C₂₀ alkyl group, a cyclopentyl group, a cyclohexyl group, a cycloheptyl group, a cyclooctyl group, an adamantanyl group, a norbornanyl group(a bicyclo[2.2.1]heptyl group), a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.2]octyl group, a (C₁-C₂₀ alkyl)cyclopentyl group, a (C₁-C₂₀ alkyl)cyclohexyl group, a (C₁-C₂₀ alkyl)cycloheptyl group, a (C₁-C₂₀ alkyl)cyclooctyl group, a (C₁-C₂₀ alkyl)adamantanyl group, a (C₁-C₂₀ alkyl)norbornanyl group, a (C₁-C₂₀ alkyl)norbornenyl group, a (C₁-C₂₀ alkyl)cyclopentenyl group, a (C₁-C₂₀ alkyl)cyclohexenyl group, a (C₁-C₂₀ alkyl)cycloheptenyl group, a (C₁-C₂₀ alkyl)bicyclo[1.1.1]pentyl group, a (C₁-C₂₀ alkyl)bicyclo[2.1.1]hexyl group, a (C₁-C₂₀ alkyl)bicyclo[2.2.2]octyl group, a phenyl group, a (C₁-C₂₀ alkyl)phenyl group, a biphenyl group, a terphenyl group, a naphthyl group, a 1,2,3,4-tetrahydronaphthyl group, a pyridinyl group, a pyrimidinyl group, or a combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.2]octyl group, a phenyl group, a (C₁-C₂₀ alkyl)phenyl group, a biphenyl group, a terphenyl group, a naphthyl group, a 1,2,3,4-tetrahydronaphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzoisothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, or an azadibenzothiophenyl group, each unsubstituted or substituted with at least one of deuterium, -F, -Cl, -Br, -I, -SF₅, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a deuterated C₁-C₂₀ alkyl group, a C₁-C₂₀ alkoxy group, a C₁-C₂₀ alkylthio group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.2]octyl group, a (C₁-C₂₀ alkyl)cyclopentyl group, a (C₁-C₂₀ alkyl)cyclohexyl group, a (C₁-C₂₀ alkyl)cycloheptyl group, a (C₁-C₂₀ alkyl)cyclooctyl group, a (C₁-C₂₀ alkyl)adamantanyl group, a (C₁-C₂₀ alkyl)norbornanyl group, a (C₁-C₂₀ alkyl)norbornenyl group, a (C₁-C₂₀ alkyl)cyclopentenyl group, a (C₁-C₂₀ alkyl)cyclohexenyl group, a (C₁-C₂₀ alkyl)cycloheptenyl group, a (C₁-C₂₀ alkyl)bicyclo[1.1.1]pentyl group, a (C₁-C₂₀ alkyl)bicyclo[2.1.1]hexyl group, a (C₁-C₂₀ alkyl)bicyclo[2.2.2]octyl group, a phenyl group, a (C₁-C₂₀ alkyl)phenyl group, a biphenyl group, a terphenyl group, a naphthyl group, a 1,2,3,4-tetrahydronaphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzoimidazolyl group, a benzofuranyl group, a benzothiophenyl group, a benzoisothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, - Si(Q₃₃)(Q₃₄)(Q₃₅), -Ge(Q₃₃)(Q₃₄)(Q₃₅), or a combination thereof; or
-N(Q₁)(Q₂), -Si(Q₃)(Q₄)(Q₅), -Ge(Q₃)(Q₄)(Q₅), -B(Q₆)(Q₇), - P(=O)(Q₈)(Q₉), or -P(Q₈)(Q₉),
wherein Q₁ to Q₉ and Q₃₃ to Q₃₅ are each independently:
   -CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, or - CD₂CDH₂; or
   an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, a phenyl group, a biphenyl group, or a naphthyl group, each unsubstituted or substituted with at least one of deuterium, a C₁-C₂₀ alkyl group, a phenyl group, or a combination thereof.

In one or more embodiments, R₁, R₁₁, R₁₂, R₂, R₂₉ₐ, R_{29b}, and Z₁ may each independently be:
hydrogen, deuterium, -F, or a cyano group;
a C₁-C₂₀ alkyl group, a C₃-C₁₀ cycloalkyl group, a phenyl group, a naphthyl group, a pyridinyl group, a furanyl group, a thiophenyl group, a benzofuranyl group, a benzothiophenyl group, a dibenzofuranyl group, or a dibenzothiophenyl group, each unsubstituted or substituted with at least one of deuterium, -F, a cyano group, a C₁-C₂₀ alkyl group, a deuterated C₁-C₂₀ alkyl group, a fluorinated C₁-C₂₀ alkyl group, a C₃-C₁₀ cycloalkyl group, a deuterated C₃-C₁₀ cycloalkyl group, a fluorinated C₃-C₁₀ cycloalkyl group, a (C₁-C₂₀ alkyl)C₃-C₁₀ cycloalkyl group, a phenyl group, a deuterated a phenyl group, a fluorinated a phenyl group, a (C₁-C₂₀ alkyl)phenyl group, a naphthyl group, a pyridinyl group, a furanyl group, a thiophenyl group, a benzofuranyl group, a benzothiophenyl group, a dibenzofuranyl group, a dibenzothiophenyl group, - Si(Q₃₃)(Q₃₄)(Q₃₅), -Ge(Q₃₃)(Q₃₄)(Q₃₅), or a combination thereof; or
-Si(Q₃)(Q₄)(Q₅), or -Ge(Q₃)(Q₄)(Q₅).

In one or more embodiments, R₁, R₁₁, R₁₂, R₂, R₂₉ₐ, R_{29b} and Z₁ may each independently be hydrogen, deuterium, -F, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, -CH₃, -CD₃, -CD₂H, -CDH₂, -CF₃, -CF₂H, -CFH₂, -OCH₃, - OCDH₂, -OCD₂H, -OCD₃, -SCH₃, -SCDH₂, -SCD₂H, -SCD₃, a group represented by one of Formulae 9-1 to 9-39, a group represented by one of Formulae 9-1 to 9-39 in which at least one hydrogen is substituted with deuterium, a group represented by one of Formulae 9-1 to 9-39 in which at least one hydrogen is substituted with -F, a group represented by one of Formulae 9-201 to 9-230, a group represented by one of Formulae 9-201 to 9-230 in which at least one hydrogen is substituted with deuterium, a group represented by one of Formulae 9-201 to 9-230 in which at least one hydrogen is substituted with -F, a group represented by one of Formulae 10-1 to 10-145, a group represented by one of Formulae 10-1 to 10-145 in which at least one hydrogen is substituted with deuterium, a group represented by one of Formulae 10-1 to 10-145 in which at least one hydrogen is substituted with -F, a group represented by one of Formulae 10-201 to 10-354, a group represented by one of Formulae 10-201 to 10-354 in which at least one hydrogen is substituted with deuterium, a group represented by one of Formulae 10-201 to 10-354 in which at least one hydrogen is substituted with -F, -Si(Q₃)(Q₄)(Q₅), or -Ge(Q₃)(Q₄)(Q₅), wherein Q₃ to Q₅ are as described herein:

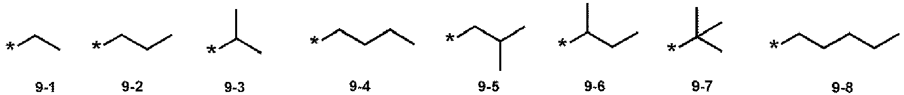

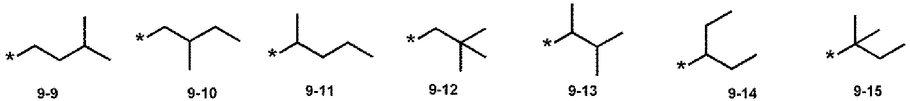

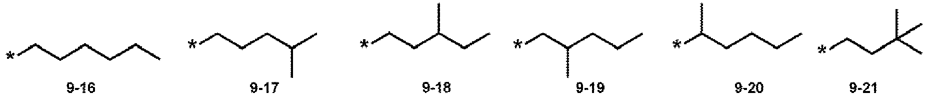

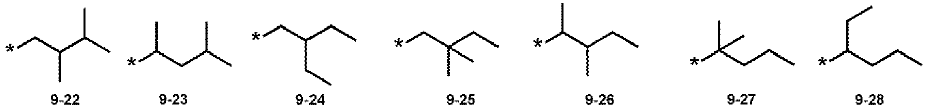

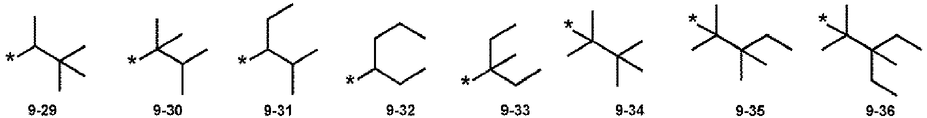

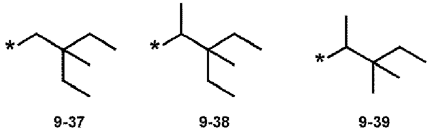

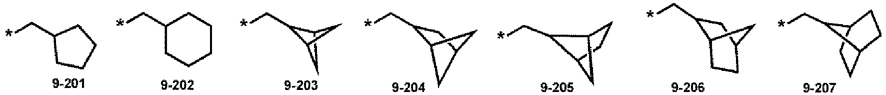

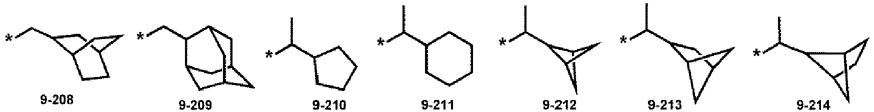

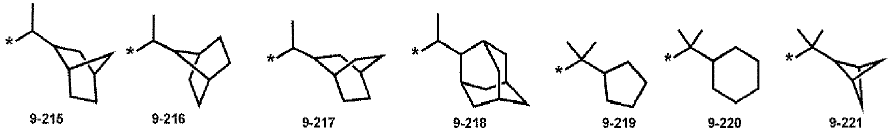

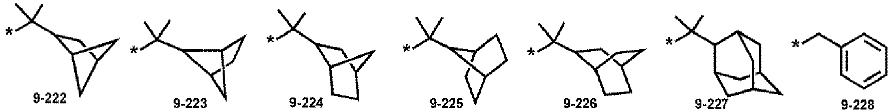

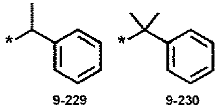

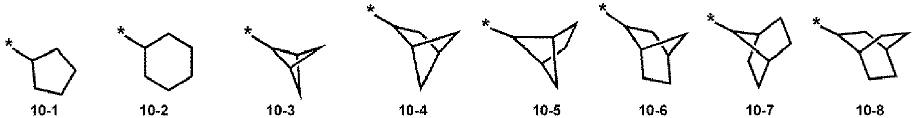

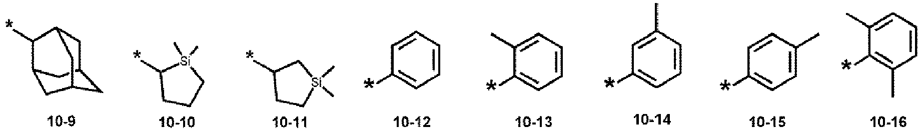

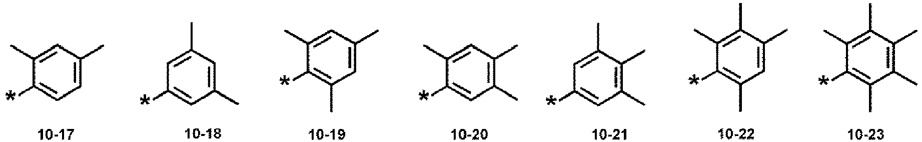

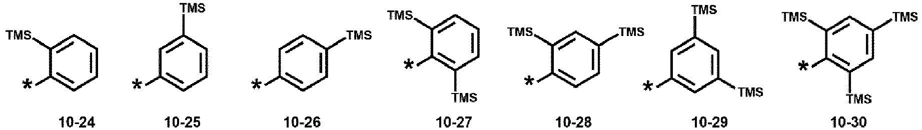

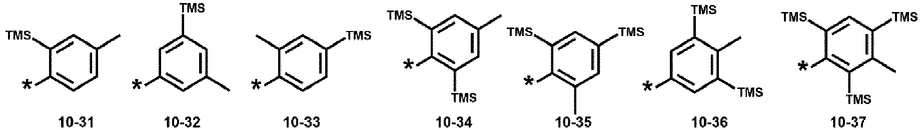

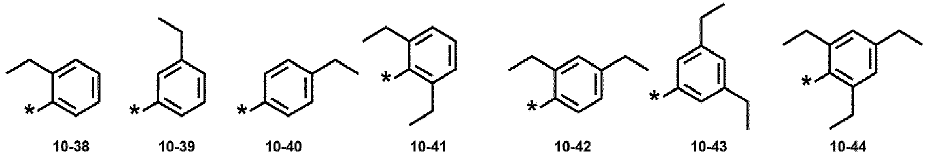

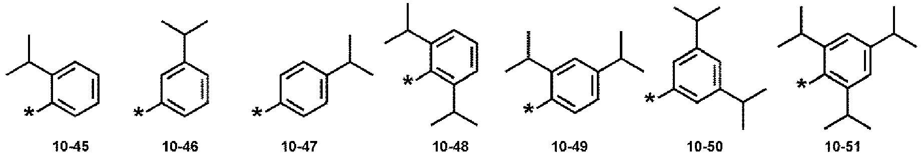

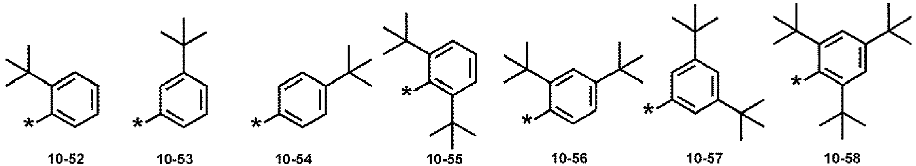

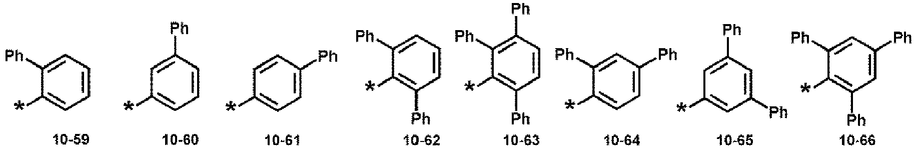

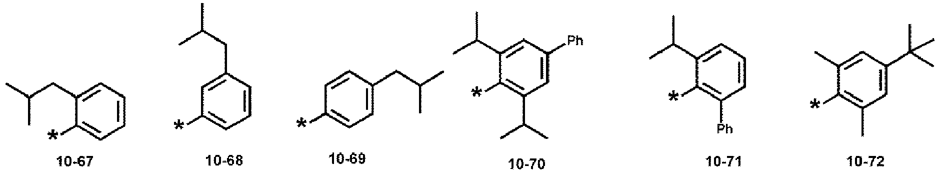

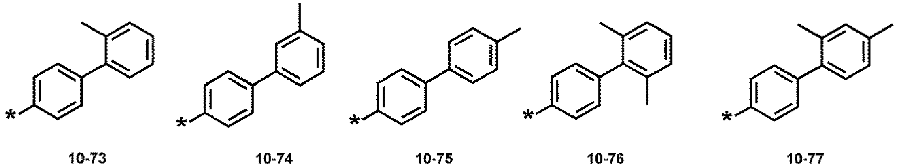

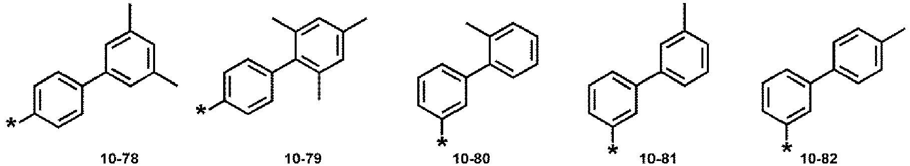

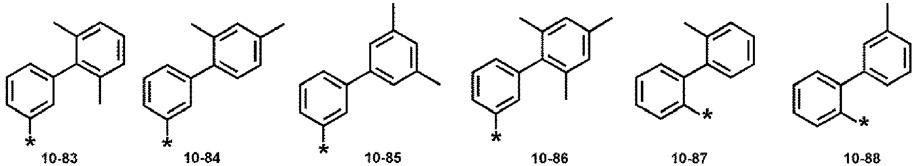

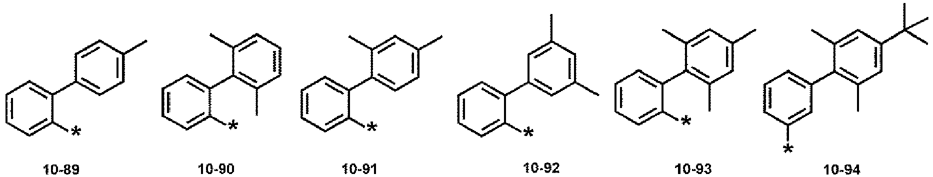

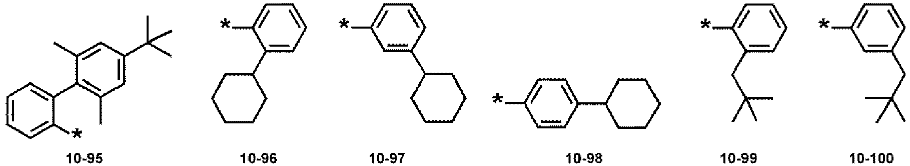

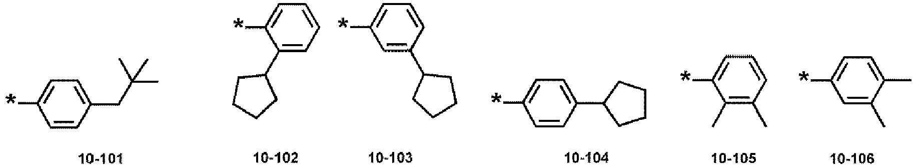

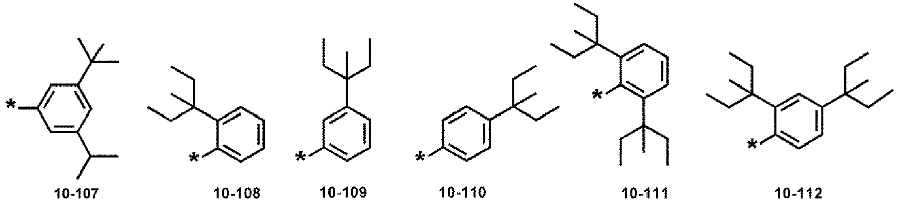

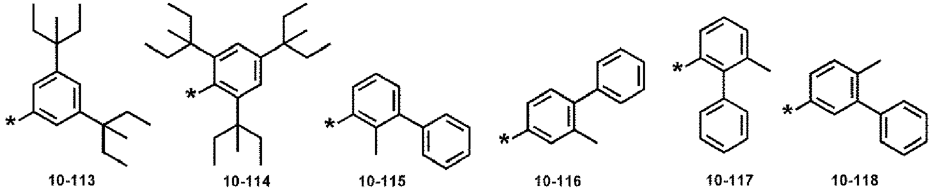

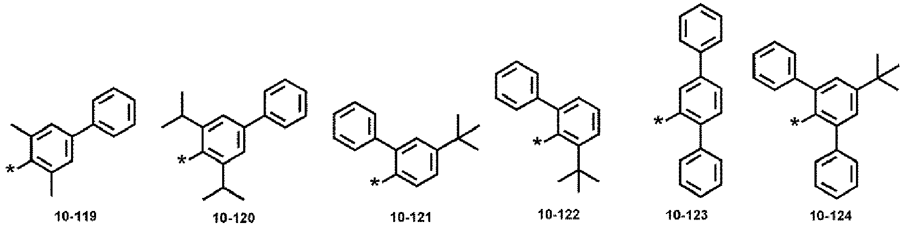

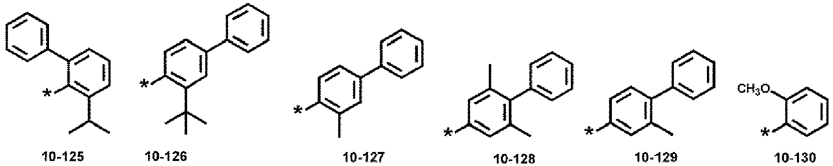

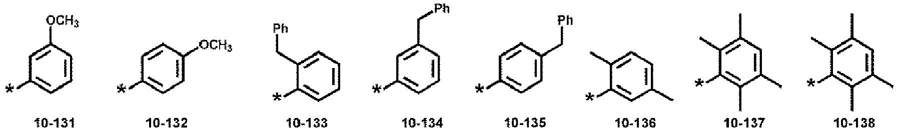

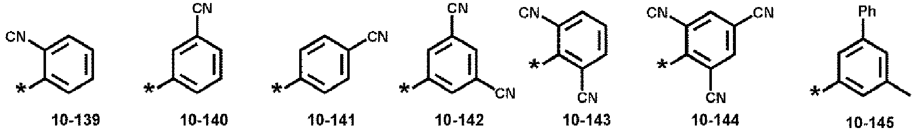

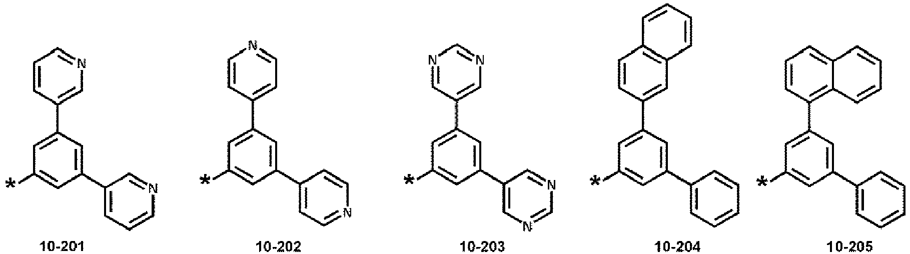

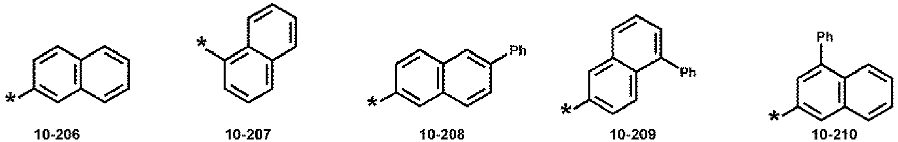

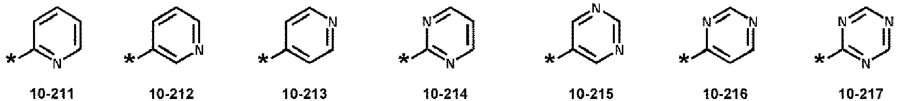

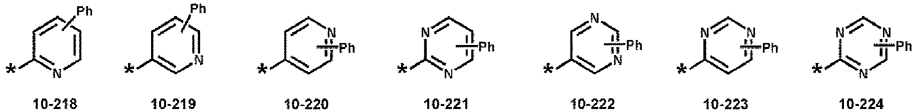

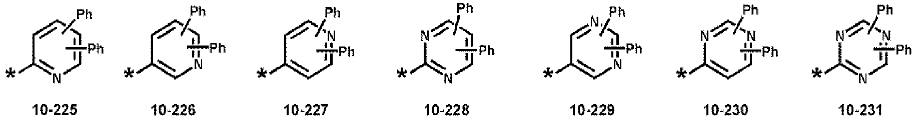

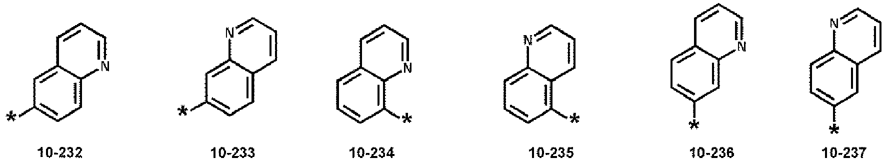

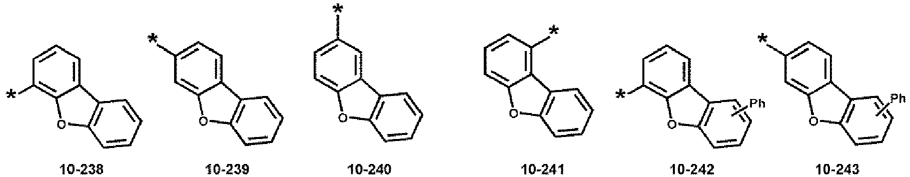

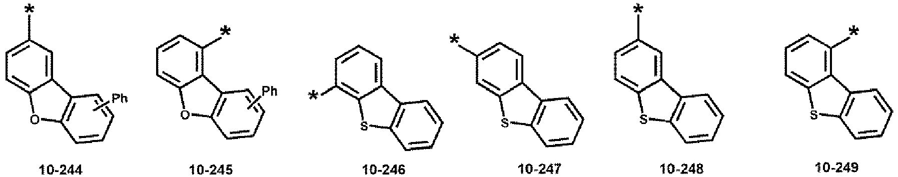

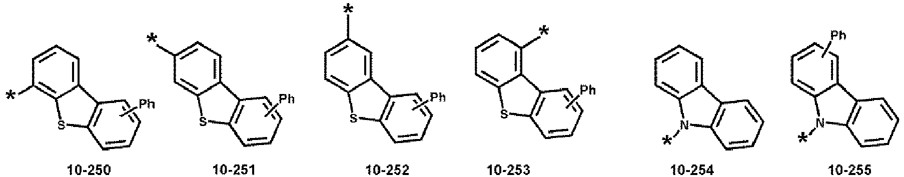

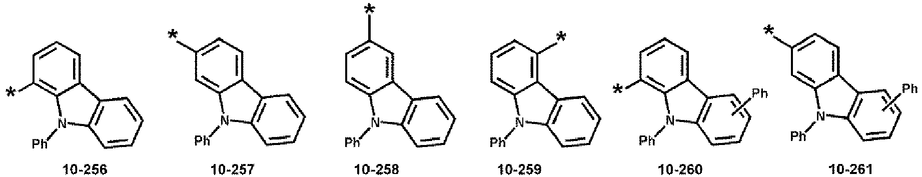

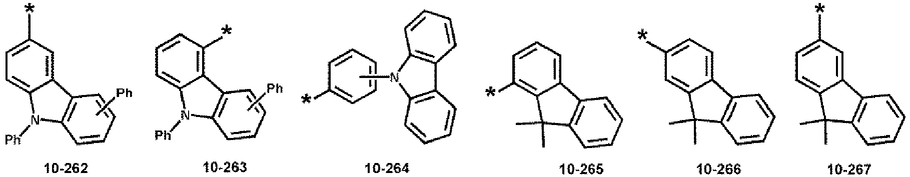

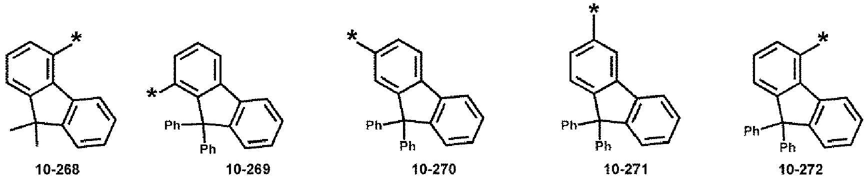

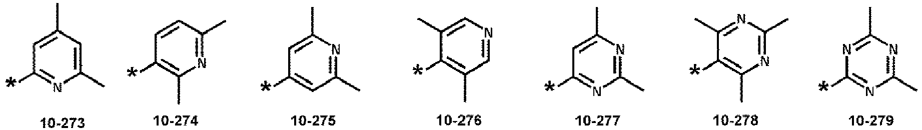

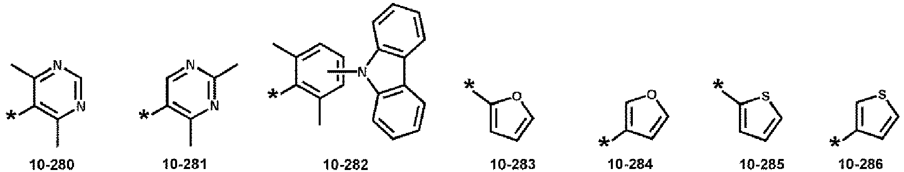

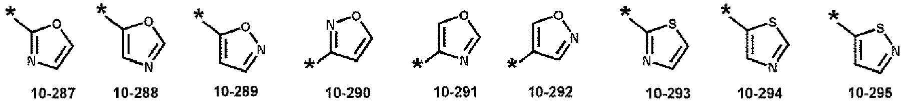

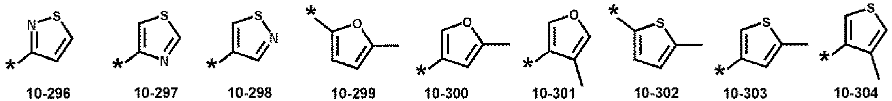

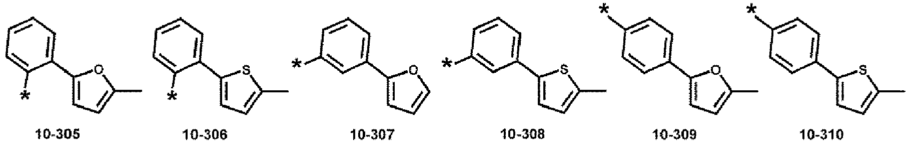

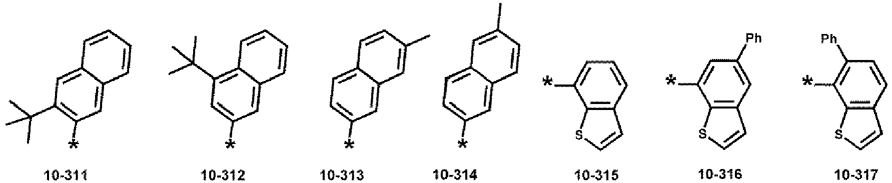

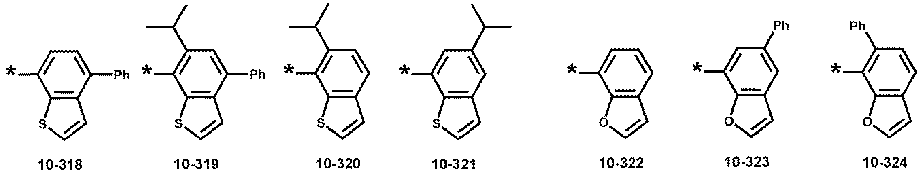

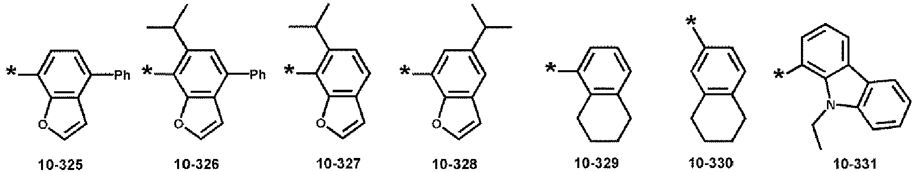

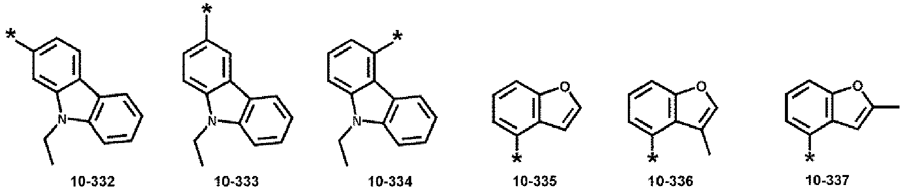

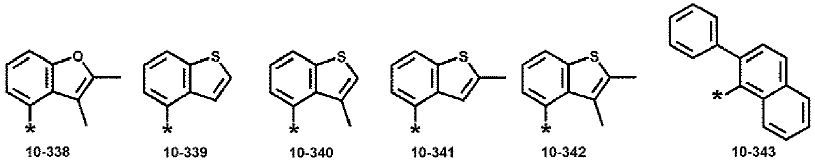

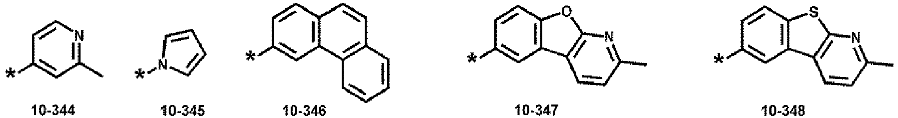

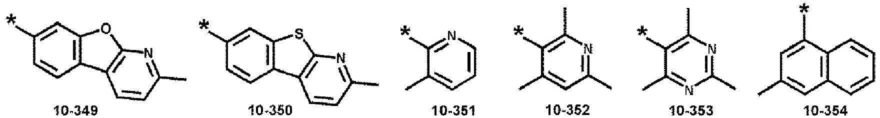

In Formulae 9-1 to 9-39, 9-201 to 9-230, 10-1 to 10-145 and 10-201 to 10-354, * indicates a binding site to a neighboring atom, "Ph" is a phenyl group, "TMS" is a trimethylsilyl group, and "TMG" is a trimethylgermyl group.

The "group represented by one of Formulae 9-1 to 9-39 in which at least one hydrogen is substituted with deuterium" and the "group represented by one of Formulae 9-201 to 9-230 in which at least one hydrogen is substituted with deuterium" may be, for example, a group represented by one of Formulae 9-501 to 9-514 or 9-601 to 9-637:

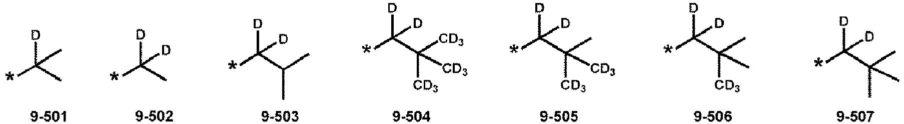

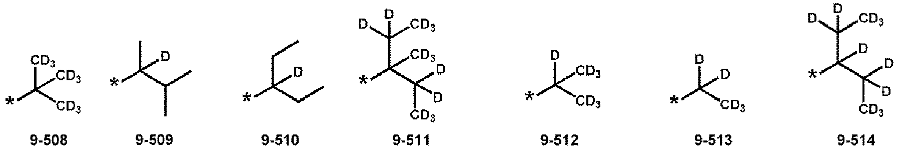

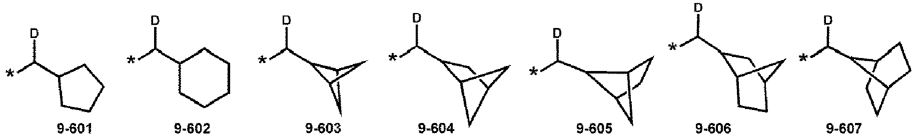

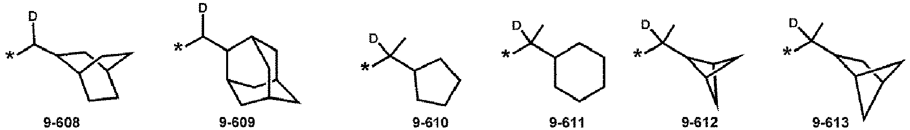

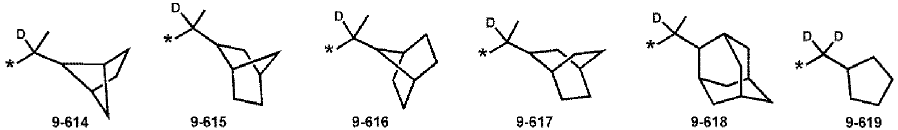

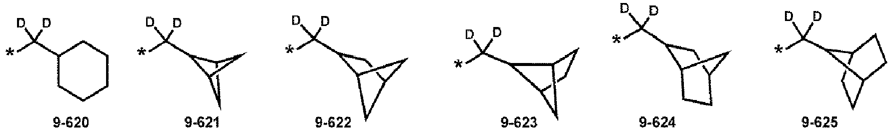

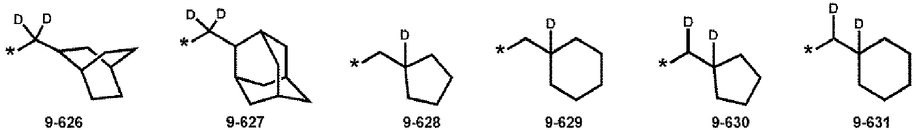

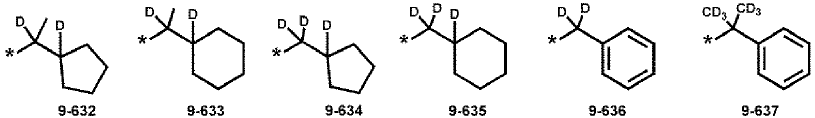

The "group represented by one of Formulae 9-1 to 9-39 in which at least one hydrogen is substituted with -F" and the "group represented by one of Formulae 9-201 to 9-230 in which at least one hydrogen is substituted with -F" may be, for example, a group represented by one of Formulae 9-701 to 9-710:

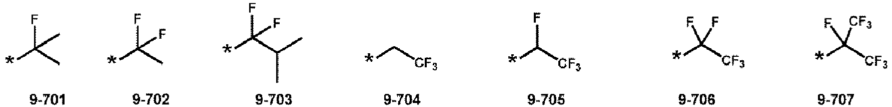

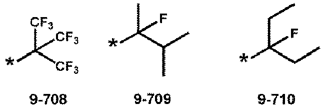

The "group represented by one of Formulae 10-1 to 10-145 in which at least one hydrogen is substituted with deuterium" and the "group represented by one of Formulae 10-201 to 10-354 in which at least one hydrogen is substituted with deuterium" may be, for example, a group represented by one of Formulae 10-501 to 10-553:

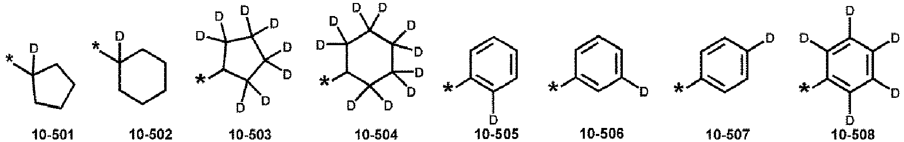

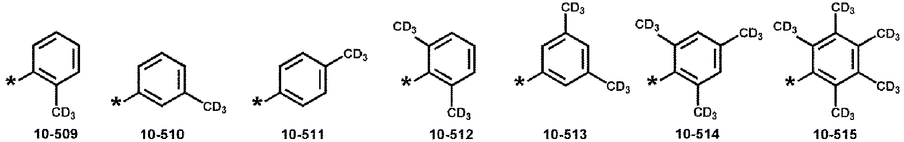

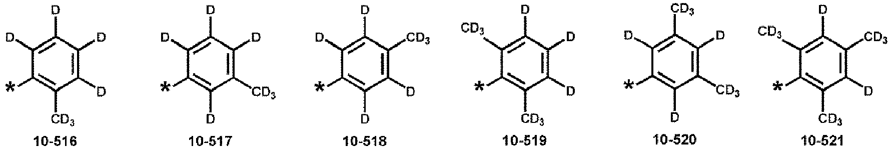

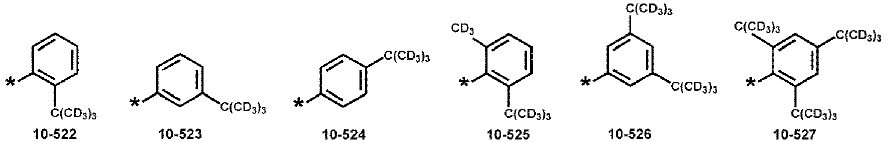

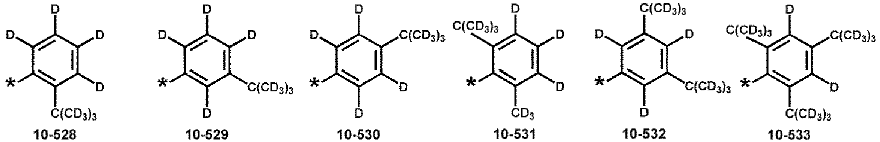

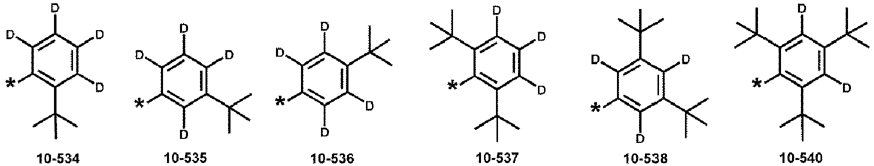

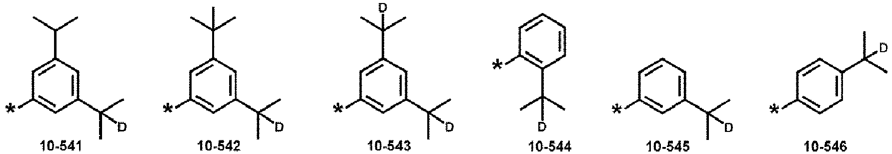

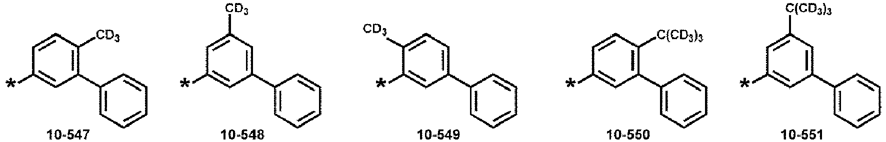

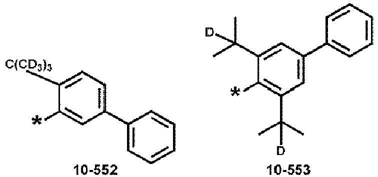

The "group represented by one of Formulae 10-1 to 10-145 in which at least one hydrogen is substituted with -F" and the "group represented by one of Formulae 10-201 to 10-354 in which at least one hydrogen is substituted with -F" may be, for example, a group represented by one of Formulae 10-601 to 10-636:

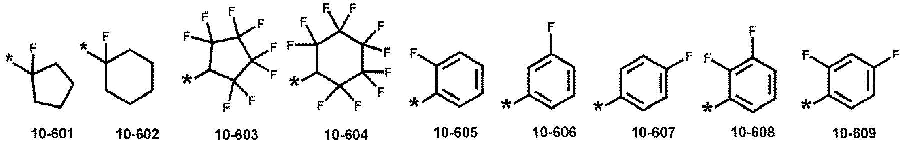

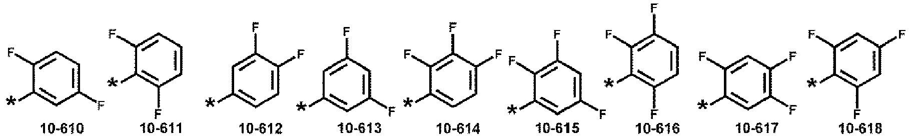

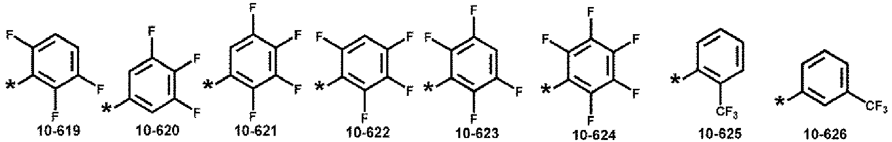

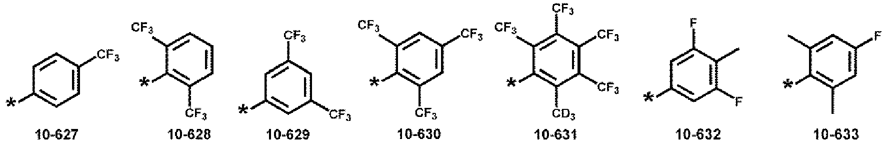

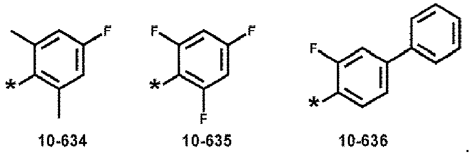

In Formulae 2-1 and 2-2, a1 and a2 each indicates a number of R₁ groups and R₂ groups, respectively, and are each independently an integer from 0 to 20. When a1 is 2 or greater, two or more of R₁ may be identical to or different from each other; when a2 is 2 or greater, two or more of R₂ may be identical to or different from each other. For example, a1 and a2 may each independently be 0, 1, 2, 3, or 4.

In one or more embodiments, in Formulae 2-1 and 2-2, a2 may not be 0, and R₂ may not be hydrogen.

In one or more embodiments, the organometallic compound represented by Formula 1 may include at least one of deuterium, a fluoro group, a deuterated C₁-C₂₀ alkyl group, a fluorinated C₁-C₂₀ alkyl group, -Si(Q₃)(Q₄)(Q₅), -Ge(Q₃)(Q₄)(Q₅), or a combination thereof. Q₃ to Q₅ are each as described herein.

In one or more embodiments, X₁ in Formulae 2-1 and 2-2 may be N-{(T₁)_{b1}-(Z₁)_{c1}}, and X₁ may satisfy at least one of Condition A and Condition B, or X₁ may satisfy Condition C:
Condition A
   T₁ is a C₅-C₃₀ carbocyclic group that is unsubstituted or substituted with at least one R₁₀ₐ, or a C₁-C₃₀ heterocyclic group that is unsubstituted or substituted with at least one R₁₀ₐ;
Condition B
   Z₁ is a substituted or unsubstituted C₆-C₆₀ aryl group, a substituted or unsubstituted C₁-C₆₀ heteroaryl group, a substituted or unsubstituted monovalent non-aromatic condensed polycyclic group, or a substituted or unsubstituted monovalent non-aromatic condensed heteropolycyclic group;
Condition C
   T₁ is a single bond, or a C₁-C₂₀ alkylene group that is unsubstituted or substituted with at least one R₁₀ₐ, and
   Z₁ and R₁₀ₐ are each independently hydrogen, deuterium, -F, a cyano group, a C₁-C₂₀ alkyl group, a deuterated C₁-C₂₀ alkyl group, a fluorinated C₁-C₂₀ alkyl group, -Si(Q₃₃)(Q₃₄)(Q₃₅), -Ge(Q₃₃)(Q₃₄)(Q₃₅), or a combination thereof.

In one or more embodiments, the organometallic compound represented by Formula 1 may satisfy at least one of Condition 1 to Condition 3:
Condition 1
   a1 is not 0, and
   R₁ includes at least one of deuterium, a fluoro group, a cyano group, a deuterated C₁-C₂₀ alkyl group, a fluorinated C₁-C₂₀ alkyl group, a phenyl group, a deuterated a phenyl group, a fluorinated a phenyl group, a (C₁-C₂₀ alkyl)phenyl group, -Si(Q₃)(Q₄)(Q₅), -Ge(Q₃)(Q₄)(Q₅), or a combination thereof
Condition 2
   a2 in Formulae 2-1 and 2-2 is not 0, and
   R₂ includes at least one of deuterium, a fluoro group, a cyano group, a deuterated C₁-C₂₀ alkyl group, a fluorinated C₁-C₂₀ alkyl group, a phenyl group, a deuterated a phenyl group, a fluorinated a phenyl group, a (C₁-C₂₀ alkyl)phenyl group, -Si(Q₃)(Q₄)(Q₅), -Ge(Q₃)(Q₄)(Q₅), or a combination thereof
Condition 3
   X₁ in Formulae 2-1 and 2-2 is N-{(T₁)_{b1}-(Z₁)_{c1}}, and
   a group represented by *-(T₁)_{b1}-(Z₁)_{c1} includes at least one of deuterium, a fluoro group, a cyano group, a deuterated C₁-C₂₀ alkyl group, a fluorinated C₁-C₂₀ alkyl group, a phenyl group, a deuterated a phenyl group, a fluorinated phenyl group, a (C₁-C₂₀ alkyl)phenyl group, -Si(Q₃)(Q₄)(Q₅), -Ge(Q₃)(Q₄)(Q₅), or a combination thereof.

In Formulae 2-1 and 2-2, i) two or more of a plurality of R₁ are optionally linked to each other to form a C₅-C₃₀ carbocyclic group that is unsubstituted or substituted with at least one R₁₀ₐ, or a C₁-C₃₀ heterocyclic group that is unsubstituted or substituted with at least one R_{10a; and} ii) two or more of a plurality of R₂ are optionally linked to each other to form a C₅-C₃₀ carbocyclic group that is unsubstituted or substituted with at least one R₁₀ₐ, or a C₁-C₃₀ heterocyclic group that is unsubstituted or substituted with at least one R₁₀ₐ. That is, i) two or more of a plurality of R₁ may optionally be linked to each other to form a C₅-C₃₀ carbocyclic group that is unsubstituted or substituted with at least one R₁₀ₐ, or a C₁-C₃₀ heterocyclic group that is unsubstituted or substituted with at least one R₁₀ₐ, and ii) two or more of a plurality of R₂ may optionally be linked to each other to form a C₅-C₃₀ carbocyclic group that is unsubstituted or substituted with at least one R₁₀ₐ, or a C₁-C₃₀ heterocyclic group that is unsubstituted or substituted with at least one R₁₀ₐ.

In Formulae 2-1 and 2-2, R₁ and R₂ are optionally linked to each other to form a C₅-C₃₀ carbocyclic group that is unsubstituted or substituted with at least one R₁₀ₐ, or a C₁-C₃₀ heterocyclic group that is unsubstituted or substituted with at least one R₁₀ₐ.

R₁₀ₐ is as described in connection with R₂. For example, R₁₀ₐ may be understood by referring to the description provided in connection with R₂, and R₁₀ₐ may not be hydrogen.

In Formulae 2-1 and 2-2, * and *' each indicates a binding site to M in Formula 1.

At least one substituent of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₁-C₆₀ alkylthio group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₇-C₆₀ alkyl aryl group, the substituted C₇-C₆₀ aryl alkyl group, the substituted C₆-C₆₀ aryloxy group, the substituted C₆-C₆₀ arylthio group, the substituted C₁-C₆₀ heteroaryl group, the substituted C₂-C₆₀ alkyl heteroaryl group, the substituted C₂-C₆₀ heteroaryl alkyl group, the substituted C₁-C₆₀ heteroaryloxy group, the substituted C₁-C₆₀ heteroarylthio group, the substituted monovalent non-aromatic condensed polycyclic group, and the substituted monovalent non-aromatic condensed heteropolycyclic group is:
deuterium, -F, -Cl, -Br, -I, -SF₅, -CD₃, -CD₂H, -CDH₂, -CF₃, -CF₂H, - CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, or a C₁-C₆₀ alkylthio group;
a C₁-C₆₀ alkyl group, a C₂-C₆₀ alkenyl group, a C₂-C₆₀ alkynyl group, a C₁-C₆₀ alkoxy group, or a C₁-C₆₀ alkylthio group, each substituted with at least one of deuterium, -F, -Cl, -Br, -I, -SF₅, -CD₃, -CD₂H, -CDH₂, -CF₃, -CF₂H, - CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇-C₆₀ alkyl aryl group, a C₆-C₆₀ aryloxy group, a C₆-C₆₀ arylthio group, a C₁-C₆₀ heteroaryl group, a C₂-C₆₀ alkyl heteroaryl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), -Ge(Q₁₃)(Q₁₄)(Q₁₅), - B(Q₁₆)(Q₁₇), -P(=O)(Q₁₈)(Q₁₉), -P(Q₁₈)(Q₁₉), or a combination thereof;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇-C₆₀ alkyl aryl group, a C₆-C₆₀ aryloxy group, a C₆-C₆₀ arylthio group, a C₁-C₆₀ heteroaryl group, a C₂-C₆₀ alkyl heteroaryl group, a C₁-C₆₀ heteroaryloxy group, a C₁-C₆₀ heteroarylthio group, a monovalent non-aromatic condensed polycyclic group, or a monovalent non-aromatic condensed heteropolycyclic group, each unsubstituted or substituted with at least one of deuterium, -F, -Cl, -Br, -I, -SF₅, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₁-C₆₀ alkylthio group, C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇-C₆₀ alkyl aryl group, a C₇-C₆₀ aryl alkyl group, a C₆-C₆₀ aryloxy group, a C₆-C₆₀ arylthio group, a C₁-C₆₀ heteroaryl group, a C₂-C₆₀ alkyl heteroaryl group, a C₂-C₆₀ heteroaryl alkyl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), - Ge(Q₂₃)(Q₂₄)(Q₂₅), -B(Q₂₆)(Q₂₇), -P(=O)(Q₂₈)(Q₂₉), P(Q₂₈)(Q₂₉), or a combination thereof;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), - P(=O)(Q₃₈)(Q₃₉), or -P(Q₃₈)(Q₃₉); or
a combination thereof,
wherein Q₁ to Q₉, Q₁₁ to Q₁₉, Q₂₁ to Q₂₉, and Q₃₁ to Q₃₉ are each independently hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C ₆₀ alkyl group, a substituted or unsubstituted C₂-C₆₀ alkenyl group, a substituted or unsubstituted C₂-C₆₀ alkynyl group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₇-C₆₀ alkyl aryl group, a substituted or unsubstituted C₇-C₆₀ aryl alkyl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₂-C₆₀ alkyl heteroaryl group, a substituted or unsubstituted C₂-C₆₀ heteroaryl alkyl group, a substituted or unsubstituted C₁-C₆₀ heteroaryloxy group, a substituted or unsubstituted C₁-C₆₀ heteroarylthio group, a substituted or unsubstituted monovalent non-aromatic condensed polycyclic group, or a substituted or unsubstituted monovalent non-aromatic condensed heteropolycyclic group.

In one or more embodiments, the group represented by 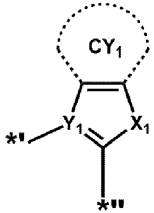 in Formula 2-1 may be a group represented by one of Formulae CY1-1 to CY1-20: 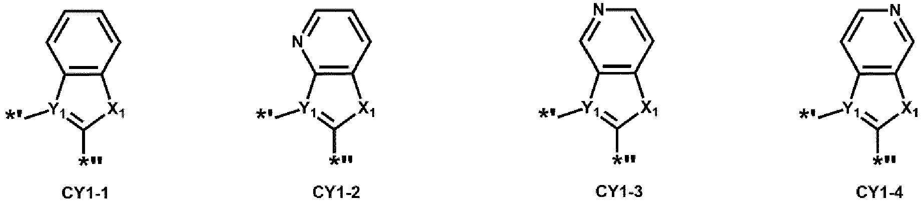 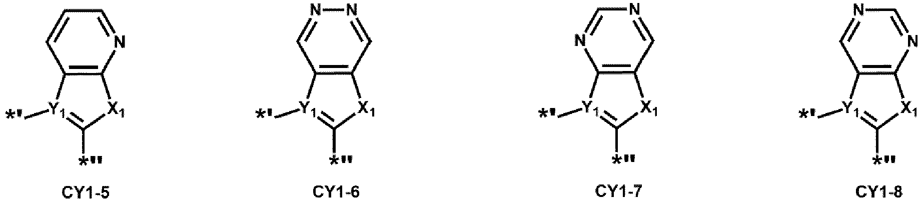 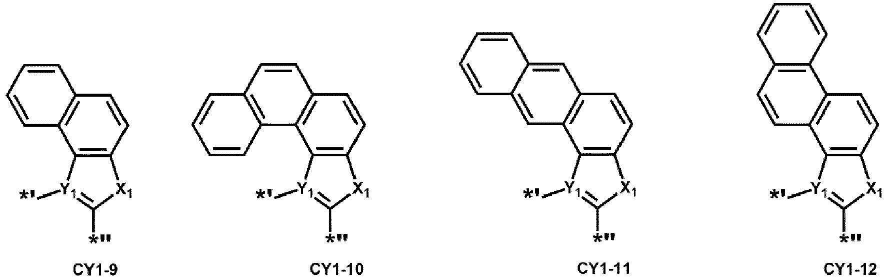 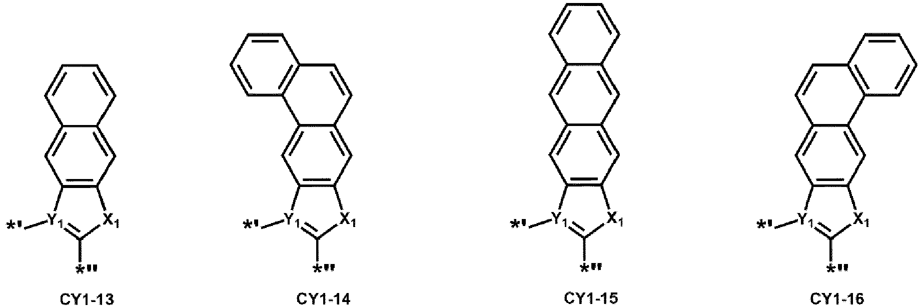 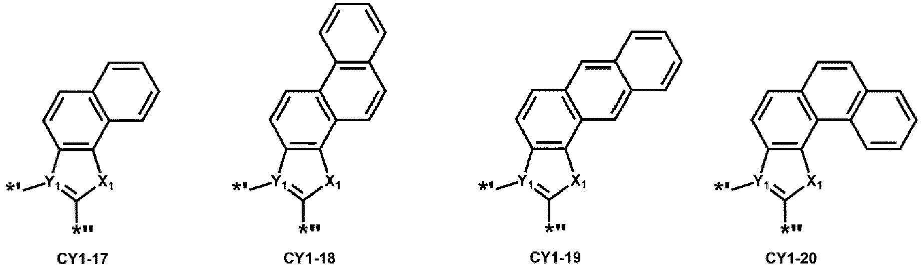 wherein, in Formulae CY1-1 to CY1-20,
Y₁ and X₁ are each as described herein,
*' indicates a binding site to M in Formula 1, and
*" indicates a binding site to one of A₁ to A₄ of Formula 2-1.

In one or more embodiments, a group represented by 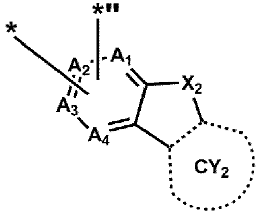 in Formulae 2-1 and 2-2 may be a group represented by one of Formulae CY2(1) to CY2(6): 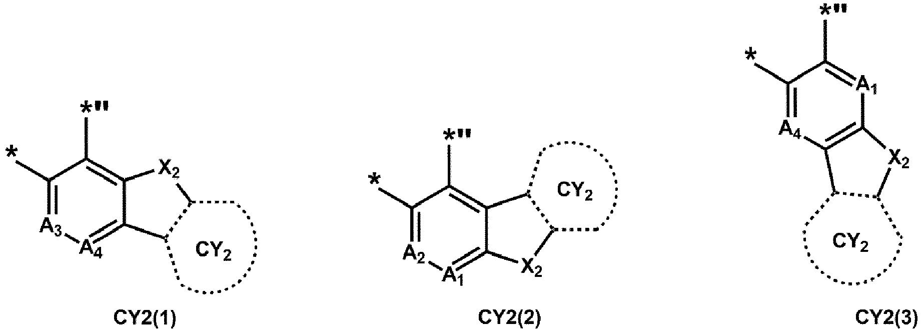 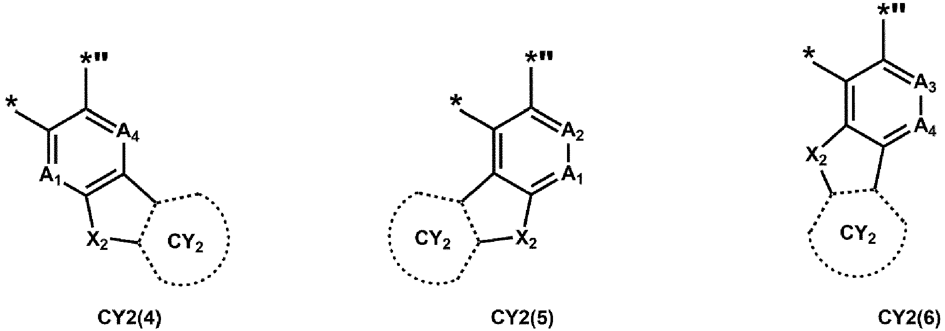 wherein, in Formulae CY2(1) to CY2(6),
X₂ and ring CY₂ are as described herein,
A₁ to A₄ may each be C or N,
*" is a binding site to a neighboring 5-membered ring, and
* is a binding site to M in Formula 1.

In one or more embodiments, a group represented by 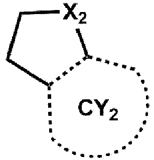 may be a group represented by one of Formulae CY2-1 to CY2-20: 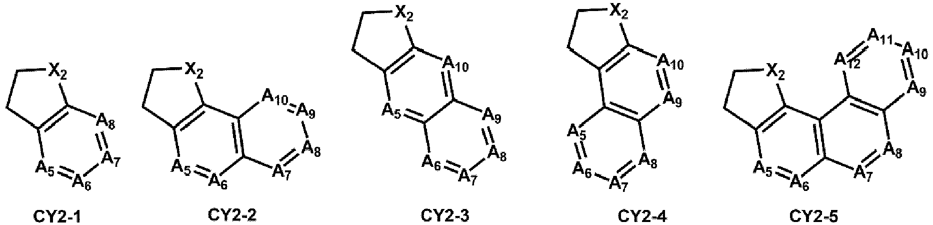 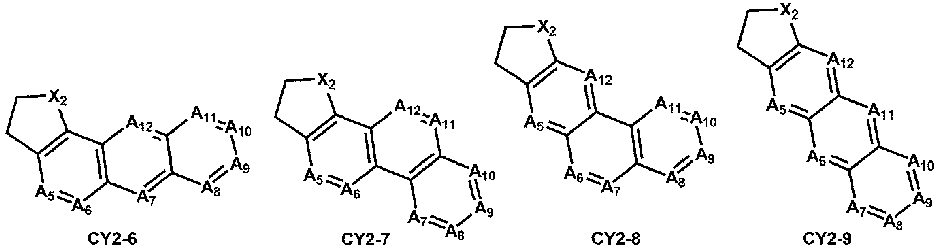 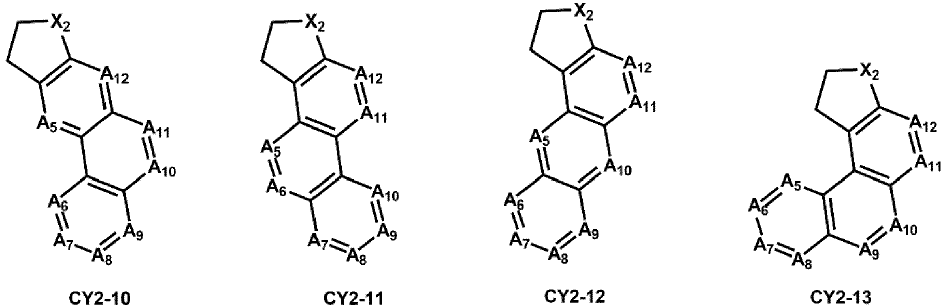 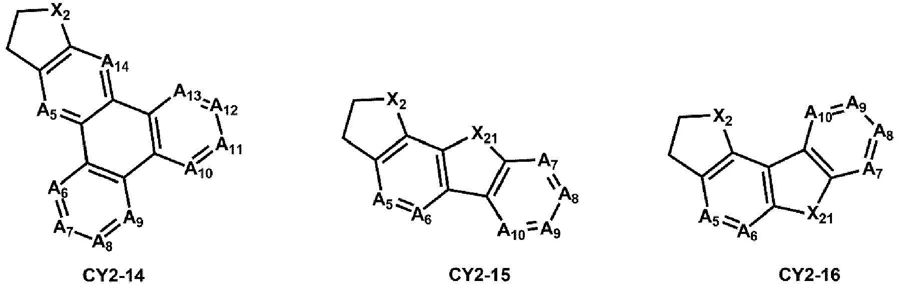 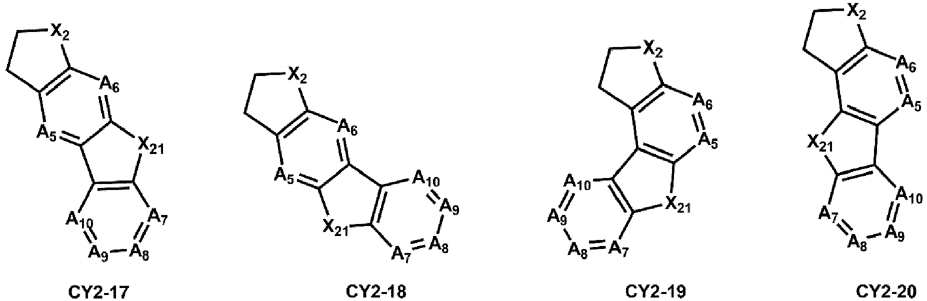 wherein, in Formulae CY2-1 to CY2-20,
X₂ is as described herein,
X₂₁ may be O, S, Se, N(R_{29c}), C(R_{29c})(R_{29d}), Si(R_{29c})(R_{29d}), or Ge(R_{29c})(R_{29d}),
R_{29c} and R_{29d} are each as described in connection with R₂₉ₐ, and
A₅ to A₁₄ may each independently be C or N.

In one or more embodiments, a group represented by 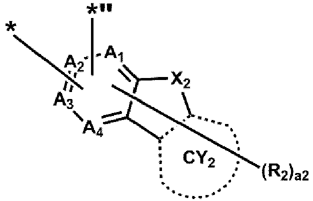 may be a group represented by one of Formulae CY2A to CY2D:

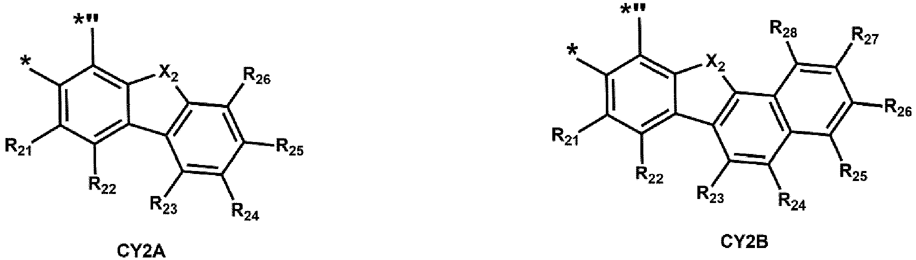

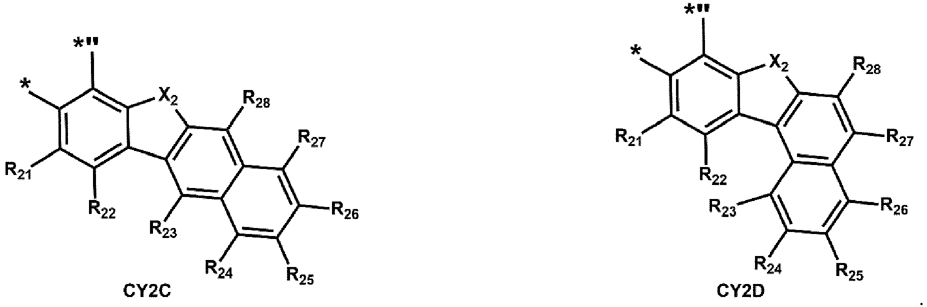

Regarding Formulae CY2A to CY2D,
X₂ is as described herein,
R₂₁ to R₂₈ are each as described in connection with R₂,
*" is a binding site to a neighboring 5-membered ring, and
* is a binding site to M in Formula 1.

In one or more embodiments, the group represented by 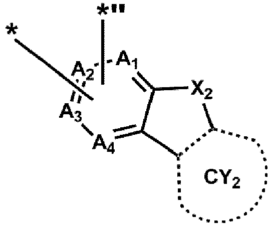
may be substituted with at least one R₂,
   ring CY₁ in the group represented by 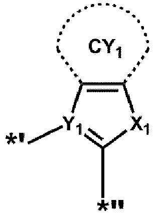
may be substituted with at least one R₁, which may be easily identified from Formulae 2-1 and 2-2 of the present application.

In one or more embodiments, the organometallic compound represented by Formula 1 may be at least one of Compounds 1 to 50:

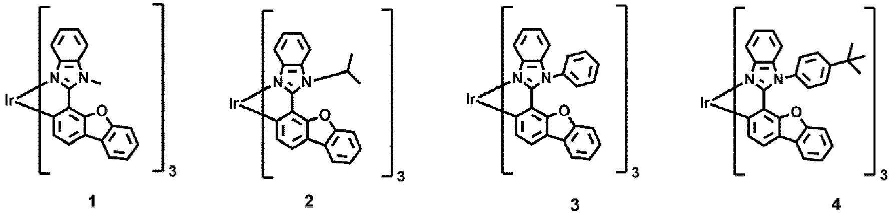

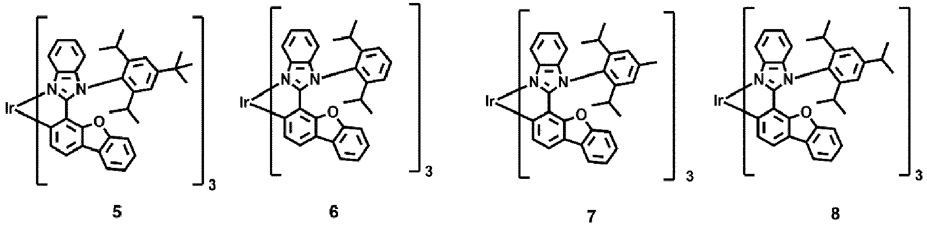

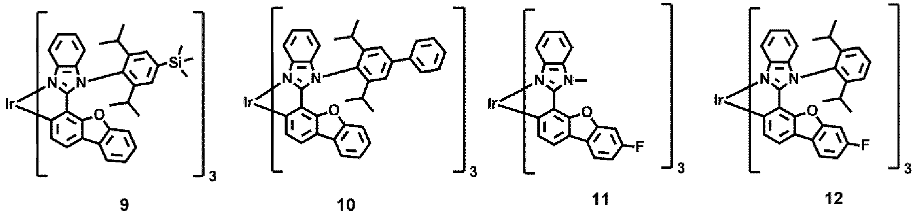

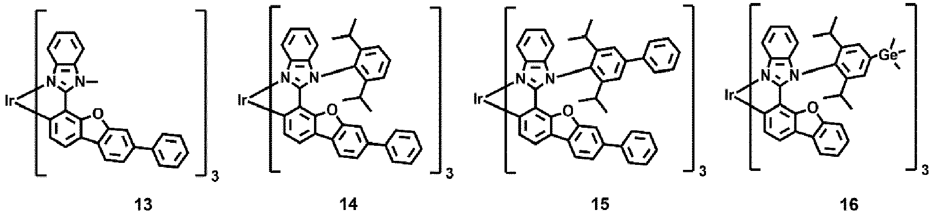

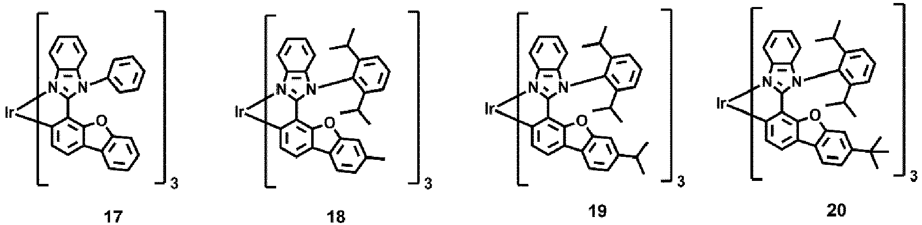

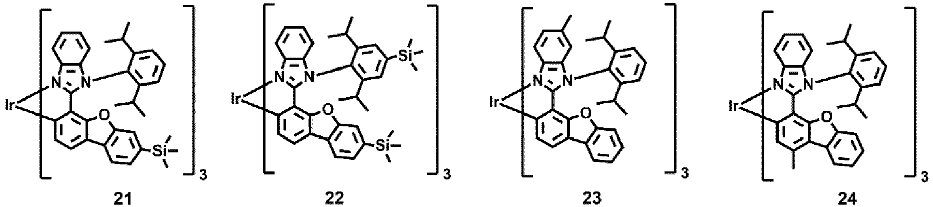

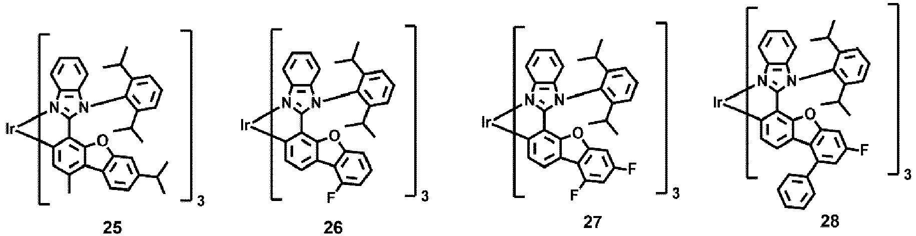

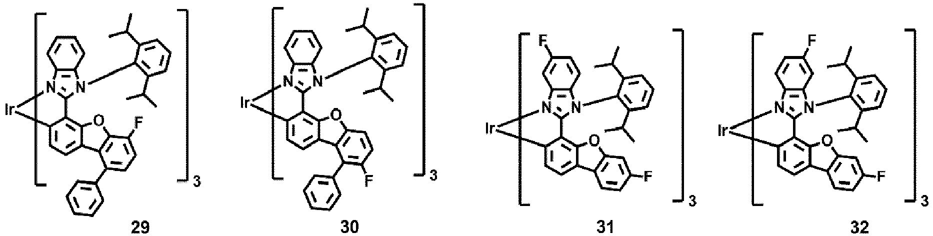

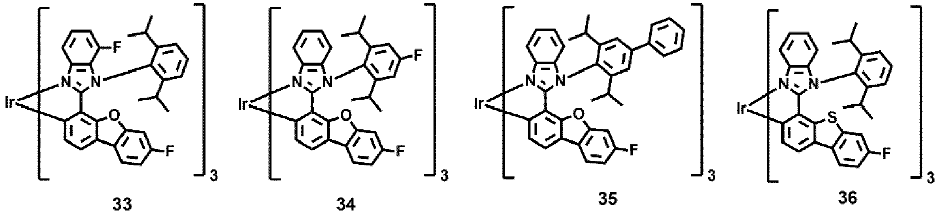

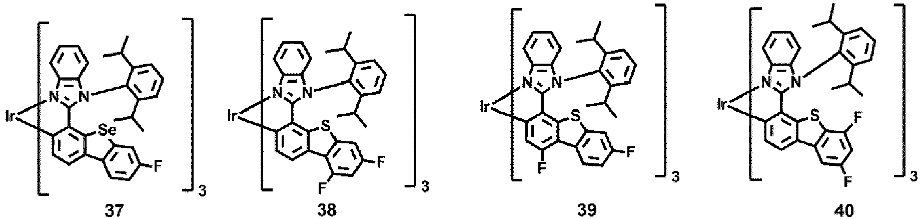

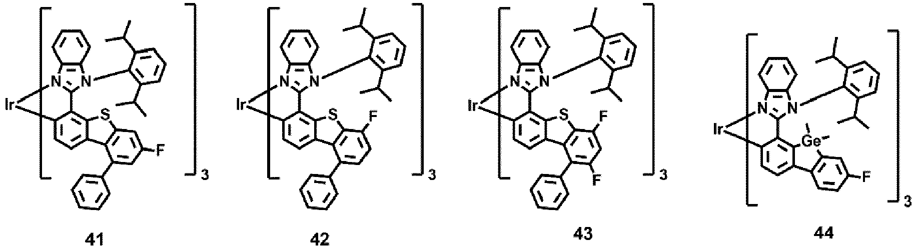

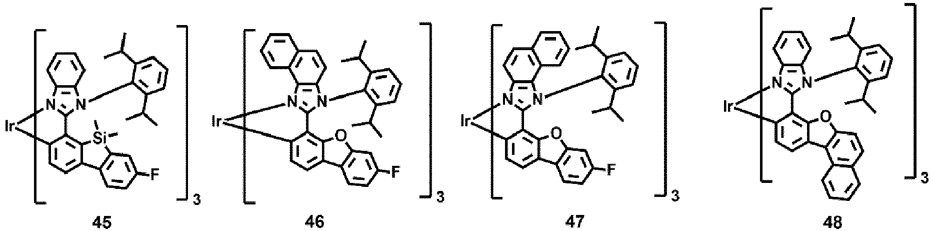

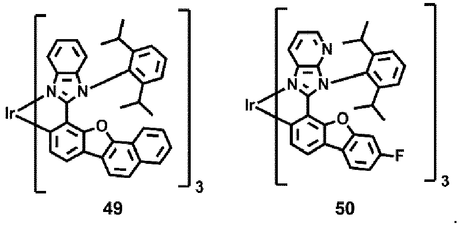

The organometallic compound represented by Formula 1 may be a homoleptic complex with three identical ligands. The ligands each may be a ligand represented by Formula 2-1 or a ligand represented by Formula 2-2 as described herein. In Formulae 2-1 and 2-2, i) a cyclic group including Y₁ may be a 5-membered ring, and ii) X₂ may be O, S, Se, Si(R₂₉ₐ)(R_{29b}), or Ge(R₂₉ₐ)(R_{29b}). Accordingly, the organometallic compound may emit a light with a narrow full width at half maximum (FWHM). In addition, the organometallic compound is structurally strong, and thus, has excellent electrical and thermal stability. An electronic device, for example, a light-emitting device, including at least one of the organometallic compounds may have excellent lifespan characteristics while emitting a light having a relatively narrow FWHM.

A highest occupied molecular orbital (HOMO) energy level, a lowest unoccupied molecular orbital (LUMO) energy level, a band gap, a singlet (S₁) energy level, and a triplet (T₁) energy level of some compounds of the organometallic compound represented by Formula 1 were calculated using a density functional theory (DFT) method of the Gaussian 09 program with the molecular structure optimized at the B3LYP level, and results thereof are shown in Table 1. The energy levels are expressed in electron volts (eV).

**Table 1**

| Compound No. | HOMO (eV) | LUMO (eV) | Band gap (eV) | S₁ (eV) | T₁ (eV) |
|---|---|---|---|---|---|
| 6 | -4.732 | -1.177 | 3.555 | 2.909 | 2.534 |
| 12 | -4.832 | -1.286 | 3.546 | 2.903 | 2.530 |
| 1 | -4.715 | -1.115 | 3.6 | 2.935 | 2.551 |
| 2 | -4.709 | -1.169 | 3.54 | 2.879 | 2.53 |
| 3 | -4.697 | -1.127 | 3.57 | 2.907 | 2.531 |
| 4 | -4.659 | -1.078 | 3.581 | 2.91 | 2.533 |
| 5 | -4.702 | -1.134 | 3.569 | 2.923 | 2.539 |
| 8 | -4.702 | -1.134 | 3.568 | 2.922 | 2.539 |
| 9 | -4.721 | -1.158 | 3.563 | 2.917 | 2.537 |
| 10 | -4.731 | -1.174 | 3.557 | 2.911 | 2.534 |
| 13 | -4.733 | -1.193 | 3.54 | 2.913 | 2.508 |
| 15 | -4.747 | -1.236 | 3.511 | 2.899 | 2.491 |

From Table 1, it was confirmed that the organometallic compound represented by Formula 1 has such electric characteristics that are suitable for use as a dopant for an electronic device, for example, an organic light-emitting device.

For example, the absolute value of the HOMO energy level of the organometallic compound represented by Formula 1 may be from about 4.600 eV to about 4.900 eV, for example, from about 4.659 eV to about 4.832 eV.

For example, the absolute value of LUMO energy level of the organometallic compound represented by Formula 1 may be from about 1.000 eV to about 1.400 eV, for example, from about 1.078 eV to about 1.286 eV.

For example, the S₁ energy level of the organometallic compound represented by Formula 1 may be from about 2.700 eV to about 3.000 eV, for example, from about 2.879 eV to about 2.935 eV.

For example, the T₁ energy level of the organometallic compound represented by Formula 1 may be from about 2.300 eV to about 2.700 eV, for example, from about 2.491 eV to about 2.551 eV.

In one or more embodiments, the organometallic compound represented by Formula 1 may emit a red light or a green light, for example, a red light or a green light, each having a maximum luminescence wavelength of about 500 nm or greater, for example, from about 500 nm to about 850 nm or less. For example, the organometallic compound may emit a green light. In one or more embodiments, the organometallic compound may emit a light (for example, a green light) having a maximum emission wavelength from about 515 nm to about 550 nm, from about 520 nm to about 540 nm, or from about 520 nm to about 530 nm.

In one or more embodiments, the organometallic compound represented by Formula 1 may emit a light (for example, a green light) with a FWHM from about 20 nm to about 65 nm, from about 30 nm to about 65 nm, from about 40 nm to about 65 nm, from about 50 nm to about 65 nm, or from about 60 nm to about 65 nm.

Synthesis methods of the organometallic compounds represented by Formula 1 may be recognizable by one of ordinary skill in the art and by referring to Synthesis Examples provided herein.

Accordingly, the organometallic compound represented by Formula 1 is suitable for use as a material for an organic layer of organic light-emitting device, for example, as a dopant in an emission layer of the organic layer. Thus, another aspect provides an organic light-emitting device including a first electrode; a second electrode; and an organic layer located between the first electrode and the second electrode, wherein the organic layer includes an emission layer, and wherein the organic layer further includes at least one organometallic compound represented by Formula 1.

The organic light-emitting device may have improved lifespan characteristics while emitting light having a relatively small or narrow FWHM by having an organic layer including at least one of the organometallic compounds represented by Formula 1 as described herein.

At least one of the organometallic compounds of Formula 1 may be used between a pair of electrodes of an organic light-emitting device. For example, at least one of the organometallic compounds represented by Formula 1 may be included in the emission layer. In this regard, the at least one organometallic compound may act as a dopant, and the emission layer may further include a host (that is, an amount of the at least one organometallic compound represented by Formula 1 is less than an amount of the host, based on weight). In other words, in one or more embodiments, an amount of the host in the emission layer is greater than an amount of the at least one organometallic compound in the emission layer, based on weight.

The emission layer may emit a red light or a green light, for example, a red light or a green light having a maximum luminescence wavelength of about 500 nm or greater, for example, a maximum luminescence wavelength from about 500 nm to about 850 nm. For example, the organometallic compound may emit a green light. In one or more embodiments, the emission layer (or an organic light-emitting device) may emit a light (for example, a green light) having a maximum emission wavelength from about 515 nm to about 550 nm, from about 520 nm to about 540 nm, or from about 520 nm to about 530 nm. In one or more embodiments, the emission layer (or an organic light-emitting device) may emit a light (for example, a green light) with a FWHM from about 20 nm to about 65 nm, from about 30 nm to about 65 nm, from about 40 nm to about 65 nm, from about 50 nm to about 65 nm, or from about 60 nm to about 65 nm.

The expression "(an organic layer) includes at least one of organometallic compounds" used herein may include a case in which "(an organic layer) includes identical organometallic compounds represented by Formula 1" and a case in which "(an organic layer) includes two or more different organometallic compounds represented by Formula 1".

For example, the organic layer may include, as the at least one organometallic compound, only Compound 1. In this embodiment, Compound 1 may be included in the emission layer of the organic light-emitting device. In one or more embodiments, the organic layer may include, as the at least one organometallic compound, Compound 1 and Compound 2. In this regard, Compound 1 and Compound 2 may exist in an identical layer (for example, Compound 1 and Compound 2 all may exist in an emission layer).

The first electrode may be an anode, which is a hole injection electrode, and the second electrode may be a cathode, which is an electron injection electrode; or the first electrode may be a cathode, which is an electron injection electrode, and the second electrode may be an anode, which is a hole injection electrode.

In one or more embodiments, in the organic light-emitting device, the first electrode may be an anode, and the second electrode may be a cathode, and the organic layer may further include a hole transport region located between the first electrode and the emission layer, and an electron transport region located between the emission layer and the second electrode, and the hole transport region may include a hole injection layer, a hole transport layer, an electron-blocking layer, a buffer layer, or a combination thereof, and the electron transport region may include a hole-blocking layer, an electron transport layer, an electron injection layer, or a combination thereof.

The term "organic layer" as used herein refers to a single layer and/or a plurality of layers located between the first electrode and the second electrode of the organic light-emitting device. The "organic layer" may include, in addition to an organic compound, an organometallic complex including a metal.

The FIGURE is a schematic cross-sectional view of an organic light-emitting device 10 according to one or more embodiments. Hereinafter, the structure and manufacturing method of the organic light-emitting device 10 according to one or more embodiments will be described in further detail in connection with the FIGURE, but embodiments are not limited thereto. The organic light-emitting device 10 includes a first electrode 11, an organic layer 15, and a second electrode 19, which are sequentially stacked in this stated order.

A substrate may be additionally disposed under the first electrode 11 or on the second electrode 19. The substrate may be a conventional substrate used in organic light-emitting devices, e.g., a glass substrate or a transparent plastic substrate, each having excellent mechanical strength, thermal stability, transparency, surface smoothness, ease of handling, and/or water repellency.

The first electrode 11 may be produced by depositing or sputtering, onto the substrate, a material for forming the first electrode 11. The first electrode 11 may be an anode. The material for forming the first electrode 11 may include materials with a relatively high work function to facilitate hole injection. The first electrode 11 may be a reflective electrode, a semi-transmissive electrode, or a transmissive electrode. The material for forming the first electrode 11 may be indium tin oxide (ITO), indium zinc oxide (IZO), tin oxide (SnO₂), or zinc oxide (ZnO). In one or more embodiments, the material for forming the first electrode 11 may be metal, such as magnesium (Mg), aluminum (Al), silver (Ag), aluminum-lithium (Al-Li), calcium (Ca), magnesium-indium (Mg-In), or magnesium-silver (Mg-Ag).

The first electrode 11 may have a single-layered structure or a multi-layered structure including a plurality of layers. For example, the first electrode 11 may have a three-layered structure of ITO/Ag/ITO.

The organic layer 15 is located on the first electrode 11.

The organic layer 15 may include a hole transport region, an emission layer, an electron transport region, or a combination thereof.

The hole transport region may be located between the first electrode 11 and the emission layer.

The hole transport region may include a hole injection layer, a hole transport layer, an electron-blocking layer, a buffer layer, or a combination thereof.

The hole transport region may include only either a hole injection layer or a hole transport layer. In one or more embodiments, the hole transport region may have a hole injection layer/hole transport layer structure or a hole injection layer/hole transport layer/electron-blocking layer structure, wherein, for each structure, respective layers are sequentially stacked in this stated order from the first electrode 11.

When the hole transport region includes a hole injection layer, the hole injection layer may be formed on the first electrode 11 by using one or more suitable methods, for example, vacuum deposition, spin coating, casting, and/or Langmuir-Blodgett (LB) deposition.

When a hole injection layer is formed by vacuum deposition, the deposition conditions may vary depending on a material that is used to form the hole injection layer, and the structure and thermal characteristics of the hole injection layer. For example, the deposition conditions may include a deposition temperature of about 100°C to about 500°C, a vacuum pressure of about 10⁻⁸ torr to about 10⁻³ torr, and a deposition rate of about 0.01 angstroms per second (Å/sec) to about 100 Å/sec.

When the hole injection layer is formed by spin coating, the coating conditions may vary depending on a material for forming the hole injection layer, and the structure and thermal characteristics of the hole injection layer. For example, the coating conditions may include a coating speed in a range of about 2,000 revolutions per minute (rpm) to about 5,000 rpm and a heat treatment temperature from about 80 °C to about 200 °C for removing a solvent after coating.

The conditions for forming the hole transport layer and the electron-blocking layer may be similar to or the same as the conditions for forming the hole injection layer.

The hole transport region may include at least one of 4,4',4"-tris(3-methylphenylphenylamino)triphenylamine (m-MTDATA), 4,4',4"-tris(N,N-diphenylamino)triphenylamine (TDATA), 4,4',4"-tris{N-(2-naphthyl)-N-phenylamino}-triphenylamine (2-TNATA), N,N'-di(1-naphthyl)-N,N'-diphenylbenzidine (NPB), β-NPB, N,N'-bis(3-methylphenyl)-N,N'-diphenyl-[1,1-biphenyl]-4,4'-diamine (TPD), spiro-TPD, spiro-NPB, methylated NPB, 4,4'-cyclohexylidene bis[N,N-bis(4-methylphenyl)benzenamine] (TAPC), 4,4'-bis[N,N'-(3-tolyl)amino]-3,3'-dimethylbiphenyl (HMTPD), 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), a compound represented by Formula 201 below, a compound represented by Formula 202 below, or a combination thereof, but embodiments are not limited thereto:

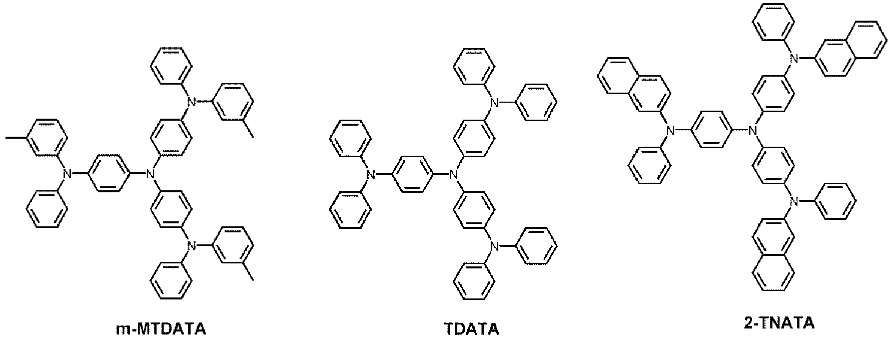

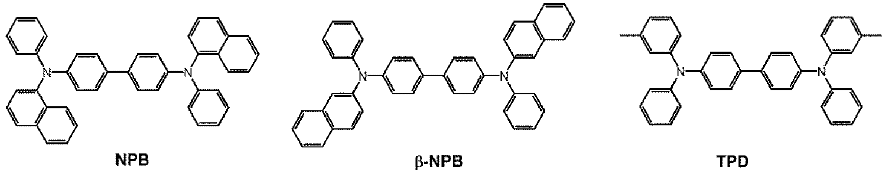

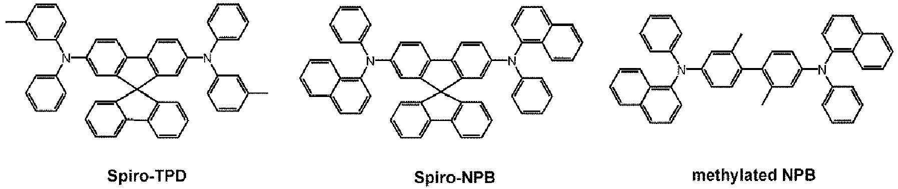

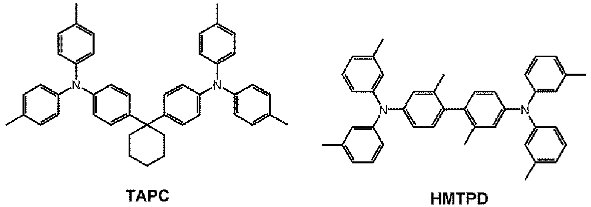

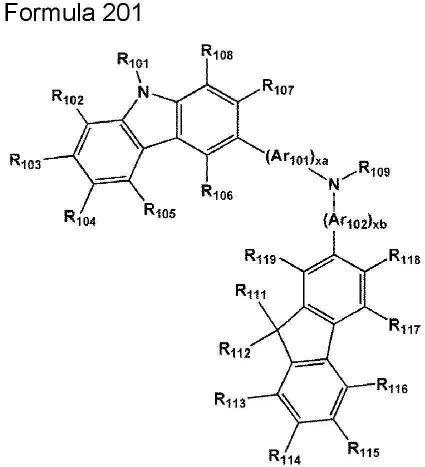

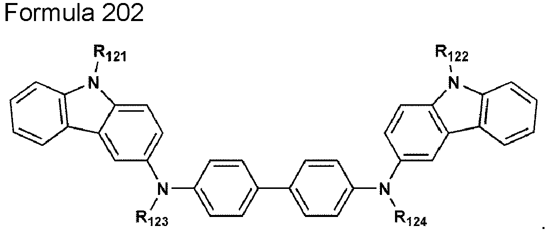

In Formula 201, Ar₁₀₁ and Ar₁₀₂ may each independently be a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group, each unsubstituted or substituted with at least one of deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₁-C₆₀ alkylthio group, a C₃-C₁₀ cycloalkyl group, a C₃-C₁₀ cycloalkenyl group, a C₁-C₁₀ heterocycloalkyl group, a C₁-C₁₀ heterocycloalkenyl group, a C₆-C₆₀ aryl group, a C₇-C₆₀ alkyl aryl group, a C₇-C₆₀ aryl alkyl group, a C₆-C₆₀ aryloxy group, a C₆-C₆₀ arylthio group, a C₁-C₆₀ heteroaryl group, a C₂-C₆₀ alkyl heteroaryl group, a C₂-C₆₀ heteroaryl alkyl group, a C₁-C₆₀ heteroaryloxy group, a C₁-C₆₀ heteroarylthio group, a monovalent non-aromatic condensed polycyclic group, a monovalent non-aromatic condensed heteropolycyclic group, or a combination thereof.

In Formula 201, xa and xb may each independently be an integer from 0 to 5, or 0, 1, or 2. For example, xa may be 1 and xb may be 0.

In Formulae 201 and 202, R₁₀₁ to R₁₀₈, R₁₁₁ to R₁₁₉ and R₁₂₁ to R₁₂₄ may each independently be:
hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group (for example, a methyl group, an ethyl group, a propyl group, a butyl group, a pentyl group, a hexyl group, or the like), a C₁-C₁₀ alkoxy group (for example, a methoxy group, an ethoxy group, a propoxy group, a butoxy group, a pentoxy group, or the like), or a C₁-C₁₀ alkylthio group;
a C₁-C₁₀ alkyl group, a C₁-C₁₀ alkoxy group, or a C₁-C₁₀ alkylthio group, each substituted with at least one of deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, or a combination thereof; or
a phenyl group, a naphthyl group, an anthracenyl group, a fluorenyl group, or a pyrenyl group, each unsubstituted or substituted with at least one of deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a C₁-C₁₀ alkylthio group, or a combination thereof.

In Formula 201, R₁₀₉ may be a phenyl group, a naphthyl group, an anthracenyl group, or a pyridinyl group, each unsubstituted or substituted with at least one of deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a C₁-C₂₀ alkylthio group, a phenyl group, a naphthyl group, an anthracenyl group, a pyridinyl group, or a combination thereof.

In one embodiment, the compound represented by Formula 201 may be represented by Formula 201A, but embodiments are not limited thereto: 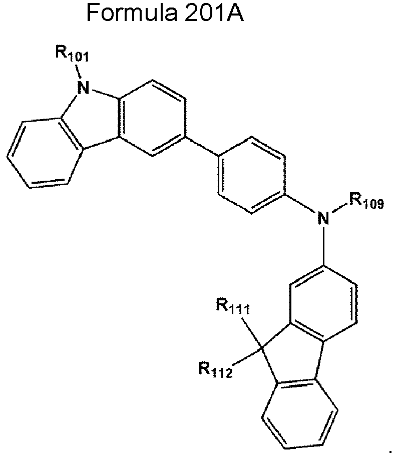 R₁₀₁, R₁₁₁, R₁₁₂, and R₁₀₉ in Formula 201A are each as described herein.

For example, the compound represented by Formula 201 and the compound represented by Formula 202 may include at least one of Compounds HT1 to HT20, but embodiments are not limited thereto:

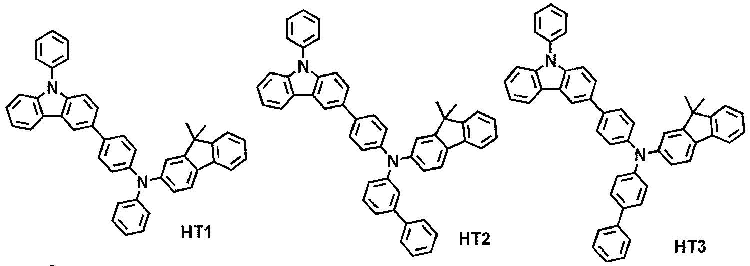

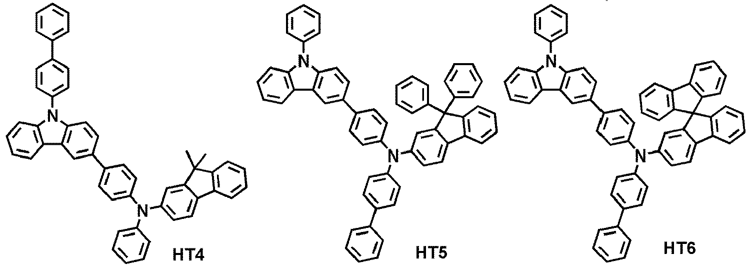

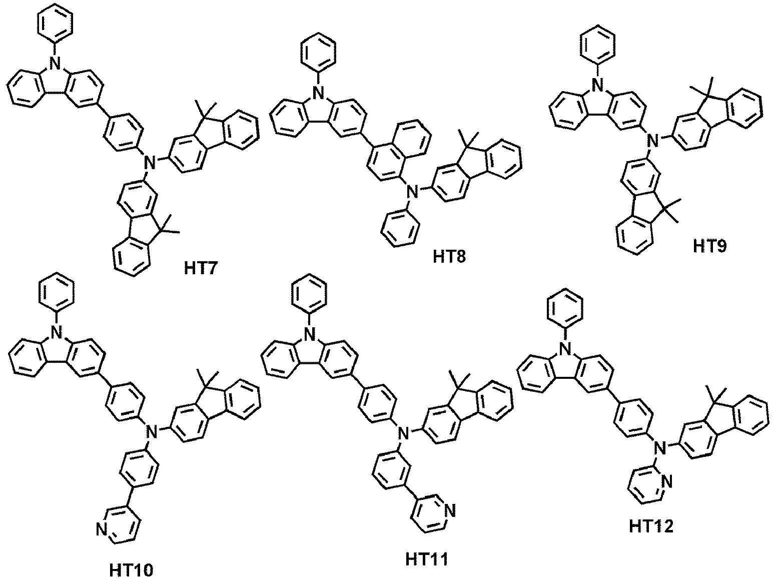

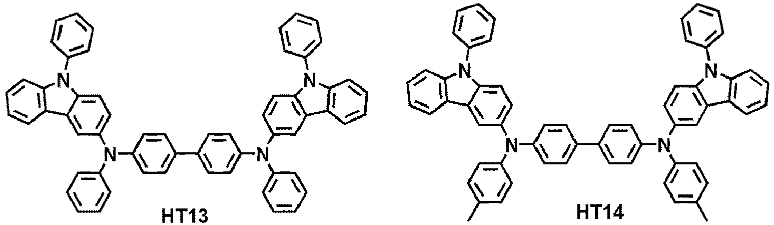

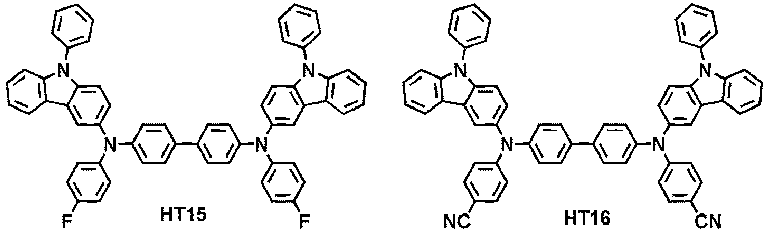

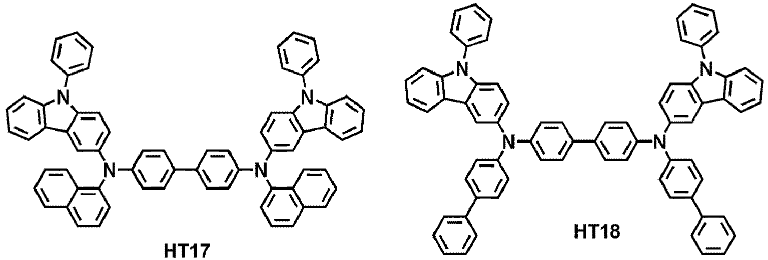

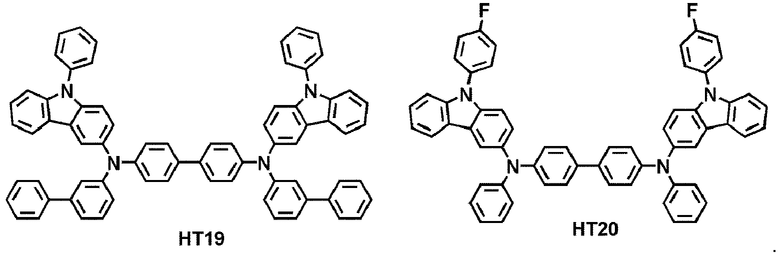

A thickness of the hole transport region may be from about 100 Å to about 10,000 Å, for example, from about 100 Å to about 3,000 Å. When the hole transport region includes at least one of a hole injection layer and a hole transport layer, the thickness of the hole injection layer may be from about 100 Å to about 2,000 Å, for example, from about 100 Å to about 1,000 Å, and the thickness of the hole transport layer may be from about 50 Å to about 2,000 Å, for example, from about 100 Å to about 1,500 Å. Without withing to be bound to theory, when the thicknesses of the hole transport region, the hole injection layer, and/or the hole transport layer are within these ranges, satisfactory hole transporting characteristics may be obtained without a substantial increase in driving voltage.

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant. The p-dopant may be one of a quinone derivative, a metal oxide, a cyano group-containing compound, or a combination thereof. For example, non-limiting examples of the p-dopant may include a quinone derivative, such as tetracyanoquinodimethane (TCNQ), 2,3,5,6-tetrafluoro-tetracyano-1,4-benzoquinonedimethane (F4-TCNQ), 1,3,4,5,7,8-hexafluorotetracyanonaphthoquinodimethane (F6-TCNNQ), or the like; a metal oxide, such as a tungsten oxide, a molybdenum oxide, or the like; or a cyano group-containing compound, such as Compound HT-D1, but embodiments are not limited thereto.

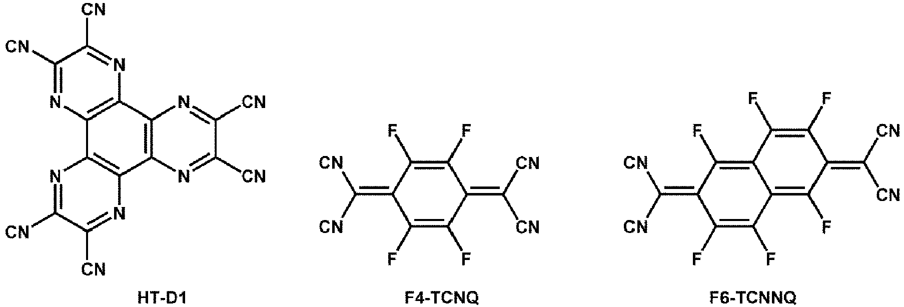

The hole transport region may include a buffer layer.

Also, the buffer layer may compensate for an optical resonance distance according to a wavelength of light emitted from the emission layer, and thus, efficiency of a formed organic light-emitting device may be improved.

Meanwhile, when the hole transport region includes an electron-blocking layer, a material for forming the electron-blocking layer may include a material that is used in the hole transport region as described herein, a host material described herein, or a combination thereof. For example, when the hole transport region includes an electron block layer, the material for forming the electron block layer may be mCP or H-H1, which will be described herein.

Then, an emission layer may be formed on the hole transport region by vacuum deposition, spin coating, casting, LB deposition, or the like. When the emission layer is formed by vacuum deposition or spin coating, the deposition or coating conditions may be similar to those applied in forming the hole injection layer, although the deposition or coating conditions may vary according to a material that is used to form the emission layer.

The emission layer may include a host and a dopant, and the dopant may include at least one of the organometallic compounds represented by Formula 1.

The host may include at least one of 1,3,5-tri(1-phenyl-1H-benzo[d]imidazol-2-yl)benzene (TPBi), 3-tert-butyl-9,10-di(naphth-2-yl)anthracene (TBADN), 9,10-di(naphthalene-2-yl)anthracene (ADN) (also referred to as "DNA"), 4,4'-bis(N-carbazolyl)-1,1'-biphenyl (CBP), 4,4'-bis(9-carbazolyl)-2,2'-dimethyl-biphenyl (CDBP), 1,3,5-tris(carbazole-9-yl)benzene (TCP), 1,3-bis(N-carbazolyl)benzene (mCP), Compound H50, Compound H51, Compound H52, Compound H-H1, Compound HE43, or a combination thereof, but embodiments are not limited thereto:

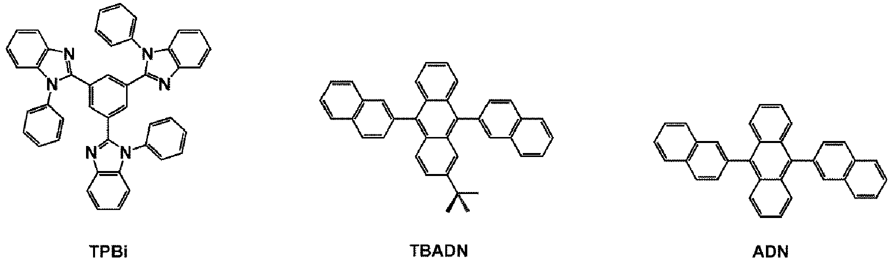

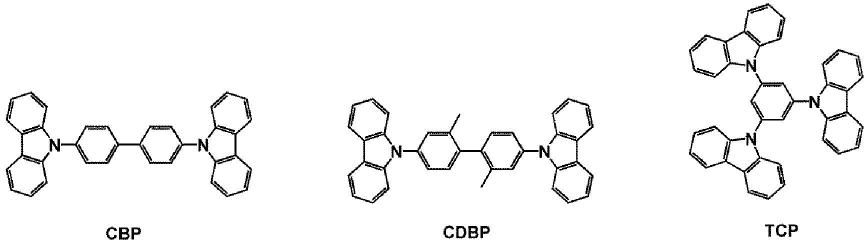

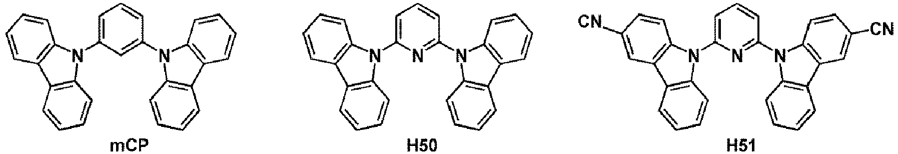

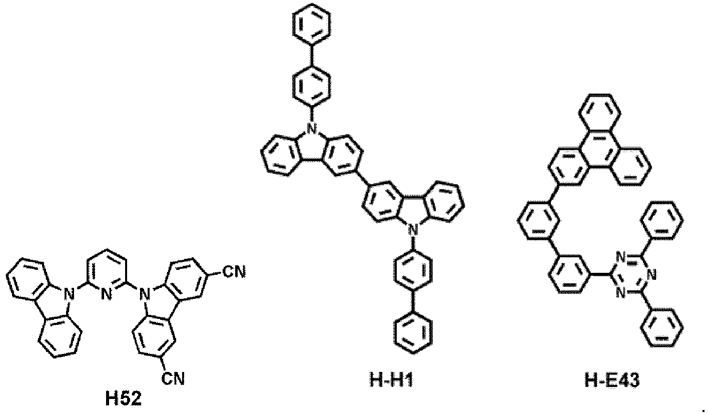

When the organic light-emitting device is a full-color organic light-emitting device, the emission layer may be patterned into a red emission layer, a green emission layer, and/or a blue emission layer. In one or more embodiments, due to a stacked structure including a red emission layer, a green emission layer, and/or a blue emission layer, the emission layer may emit a white light.

When the emission layer includes a host and a dopant, an amount (for example, by weight) of the dopant may be from about 0.01 parts by weight to about 15 parts by weight, based on 100 parts by weight of the host.

A thickness of the emission layer may be from about 100 Å to about 1,000 Å, for example, from about 200 Å to about 600 Å. Without wishing to be bound to theory, when the thickness of the emission layer is within these ranges, excellent light-emission characteristics may be obtained without a substantial increase in driving voltage.

An electron transport region may be located on the emission layer.

The electron transport region may include a hole-blocking layer, an electron transport layer, an electron injection layer, or a combination thereof.

For example, the electron transport region may have a hole-blocking layer/electron transport layer/electron injection layer structure or an electron transport layer/electron injection layer structure. The electron transport layer may have a single-layered structure or a multi-layered structure including two or more different materials.

Conditions for forming the hole-blocking layer, the electron transport layer, and the electron injection layer which constitute the electron transport region may be understood by referring to the conditions for forming the hole injection layer.

When the electron transport region includes a hole-blocking layer, the hole-blocking layer may include, for example, at least one of 2,9-dimethyl-4,7-diphenyl-1,10-phenanthroline (BCP), 4,7-diphenyl-1,10-phenanthroline (Bphen), bis(2-methyl-8-quinolinolato-N1,O8)-(1,1'-biphenyl-4-olato)aluminum (BAlq), or a combination thereof, but embodiments are not limited thereto:

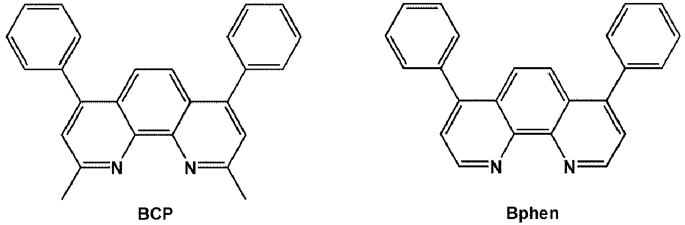

A thickness of the hole-blocking layer may be from about 20 Å to about 1,000 Å, for example, from about 30 Å to about 300 Å. Without wishing to be bound to theory, when the thickness of the hole-blocking layer is within these ranges, excellent hole-blocking characteristics may be obtained without a substantial increase in driving voltage.

In one or more embodiments, the electron transport layer may include at least one of 2,9-dimethyl-4,7-diphenyl-1,10-phenanthroline (BCP), 4,7-diphenyl-1,10-phenanthroline (Bphen), 1,3,5-tri(1-phenyl-1H-benzo[d]imidazol-2-yl)benzene (TPBi), tris(8-hydroxy-quinolinato)aluminum (Alq₃), bis(2-methyl-8-quinolinolato-N1,O8)-(1,1'-biphenyl-4-olato)aluminum (BAlq), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), or a combination thereof, but embodiments are not limited thereto:

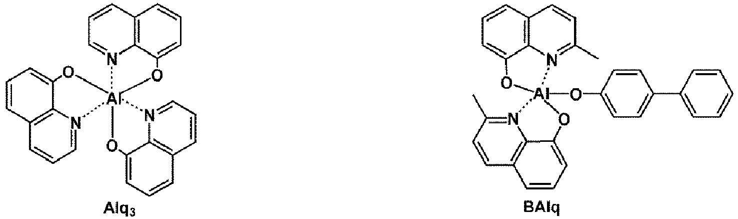

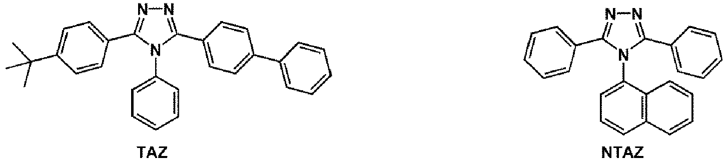

In one or more embodiments, the electron transport layer may include at least one of Compounds ET1 to ET25, or a combination thereof, but embodiments are not limited thereto:

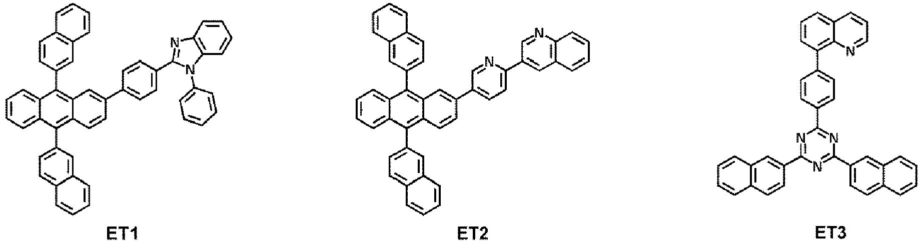

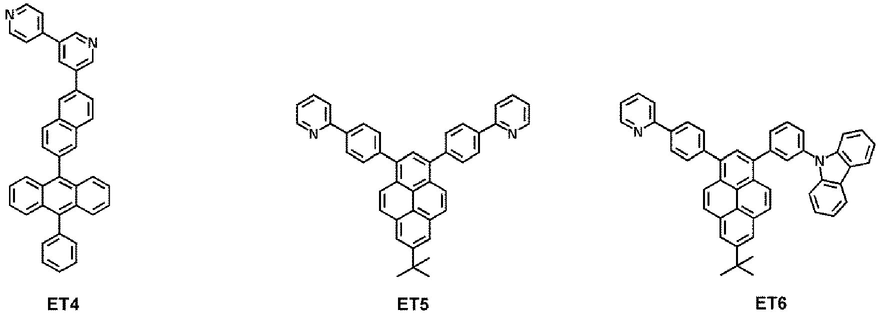

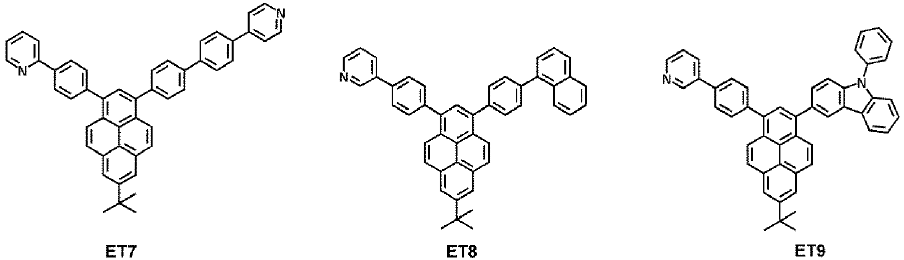

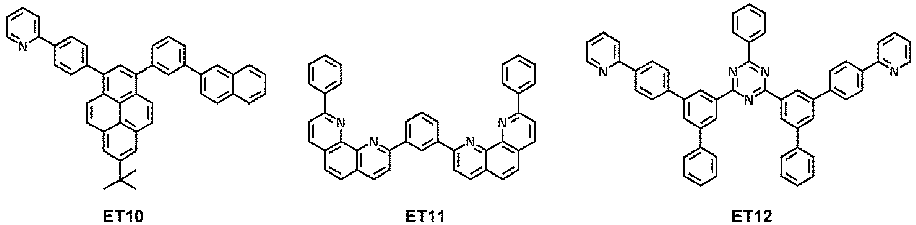

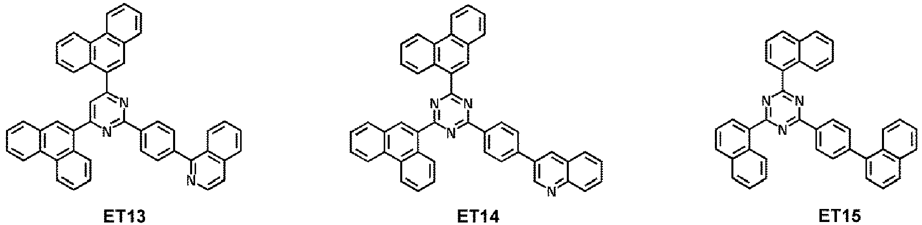

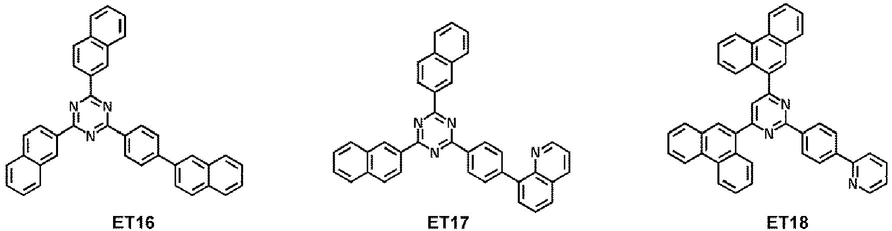

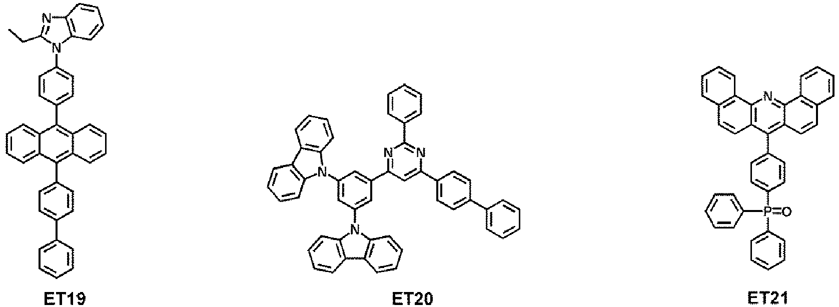

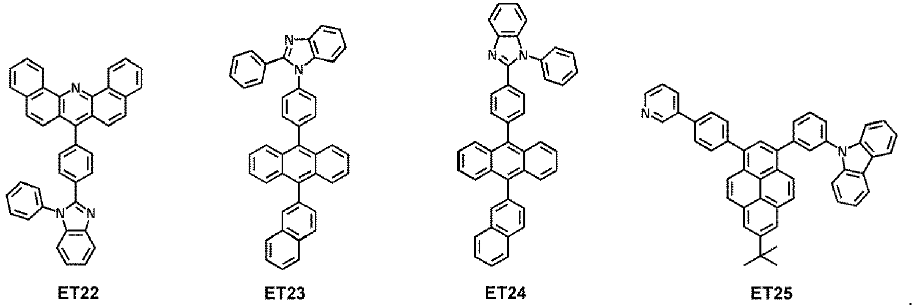

A thickness of the electron transport layer may be from about 100 Å to about 1,000 Å, for example, from about 150 Å to about 500 Å. Without wishing to be bound to theory, when the thickness of the electron transport layer is within the range described above, the electron transport layer may have satisfactory electron transporting characteristics without a substantial increase in driving voltage.

The electron transport layer may include a metal-containing material in addition to the material as described above.

The metal-containing material may include a Li complex. The Li complex may include, for example, Compound ET-D1 (LiQ) or ET-D2, but embodiments are not limited thereto:

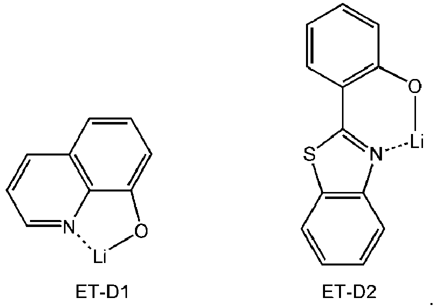

The electron transport region may include an electron injection layer that promotes the flow of electrons from the second electrode 19 thereinto.

The electron injection layer may include at least one of LiF, NaCl, CsF, Li₂O, BaO, or a combination thereof.

A thickness of the electron injection layer may be from about 1 Å to about 100 Å, and, for example, from about 3 Å to about 90 Å. Without wishing to be bound to theory, when the thickness of the electron injection layer is within the ranges described above, satisfactory electron injection characteristics may be obtained without a substantial increase in driving voltage.

The second electrode 19 is located on the organic layer 15. The second electrode 19 may be a cathode. A material for forming the second electrode 19 may be metal, an alloy, an electrically conductive compound, or a combination thereof, which have a relatively low work function. Examples of the material for forming the second electrode 19 may include lithium (Li), silver (Ag), magnesium (Mg), aluminum (Al), aluminum-lithium (Al-Li), calcium (Ca), magnesium-indium (Mg-In), and magnesium-silver (Mg-Ag). In one or more embodiments, to manufacture a top-emission type light-emitting device, a transmissive electrode formed using ITO or IZO may be used as the second electrode 19.

Hereinbefore, the organic light-emitting device has been described with reference to the FIGURE, but embodiments are not limited thereto.

According to another aspect, the organic light-emitting device may be included in an electronic apparatus. Thus, an electronic apparatus including the organic light-emitting device is also provided. The electronic apparatus may include, for example, a display, an illumination, a sensor, or the like, but embodiments are not limited thereto.

Another aspect provides a diagnostic composition including at least one organometallic compound represented by Formula 1.

The organometallic compound represented by Formula 1 provides a high luminescent efficiency. Accordingly, a diagnostic composition including at least one of the organometallic compounds represented by Formula 1 may have a high diagnostic efficiency.

The diagnostic composition may be used in various applications, for example, including a diagnosis kit, a diagnosis reagent, a biosensor, a biomarker, or the like, but embodiments are not limited thereto.

The term "C₁-C₆₀ alkyl group" as used herein refers to a linear or branched saturated aliphatic hydrocarbons monovalent group having 1 to 60 carbon atoms, and the term "C₁-C₆₀ alkylene group" as used here refers to a divalent group having the same structure as the C₁-C₆₀ alkyl group.

Non-limiting examples of the C₁-C₆₀ alkyl group, the C₁-C₂₀ alkyl group, and/or the C₁-C₁₀ alkyl group may include a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, or the like, each unsubstituted or substituted with at least one of a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, or the like, or a combination thereof. For example, Formula 9-33 is a branched C₆ alkyl group, for example, a tert-butyl group that is substituted with two methyl groups.

The term "C₁-C₆₀ alkoxy group" as used herein refers to a monovalent group represented by -OA₁₀₁ (wherein A₁₀₁ is the C₁-C₆₀ alkyl group), and non-limiting examples thereof may include a methoxy group, an ethoxy group, a propoxy group, a butoxy group, a pentoxy group, or the like.

The term "C₂-C₆₀ alkenyl group" as used herein refers to a hydrocarbon group formed by substituting at least one carbon-carbon double bond in the middle or at the terminus of the C₂-C₆₀ alkyl group, and non-limiting examples thereof may include an ethenyl group, a propenyl group, a butenyl group, or the like. The term "C₂-C₆₀ alkenylene group" as used herein refers to a divalent group having the same structure as the C₂-C₆₀ alkenyl group.

The term "C₂-C₆₀ alkynyl group" as used herein refers to a hydrocarbon group formed by substituting at least one carbon-carbon triple bond in the middle or at the terminus of the C₂-C₆₀ alkyl group, and non-limiting examples thereof may include an ethynyl group, a propynyl group, or the like. The term "C₂-C₆₀ alkynylene group" as used herein refers to a divalent group having the same structure as the C₂-C₆₀ alkynyl group.

The term "C₃-C₁₀ cycloalkyl group" as used herein refers to a monovalent saturated hydrocarbon cyclic group having 3 to 10 carbon atoms, and the term "C₃-C₁₀ cycloalkylene group" as used herein refers to a divalent group having the same structure as the C₃-C₁₀ cycloalkyl group.

Non-limiting examples of the C₃-C₁₀cycloalkyl groupmay include acyclopropyl group, a cyclobutyl group, a cyclopentyl, cyclohexyl group, a cycloheptyl group, a cyclooctyl group, an adamantanyl group, a norbornanyl group (or a bicyclo[2.2.1]heptyl group), a bicyclo[1.1.1]pentyl group, a bicyclo[2.1.1]hexyl group, a bicyclo[2.2.2]octyl group, or the like.

The term "C₁-C₁₀ heterocycloalkyl group" as used herein refers to a monovalent saturated cyclic group that includes at least one heteroatom selected from N, O, P, Si, S, Se, Ge, and B as a ring-forming atom and 1 to 10 carbon atoms as ring-forming atom(s), and the term "C₁-C₁₀ heterocycloalkylene group" as used herein refers to a divalent group having the same structure as the C₁-C₁₀ heterocycloalkyl group.

Non-limiting examples of the C₁-C₁₀ heterocycloalkyl group may include a silolanyl group, a silinanyl group, tetrahydrofuranyl group, a tetrahydro-2H-pyranyl group, a tetrahydrothiophenyl group, or the like.

The term "C₃-C₁₀ cycloalkenyl group" as used herein refers to a monovalent cyclic group that includes 3 to 10 carbon atoms and at least one carbon-carbon double bond in the ring thereof and has no aromaticity, and non-limiting examples thereof may include a cyclopentenyl group, a cyclohexenyl group, a cycloheptenyl group, or the like. The term "C₃-C₁₀ cycloalkenylene group" as used herein refers to a divalent group having the same structure as the C₃-C₁₀ cycloalkenyl group.

The term "C₁-C₁₀ heterocycloalkenyl group" as used herein refers to a monovalent cyclic group that includes at least one heteroatom selected from N, O, P, Si, S, Se, Ge, and B as a ring-forming atom, 1 to 10 carbon atoms as ring-forming atom(s), and at least one double bond in the ring thereof and has no aromaticity. Non-limiting examples of the C₁-C₁₀ heterocycloalkenyl group may include a 2,3-dihydrofuranyl group, a 2,3-dihydrothiophenyl group, or the like. The term "C₁-C₁₀ heterocycloalkenylene group" as used herein refers to a divalent group having the same structure as the C₁-C₁₀ heterocycloalkenyl group.

The term "C₆-C₆₀ aryl group" as used herein refers to a monovalent group having a carbocyclic aromatic system having 6 to 60 carbon atoms, and the term "C₆-C₆₀ arylene group" as used herein refers to a divalent group having a carbocyclic aromatic system having 6 to 60 carbon atoms. Non-limiting examples of the C₆-C₆₀ aryl group may include a phenyl group, a naphthyl group, an anthracenyl group, a phenanthrenyl group, a pyrenyl group, a chrysenyl group, or the like. When the C₆-C₆₀ aryl group and the C₆-C₆₀ arylene group each include two or more rings, the rings may be fused to each other.

The term "C₇-C₆₀ alkyl aryl group" as used herein refers to a C₆-C₆₀ aryl group substituted with at least one C₁-C₆₀ alkyl group. The term "C₇-C₆₀ aryl alkyl group" as used herein refers to a C₁-C₆₀ alkyl group substituted with at least one C₆-C₆₀ aryl group.

The term "C₁-C₆₀ heteroaryl group" as used herein refers to a monovalent group that includes a cyclic aromatic system having at least one heteroatom selected from N, O, P, Si, S, Se, Ge, and B as a ring-forming atom and 1 to 60 carbon atoms as ring-forming atom(s), and the term "C₁-C₆₀ heteroarylene group" as used herein refers to a divalent group that includes a cyclic aromatic system having at least one heteroatom selected from N, O, P, Si, S, Se, Ge, and B as a ring-forming atom and 1 to 60 carbon atoms as ring-forming atom(s). Non-limiting examples of the C₁-C₆₀ heteroaryl group may include a pyridinyl group, a pyrimidinyl group, a pyrazinyl group, a pyridazinyl group, a triazinyl group, a quinolinyl group, an isoquinolinyl group, or the like. When the C₁-C₆₀ heteroaryl group and the C₁-C₆₀ heteroarylene group each include two or more rings, the rings may be fused to each other.

The term "C₂-C₆₀ alkyl heteroaryl group" as used herein refers to a C₁-C₆₀ heteroaryl group substituted with at least one C₁-C₆₀ alkyl group. The term "C₂-C₆₀ heteroaryl alkyl group" as used herein refers to a C₁-C₆₀ alkyl group substituted with at least one C₁-C₆₀ heteroaryl group.

The term "C₆-C₆₀ aryloxy group" as used herein indicates -OA₁₀₂ (wherein A₁₀₂ indicates the C₆-C₆₀ aryl group), the term "C₆-C₆₀ arylthio group" as used herein indicates -SA₁₀₃ (wherein A₁₀₃ indicates the C₆-C₆₀ aryl group), and the term "C₁-C₆₀ alkylthio group" as used herein indicates -SA₁₀₄ (wherein A₁₀₄ indicates the C₁-C₆₀ alkyl group).

The term "monovalent non-aromatic condensed polycyclic group" as used herein refers to a monovalent group (for example, having from about 8 to about 60 carbon atoms) having two or more rings condensed to each other, only carbon atoms as ring-forming atoms, and no aromaticity in its entire molecular structure. Non-limiting examples of the monovalent non-aromatic condensed polycyclic group may include a fluorenyl group or the like. The term "divalent non-aromatic condensed polycyclic group" as used herein refers to a divalent group having the same structure as the monovalent non-aromatic condensed polycyclic group described above.

The term "monovalent non-aromatic condensed heteropolycyclic group" as used herein refers to a monovalent group (for example, having from about 1 to about 60 carbon atoms) having two or more rings condensed to each other, at least one heteroatom selected from N, O, P, Si, S, Se, Ge, and B, other than carbon atoms, as a ring-forming atom, and no aromaticity in its entire molecular structure. Non-limiting examples of the monovalent non-aromatic condensed heteropolycyclic group may include a carbazolyl group or the like. The term "divalent non-aromatic condensed heteropolycyclic group" as used herein refers to a divalent group having the same structure as the monovalent non-aromatic condensed heteropolycyclic group described above.

The term "C₅-C₃₀ carbocyclic group" as used herein refers to a saturated or unsaturated cyclic group having, as a ring-forming atom, 5 to 30 carbon atoms only. The C₅-C₃₀ carbocyclic group may be a monocyclic group or a polycyclic group. Non-limiting examples of the "C₅-C₃₀ carbocyclic group (unsubstituted or substituted with at least one R₁₀ₐ)" as used herein may include an adamantane group, a norbornene group, a bicyclo[1.1.1]pentane group, a bicyclo[2.1.1]hexane group, a bicyclo[2.2.1]heptane(norbornane) group, a bicyclo[2.2.2]octane group, a cyclopentane group, a cyclohexane group, a cyclohexene group, a benzene group, a naphthalene group, an anthracene group, a phenanthrene group, a triphenylene group, a pyrene group, a chrysene group, a 1,2,3,4-tetrahydronaphthalene group, a cyclopentadiene group, a fluorene group, or the like, each unsubstituted or substituted with at least one R₁₀ₐ.

The term "C₁-C₃₀ heterocyclic group" as used herein refers to a saturated or unsaturated cyclic group having, as a ring-forming atom, at least one heteroatom selected from N, O, P, Si, S, Se, Ge, and B other than 1 to 30 carbon atoms as ring-forming atom(s). The C₁-C₃₀ heterocyclic group may be a monocyclic group or a polycyclic group. The "C₁-C₃₀ heterocyclic group (unsubstituted or substituted with at least one R₁₀ₐ)" may be, for example, a thiophene group, a furan group, a pyrrole group, a silole group, borole group, a phosphole group, a selenophene group, a germole group, a benzothiophene group, a benzofuran group, an indole group, a benzosilole group, a benzoborole group, a benzophosphole group, a benzoselenophene group, a benzogermole group, a dibenzothiophene group, a dibenzofuran group, a carbazole group, a dibenzosilole group, a dibenzoborole group, a dibenzophosphole group, a dibenzoselenophene group, a dibenzogermole group, a dibenzothiophene 5-oxide group, a 9H-fluoren-9-one group, a dibenzothiophene 5,5-dioxide group, an azabenzothiophene group, an azabenzofuran group, an azaindole group, an azaindene group, an azabenzosilole group, an azabenzoborole group, an azabenzophosphole group, an azabenzoselenophene group, an azabenzogermole group, an azadibenzothiophene group, an azadibenzofuran group, an azacarbazole group, an azafluorene group, an azadibenzosilole group, an azadibenzoborole group, an azadibenzophosphole group, an azadibenzoselenophene group, an azadibenzogermole group, an azadibenzothiophene 5-oxide group, an aza-9H-fluoren-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, a 5,6,7,8-tetrahydroquinoline group, or the like, each unsubstituted or substituted with at least one R₁₀ₐ.

Non-limiting examples of the "C₅-C₃₀ carbocyclic group" and "C₁-C₃₀ heterocyclic group" as used herein may include i) a first ring, ii) a second ring, iii) a condensed ring group in which two or more first rings are condensed with each other, iv) a condensed ring group in which two or more second rings are condensed with each other, or v) a condensed ring group in which at least one first ring is condensed with at least one second ring,
the first ring may be a cyclopentane group, a cyclopentene group, a furan group, a thiophene group, a pyrrole group, a silole group, a borole group, a phosphole group, a germole group, a selenophene group, an oxazole group, an isoxazole group, an oxadiazole group, an oxatriazole group, a thiazole group, an isothiazole group, a thiadiazole group, a thiatriazole group, a pyrazole group, an imidazole group, a triazole group, a tetrazole group, an azasilole group, a diazasilole group, or a triazasilole group, and
the second ring may be an adamantane group, a norbornane group, a norbornene group, a cyclohexane group, a cyclohexene group, a benzene group, a pyridine group, a pyrimidine group, a pyrazine group, a pyridazine group, or a triazine group.

The terms "fluorinated C₁-C₆₀ alkyl group (or a fluorinated C₁-C₂₀ alkyl group or the like)", "fluorinated C₃-C₁₀ cycloalkyl group", "fluorinated C₁-C₁₀ heterocycloalkyl group," and "fluorinated phenyl group" respectively indicate a C₁-C₆₀ alkyl group (or a C₁-C₂₀ alkyl group or the like), a C₃-C₁₀ cycloalkyl group, a C₁-C₁₀ heterocycloalkyl group, and a phenyl group, each substituted with at least one fluoro group (-F). For example, the term "fluorinated C₁ alkyl group (that is, a fluorinated methyl group)" includes -CF₃, -CF₂H, and -CFH₂. The "fluorinated C₁-C₆₀ alkyl group (or, a fluorinated C₁-C₂₀ alkyl group, or the like)", "the fluorinated C₃-C₁₀ cycloalkyl group", "the fluorinated C₁-C₁₀ heterocycloalkyl group", or "the fluorinated a phenyl group" may be i) a fully fluorinated C₁-C₆₀ alkyl group (or, a fully fluorinated C₁-C₂₀ alkyl group, or the like), a fully fluorinated C₃-C₁₀ cycloalkyl group, a fully fluorinated C₁-C₁₀ heterocycloalkyl group, or a fully fluorinated phenyl group, wherein, in each group, all hydrogen included therein is substituted with a fluoro group, or ii) a partially fluorinated C₁-C₆₀ alkyl group (or, a partially fluorinated C₁-C₂₀ alkyl group, or the like), a partially fluorinated C₃-C₁₀ cycloalkyl group, a partially fluorinated C₁-C₁₀ heterocycloalkyl group, or partially fluorinated phenyl group, wherein, in each group, all hydrogen included therein is not substituted with a fluoro group.

The terms "deuterated C₁-C₆₀ alkyl group (or a deuterated C₁-C₂₀ alkyl group or the like)", "deuterated C₃-C₁₀ cycloalkyl group", "deuterated C₁-C₁₀ heterocycloalkyl group," and "deuterated phenyl group" respectively indicate a C₁-C₆₀ alkyl group (or a C₁-C₂₀ alkyl group or the like), a C₃-C₁₀ cycloalkyl group, a C₁-C₁₀ heterocycloalkyl group, and a phenyl group, each substituted with at least one deuterium. For example, the "deuterated C₁ alkyl group (that is, the deuterated methyl group)" may include - CD₃, -CD₂H, and -CDH₂, and examples of the "deuterated C₃-C₁₀ cycloalkyl group" are, for example, Formula 10-501 or the like. The "deuterated C₁-C₆₀ alkyl group (or, the deuterated C₁-C₂₀ alkyl group or the like)", "the deuterated C₃-C₁₀ cycloalkyl group", "the deuterated C₁-C₁₀ heterocycloalkyl group", or "the deuterated phenyl group" may be i) a fully deuterated C₁-C₆₀ alkyl group (or, a fully deuterated C₁-C₂₀ alkyl group or the like), a fully deuterated C₃-C₁₀ cycloalkyl group, a fully deuterated C₁-C₁₀ heterocycloalkyl group, or a fully deuterated phenyl group, in which, in each group, all hydrogen included therein are substituted with deuterium, or ii) a partially deuterated C₁-C₆₀ alkyl group (or, a partially deuterated C₁-C₂₀ alkyl group or the like), a partially deuterated C₃-C₁₀ cycloalkyl group, a partially deuterated C₁-C₁₀ heterocycloalkyl group, or a partially deuterated phenyl group, in which, in each group, all hydrogen included therein are not substituted with deuterium.

The term "(C₁-C₂₀ alkyl) 'X' group" as used herein refers to a 'X' group that is substituted with at least one C₁-C₂₀ alkyl group. For example, the term "(C₁-C₂₀ alkyl)C₃-C₁₀ cycloalkyl group" as used herein refers to a C₃-C₁₀ cycloalkyl group substituted with at least one C₁-C₂₀ alkyl group, and the term "(C₁-C₂₀ alkyl)phenyl group" as used herein refers to a phenyl group substituted with at least one C₁-C₂₀ alkyl group. A non-limiting example of a (C₁ alkyl) phenyl group may include a toluyl group or the like.

The terms "an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluoren-9-one group, and an azadibenzothiophene 5,5-dioxide group" respectively refer to heterocyclic groups having the same backbones as "an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluoren-9-one group, and a dibenzothiophene 5,5-dioxide group," in which, in each group, at least one carbon selected from ring-forming carbons is substituted with nitrogen.

At least one substituent of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₁-C₆₀ alkylthio group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₇-C₆₀ alkyl aryl group, the substituted C₇-C₆₀ aryl alkyl group, the substituted C₆-C₆₀ aryloxy group, the substituted C₆-C₆₀ arylthio group, the substituted C₁-C₆₀ heteroaryl group, the substituted C₂-C₆₀ alkyl heteroaryl group, the substituted C₂-C₆₀ heteroaryl alkyl group, the substituted C₁-C₆₀ heteroaryloxy group, the substituted C₁-C₆₀ heteroarylthio group, the substituted monovalent non-aromatic condensed polycyclic group, and the substituted monovalent non-aromatic condensed heteropolycyclic group may be:
deuterium, -F, -Cl, -Br, -I, -SF₅, -CD₃, -CD₂H, -CDH₂, -CF₃, -CF₂H, - CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, or a C₁-C₆₀ alkylthio group;
a C₁-C₆₀ alkyl group, a C₂-C₆₀ alkenyl group, a C₂-C₆₀ alkynyl group, a C₁-C₆₀ alkoxy group, or a C₁-C₆₀ alkylthio group, each substituted with at least one of deuterium, -F, -Cl, -Br, -I, -SF₅, -CD₃, -CD₂H, -CDH₂, -CF₃, -CF₂H, - CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇-C₆₀ alkyl aryl group, a C₆-C₆₀ aryloxy group, a C₆-C₆₀ arylthio group, a C₁-C₆₀ heteroaryl group, a C₂-C₆₀ alkyl heteroaryl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), -Ge(Q₁₃)(Q₁₄)(Q₁₅), - B(Q₁₆)(Q₁₇), -P(=O)(Q₁₈)(Q₁₉), -P(Q₁₈)(Q₁₉), or a combination thereof;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇-C₆₀ alkyl aryl group, a C₆-C₆₀ aryloxy group, a C₆-C₆₀ arylthio group, a C₁-C₆₀ heteroaryl group, a C₂-C₆₀ alkyl heteroaryl group, a C₁-C₆₀ heteroaryloxy group, a C₁-C₆₀ heteroarylthio group, a monovalent non-aromatic condensed polycyclic group, or a monovalent non-aromatic condensed heteropolycyclic group, each unsubstituted or substituted with at least one of deuterium, -F, -Cl, -Br, -I, -SF₅, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₁-C₆₀ alkylthio group, C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇-C₆₀ alkyl aryl group, a C₇-C₆₀ aryl alkyl group, a C₆-C₆₀ aryloxy group, a C₆-C₆₀ arylthio group, a C₁-C₆₀ heteroaryl group, a C₂-C₆₀ alkyl heteroaryl group, a C₂-C₆₀ heteroaryl alkyl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), - Ge(Q₂₃)(Q₂₄)(Q₂₅), -B(Q₂₆)(Q₂₇), -P(=O)(Q₂₈)(Q₂₉), P(Q₂₈)(Q₂₉), or a combination thereof;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), - P(=O)(Q₃₈)(Q₃₉), or -P(Q₃₈)(Q₃₉); or
a combination thereof,
wherein Q₁ to Q₉, Q₁₁ to Q₁₉, Q₂₁ to Q₂₉, and Q₃₁ to Q₃₉ used herein may each independently be hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C ₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₇-C₆₀ alkyl aryl group, a substituted or unsubstituted C₇-C₆₀ aryl alkyl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₂-C₆₀ alkyl heteroaryl group, a substituted or unsubstituted C₂-C₆₀ heteroaryl alkyl group, a substituted or unsubstituted C₁-C₆₀ heteroaryloxy group, a substituted or unsubstituted C₁-C₆₀ heteroarylthio group, a substituted or unsubstituted monovalent non-aromatic condensed polycyclic group, or a substituted or unsubstituted monovalent non-aromatic condensed heteropolycyclic group.

For example, Q₁ to Q₉, Q₁₁ to Q₁₉, Q₂₁ to Q₂₉ and Q₃₁ to Q₃₉ as described herein may each independently be:
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, or - CD₂CDH₂; or
an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, a phenyl group, a biphenyl group, or a naphthyl group, each unsubstituted or substituted with at least one of deuterium, a C₁-C₁₀ alkyl group, a phenyl group, or a combination thereof.

Hereinafter, a compound and an organic light-emitting device according to exemplary embodiments are described in further detail with reference to Synthesis Example and Examples. However, the organic light-emitting device is not limited thereto. The wording "B was used instead of A" used in describing Synthesis Examples means that an amount of A used was identical to an amount of B used, in terms of a molar equivalent.

### EXAMPLES

### Synthesis Example 1 (Compound 6)

### Synthesis of Compound 6-1

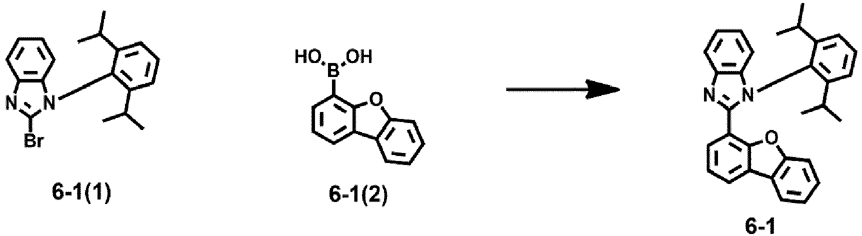

50 milliliters (mL) of tetrahydrofuran (THF) and 20 mL of deionized (DI) water were mixed with Compound 6-1(1) (5.00 g, 14.00 mmol), Compound 6-1(2) (3.56 g, 16.80 mmol), tetrakis(triphenylphosphine)palladium(0) (Pd(PPh₃)₄) (0.81 g, 0.70 mmol), and K₂CO₃ (5.80 g, 42.0 mmol), and then, the reaction content were stirred and heated under reflux for 18 hours. After the temperature was allowed to lower to room temperature, an organic layer was extracted using methylene chloride, and the organic layer was separated and dried using anhydrous magnesium sulfate (MgSO₄). The product was filtered and then the solvent was removed from the filtrate under a reduced pressure. The obtained residue was purified by column chromatography (ethyl acetate(EA):Hexane = 1:6, w/w) to obtain Compound 6-1 (4.3 g, yield of 68%).

### Synthesis of Compound 6-2

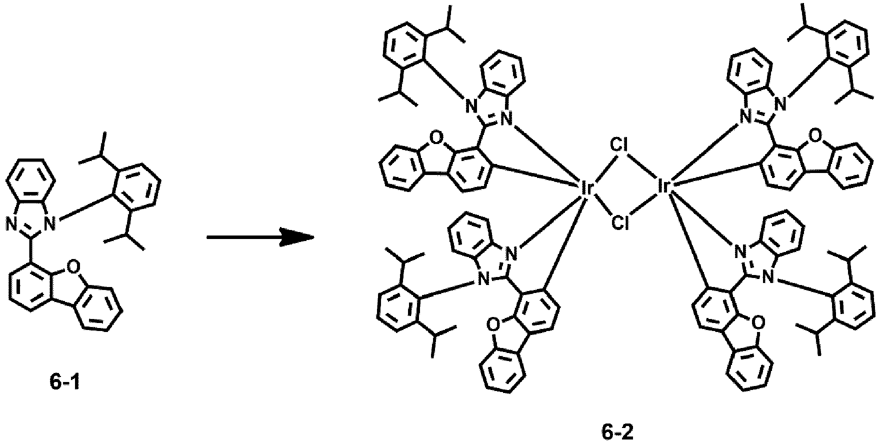

Compound 6-1 (7.0 g, 15.81 mmol) and iridium chloride (2.68 g, 7.60 mmol) were mixed with 50 mL of 2-ethoxyethanol and 20 mL of DI water, and then, the resultant mixture was stirred and heated at reflux for 24 hours. The temperature was then allowed to lower to room temperature. The resulting solid was separated by filtration and then washed thoroughly using DI water, methanol, and hexane, in the stated order. The obtained solid was dried in a vacuum oven to obtain Compound 6-2 (6.35 g, yield of 75%).

### Synthesis of Compound 6-3

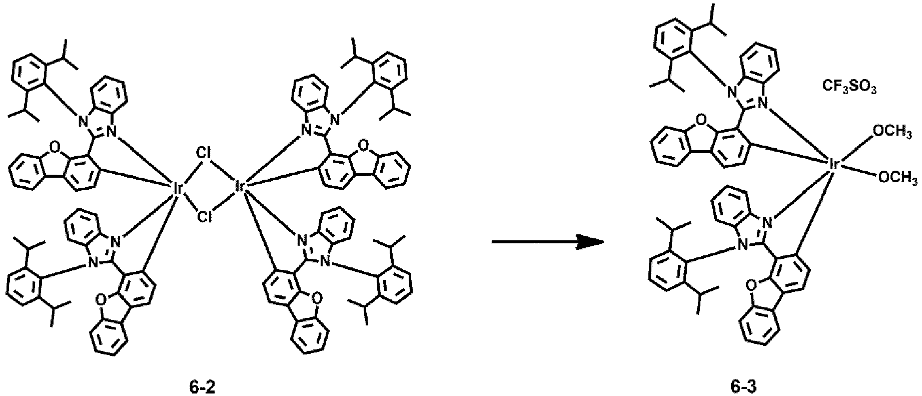

Compound 6-2 (5.8 g, 2.60 mmol) was mixed with 90 mL of methylene chloride (MC), and then silver trifluoromethanesulfonate (AgOTf) (1.4 g, 5.46 mmol) dissolved in 30 mL of methanol was added thereto. Thereafter, the reaction mixture was stirred at room temperature for 18 hours while light was blocked with aluminum foil, and then the resulting solid was removed by celite filtration, and the solvent was removed from the filtrate under a reduced pressure to obtain a solid (Compound 6-3). The obtained solid was used in the next reaction step without an additional purification process.

### Synthesis of Compound 6

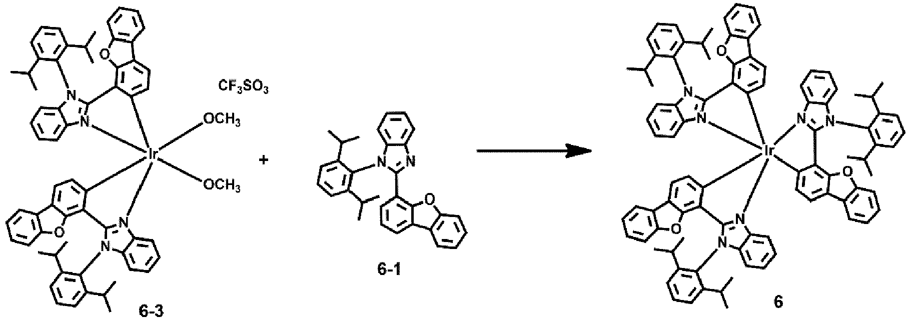

Compound 6-3 (6.45 g, 5.0 mmol) and Compound 6-1 (2.22 g, 5.0 mmol) were mixed with 80 mL of 2-ethoxyethanol and 80 mL of N,N-dimethylformamide, and then, the reaction mixture was stirred and heated under reflux at 120 °C for 24 hours. Then the temperature was allowed to lower to room temperature. The solvent was removed under a reduced pressure, and then, the product was purified by column chromatography using EA/hexane (1:8, w/w) to obtain Compound 6 (5.3 g, yield of 70%). Compound 6 was identified by high resolution mass spectrometry using matrix assisted laser desorption ionization (HRMS (MALDI)).

HRMS (MALDI) calculated for C₉₃H₈₁IrN₆O₃ : m/z: 1523.6033, found: 1523.4889.

### Synthesis Example 2 (Compound 12)

### Synthesis of Compound 12-1

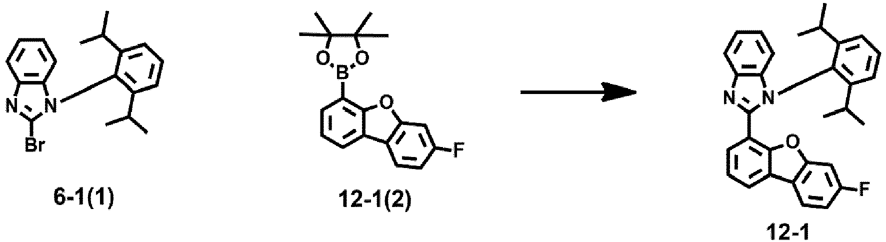

Compound 12-1 (4.05 g, yield of 65%) was obtained in a similar manner as was used to synthesize Compound 6-1 of Synthesis Example 1, except that Compound 12-1(2) (5.24 g, 16.80 mmol) was used instead of Compound 6-1(2).

### Synthesis of Compound 12

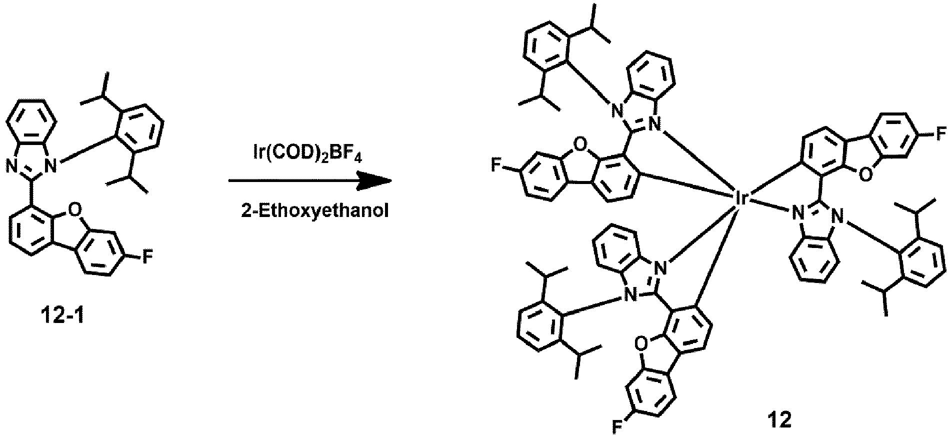

Compound 12-1 (1.96 g, 4.24 mmol) and bis(1,5-cyclooctadiene)iridium(I) tetrafluoroborate (Ir(COD)₂BF₄) (0.6 g, 1.21 mmol) were dissolved in 20 mL of 2-ethoxyethanol, and then the reaction mixture was stirred and heated at 160°C for 24 hours. The reaction temperature was then allowed to lower to room temperature. A solid was obtained by filtration and then dried. The product was purified by column chromatography using EA:hexane (1:7, w/w/) to obtain Compound 12 (0.85 g, yield of 45%).

HRMS (MALDI) calculated for C₉₃H₇₈F₃IrN₆O₃. m/z: 1576.5717, found: 1576.9442.

### Synthesis Example 3 (Compound 10)

### Synthesis of Compound 10-1

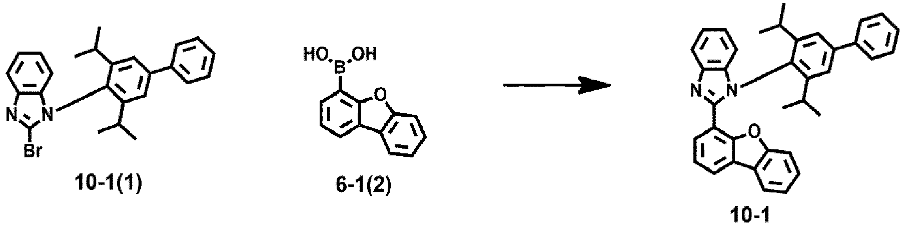

Compound 10-1 (4.93 g, yield of 66%) was obtained in a similar manner as was used to synthesize Compound 6-1 of Synthesis Example 1, except that Compound 10-1(1) (7.28 g, 16.80 mmol) was used instead of Compound 6-1(1).

### Synthesis of Compound 10

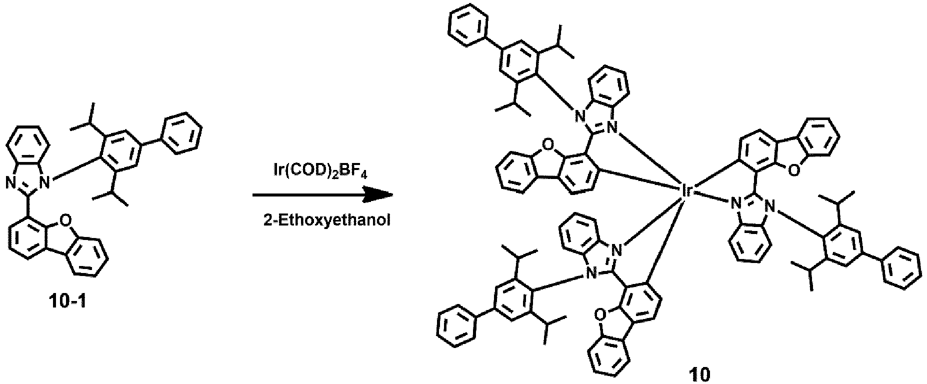

Compound 10 (0.95 g, yield of 45%) was obtained in a similar manner as was used to prepare Compound 12 in Synthesis Example 2, except that Compound 10-1 (2.21 g, 4.24 mmol) was used instead of Compound 12-1.

### Synthesis Example 4 (Compound 9)

### Synthesis of Compound 9-1

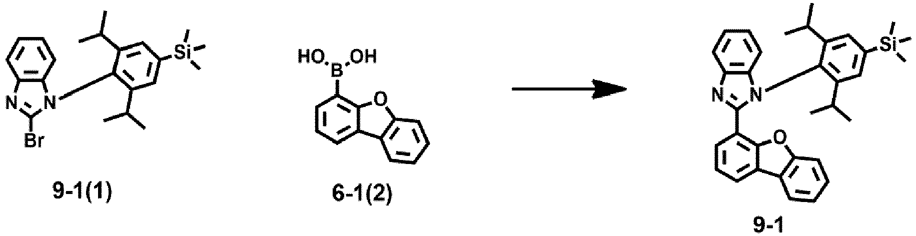

Compound 9-1 (yield of 62%) was obtained in a similar manner as was used to obtain Compound 6-1 of Synthesis Example 1, except that Compound 9-1(1) was used instead of Compound 6-1(1).

### Synthesis of Compound 9-2

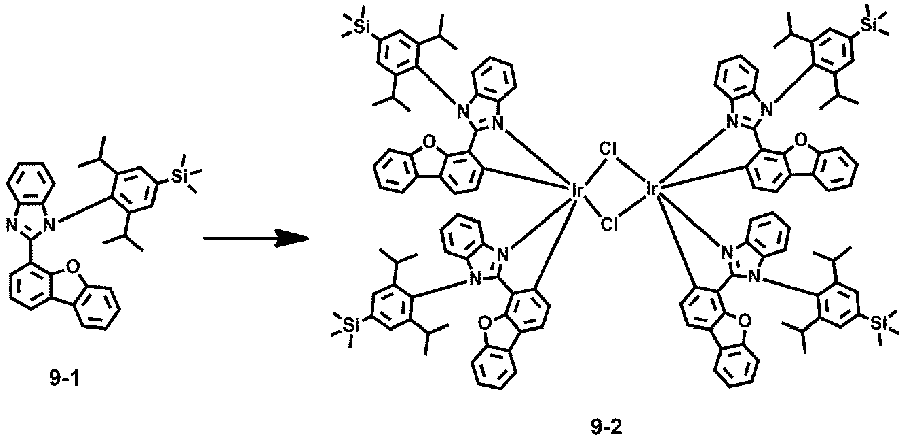

Compound 9-2 (yield of 72%) was obtained in a similar manner as was used to obtain Compound 6-2 of Synthesis Example 1, except that Compound 9-1 was used instead of Compound 6-1.

### Synthesis of Compound 9-3

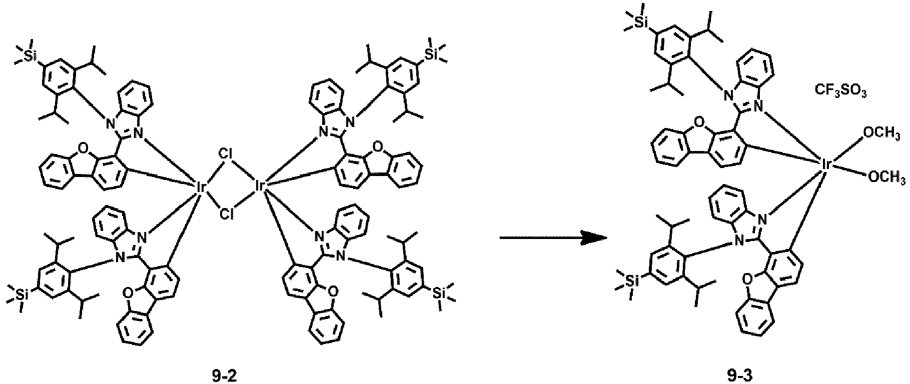

Compound 9-3 was obtained in a similar manner as was used to obtain Compound 6-3 of Synthesis Example 1, except that Compound 9-2 was used instead of Compound 6-2.

### Synthesis of Compound 9

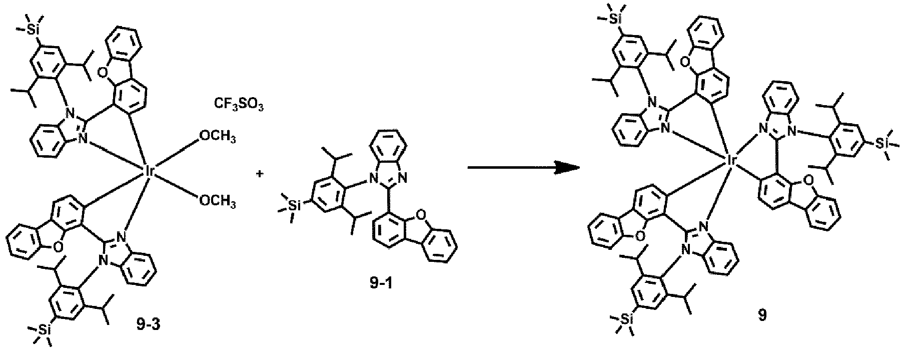

Compound 9 (yield of 68%) was obtained in a similar manner as was used to obtain Compound 6 of Synthesis Example 1, except that Compounds 9-3 and 9-1 were respectively used instead of Compounds 6-3 and 6-1.

HRMS (MALDI) calculated for C₁₀₂H₁₀₅IrN₆O₃Si₃. m/z: 1739.7219, found: 1740.0718.

### Synthesis Example 5 (Compound 8)

### Synthesis of Compound 8-1

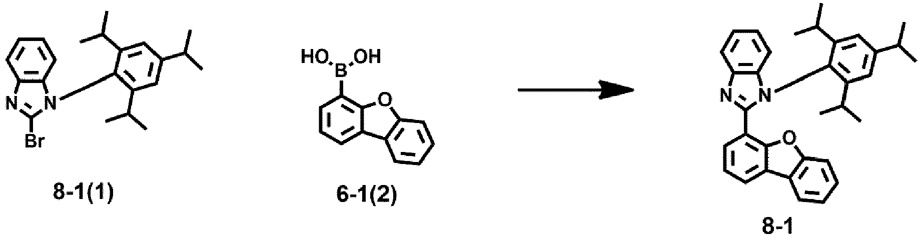

Compound 8-1 (yield of 64%) was obtained in a similar manner as was used to obtain Compound 6-1 of Synthesis Example 1, except that Compound 8-1(1) was used instead of Compound 6-1(1).

### Synthesis of Compound 8-2

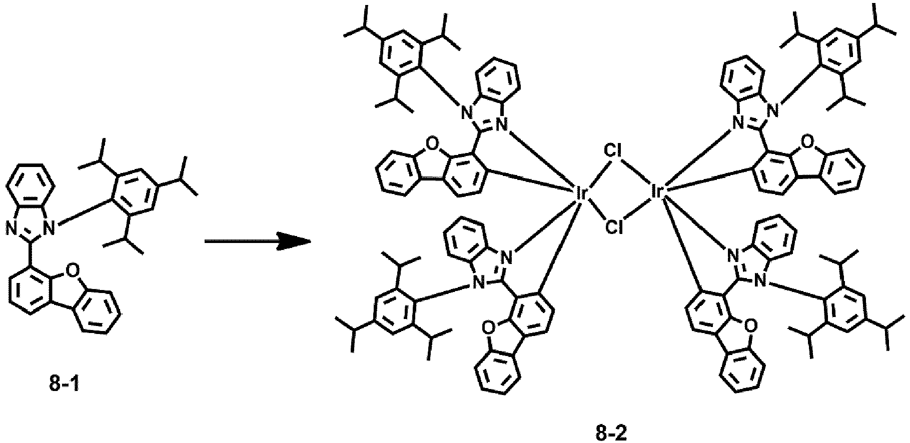

Compound 8-2 (yield of 75%) was obtained in a similar manner as was used to obtain Compound 6-2 of Synthesis Example 1, except that Compound 8-1 was used instead of Compound 6-1.

### Synthesis of Compound 8-3

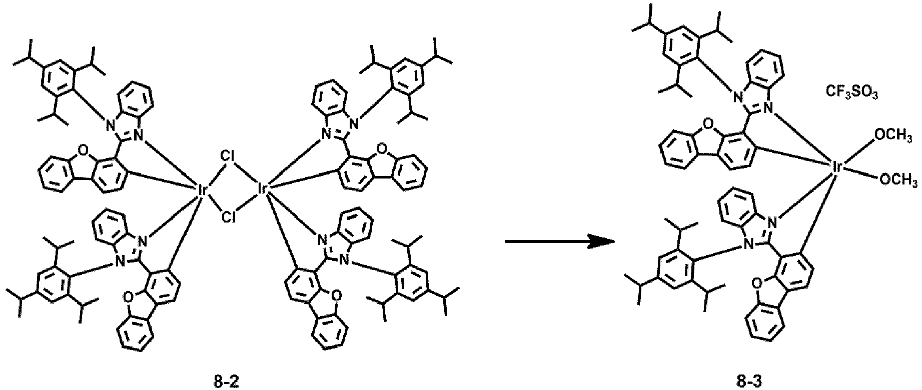

Compound 8-3 was obtained in a similar manner as was used to obtain Compound 6-3 of Synthesis Example 1, except that Compound 8-2 was used instead of Compound 6-2.

### Synthesis of Compound 8

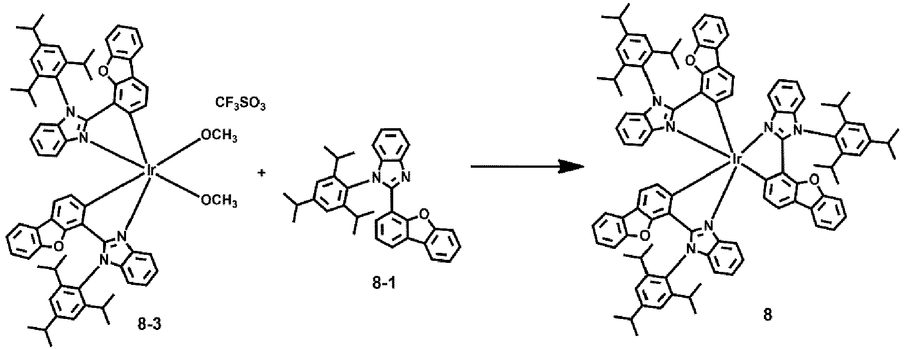

Compound 8 (yield of 65%) was obtained in a similar manner as was used to obtain Compound 6 of Synthesis Example 1, except that Compounds 8-3 and 8-1 were respectively used instead of Compounds 6-3 and 6-1.

HRMS (MALDI) calculated for C₁₀₂H₁₀₃IrN₆O₃. m/z: 1652.7721, found: 1653.0124.

### Synthesis Example 6 (Compound 23)

### Synthesis of Compound 23-1

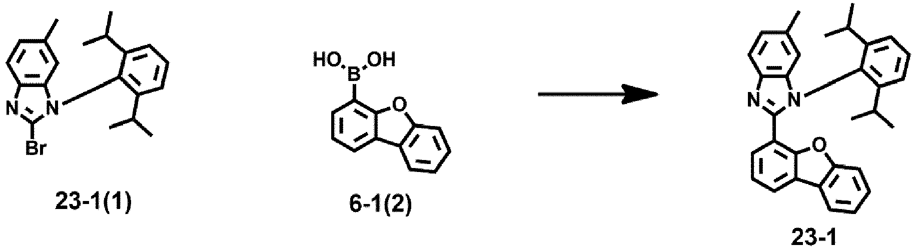

Compound 23-1 (yield of 58%) was obtained in a similar manner as was used to obtain Compound 6-1 of Synthesis Example 1, except that Compound 23-1(1) was used instead of Compound 6-1(1).

### Synthesis of Compound 23-2

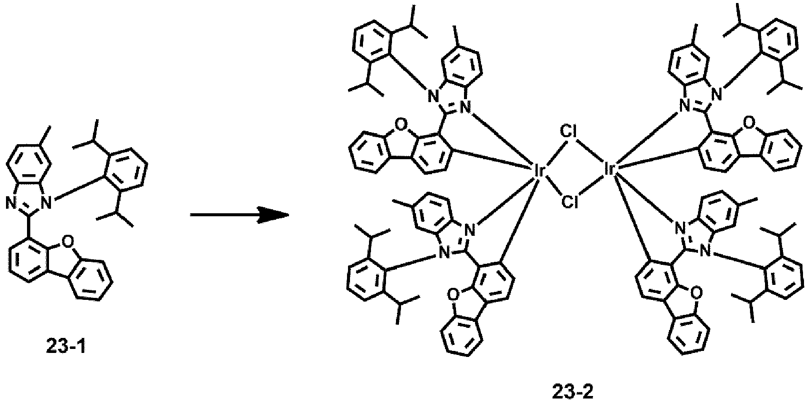

Compound 23-2 (yield of 69%) was obtained in a similar manner as was used to obtain Compound 6-2 of Synthesis Example 1, except that Compound 23-1 was used instead of Compound 6-1.

### Synthesis of Compound 23-3

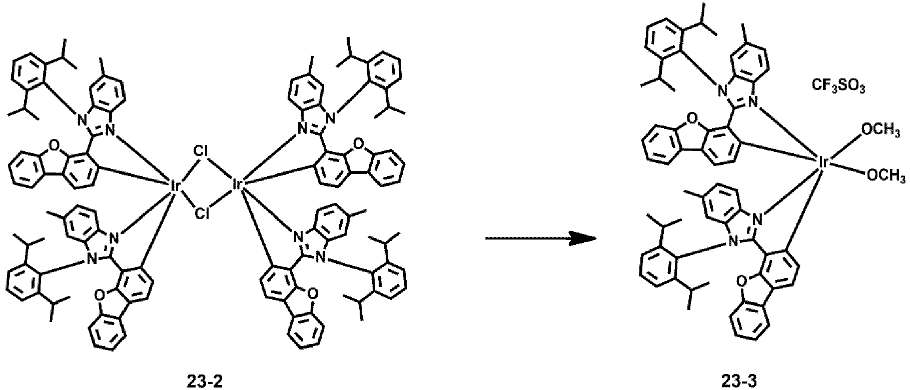

Compound 23-3 was obtained in a similar manner as was used to obtain Compound 6-3 of Synthesis Example 1, except that Compound 23-2 was used instead of Compound 6-2.

### Synthesis of Compound 23

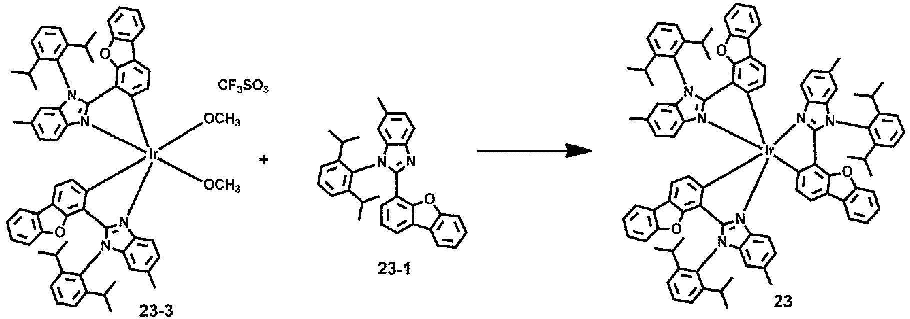

Compound 23 (yield of 55%) was obtained in a similar manner as was used to obtain Compound 6 of Synthesis Example 1, except that Compounds 23-3 and 23-1 were respectively used instead of Compounds 6-3 and 6-1.

HRMS (MALDI) calculated for C₉₆H₉₁IrN₆O₃: m/z: 1568.6782, found: 1568.9568.

### Example 1

As an anode, a glass substrate with ITO/Ag/ITO deposited thereon to a thickness of 70/1000/70 Å was cut to a size of 50 millimeters (mm) x 50 mm x 0.5 mm, sonicated with isopropyl alcohol and DI water each for 5 minutes, and then cleaned by exposure to ultraviolet rays and ozone for 30 minutes each. Then, the resultant glass substrate was loaded onto a vacuum deposition apparatus.

Compound HT3 and F6-TCNNQ were co-deposited by vacuum on the anode at the weight ratio of 98:2 to form a hole injection layer having a thickness of 100 Å, and Compound HT3 was vacuum-deposited on the hole injection layer to form a hole transport layer having a thickness of 1350 Å,and then, Compound H-H1 was deposited on the hole transport layer to form an electron-blocking layer having a thickness of 300 Å.

Then, Compound H-H1, Compound H-E43, and Compound 6 were co-deposited on the electron-blocking layer at a weight ratio of 57:38:5 to form an emission layer having a thickness of 400 Å.

Then, Compounds ET3 and ET-D1 were co-deposited on the emission layer at a 50:50 volume ratio to form an electron transport layer having a thickness of 350 Å, and LiF was vacuum-deposited on the electron transport layer to form an electron injection layer having a thickness of 1 nm. On the electron injection layer, Mg and Ag were co-deposited at a weight ratio of 90:10 to form a cathode having a thickness of 120 Å, thereby completing the manufacture of an organic light-emitting device.

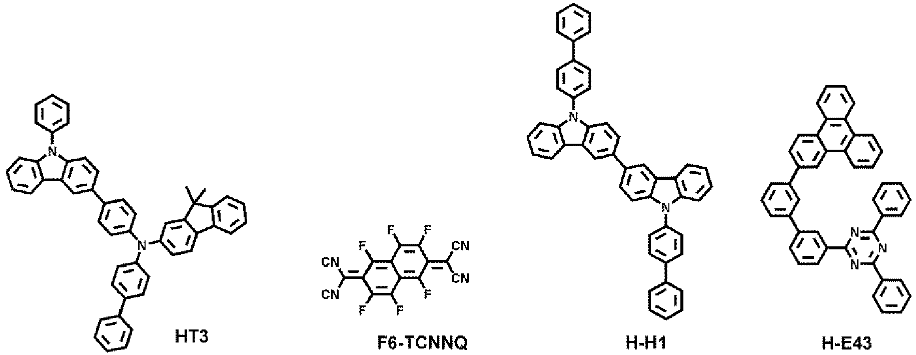

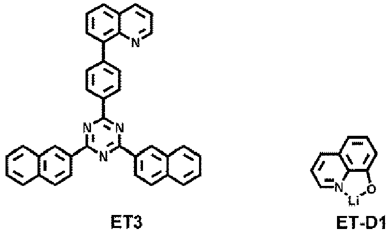

### Examples 2 to 6 and Comparative Examples A to C

Organic light-emitting devices were manufactured in a similar manner as was used in Example 1, except that the compounds shown in Table 2 were used instead of Compound 6 as a dopant in the formation of an emission layer.

### Evaluation Example 1

For each of the organic light-emitting devices manufactured in Examples 1 to 6 and Comparative Examples A to C, the lifetime (LT₉₇, relative %) at the target color coordinate CIEx = 0.245, the maximum emission wavelength (nm), and FWHM (nm) of emission peaks of the electroluminescence (EL) spectrum were evaluated, and results thereof are shown in Table 2. Current-voltmeter (Keithley 2400) and luminance meter (Minolta Cs-1000A) were used as evaluation devices, and lifespan (LT₉₇) (at 15,000 candela per square meter, cd/m²) was evaluated as time (hr) to achieve 97% of luminance compared to 100% of initial luminance.

**Table 2**

| | Dopant in emission layer Compound No. | Maximum emission wavelength (nm) | FWHM (nm) | LT₉₇ (%) (Relative value, %) |
|---|---|---|---|---|
| Example 1 | 6 | 526 | 62.3 | 145 |
| Example 2 | 8 | 526 | 62.7 | 130 |
| Example 3 | 9 | 526 | 62.7 | 125 |
| Example 4 | 10 | 527 | 63.0 | 170 |
| Example 5 | 12 | 525 | 62.1 | 130 |
| Example 6 | 23 | 528 | 63.5 | 127 |
| Comparative Example A | A | 532 | 75.0 | 100 |
| Comparative Example B | B | 531 | 66.2 | 92 |
| Comparative Example C | C | 536 | 63.4 | 86 |

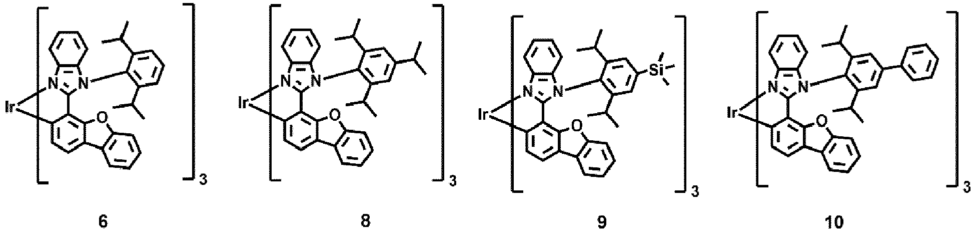

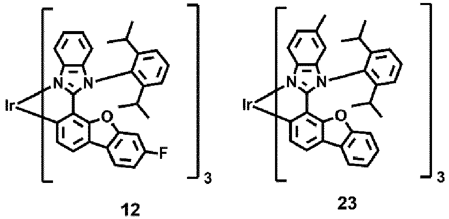

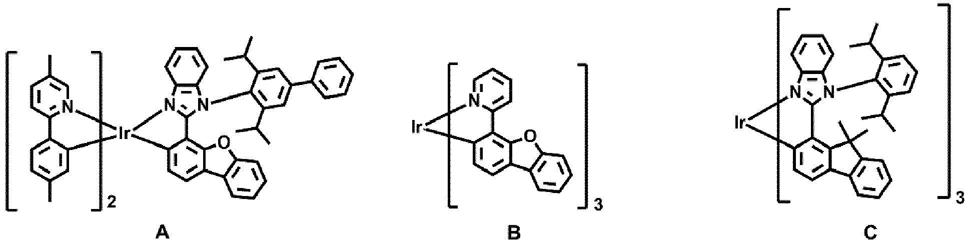

It was confirmed from Table 2 that each of the organic light-emitting devices of Examples 1 to 6 has excellent lifespan characteristics while emitting a green light with a relatively narrow FWHM, compared to the organic light-emitting devices of Comparative Examples A to C.

Since the organometallic compound has excellent thermal stability and/or electric characteristics, an electronic device, for example, an organic light-emitting device, including at least one of the organometallic compounds may have an improved lifespan characteristic, and a high-quality electronic device can be manufactured using the organic light emitting device.

## Claims

1. An organometallic compound represented by Formula 1:
Formula 1 M(L₁)₃
wherein, in Formula 1,
M is iridium, and
L₁ is a ligand represented by Formula 2-1 or a ligand represented by Formula 2-2, 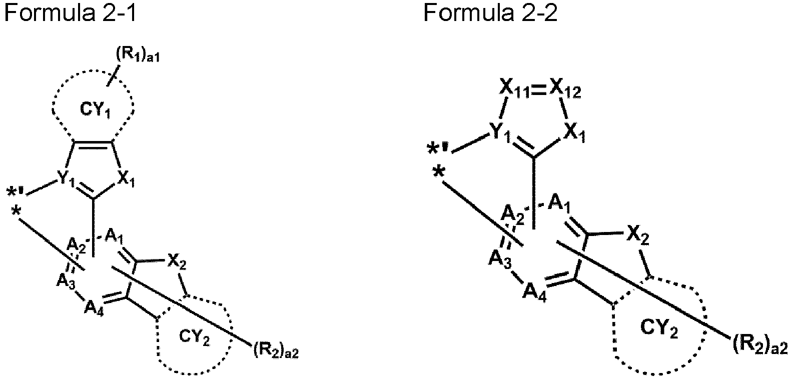
wherein, in Formulae 2-1 and 2-2,
Y₁ is N,
X₁₁ is C(R₁₁) or N,
X₁₂ is C(R₁₂) or N,
X₁ is O, S, or N-{(T₁)_{b1}-(Z₁)_{c1}},
T₁ is a single bond, a C₁-C₂₀ alkylene group that is unsubstituted or substituted with at least one R₁₀ₐ, a C₅-C₃₀ carbocyclic group that is unsubstituted or substituted with at least one R₁₀ₐ, or a C₁-C₃₀ heterocyclic group that is unsubstituted or substituted with at least one R₁₀ₐ,
b1 is an integer from 1 to 5,
c1 is an integer from 0 to 20,
A₁ to A₄ are each independently C or N, wherein one of A₁ to A₄ is C bonded to an adjacent 5-membered ring, and another of A₁ to A₄ is C bonded to M in Formula 1,
X₂ is O, S, Se, Si(R₂₉ₐ)(R_{29b}), or Ge(R₂₉ₐ)(R_{29b}),
ring CY₁ and ring CY₂ are each independently a C₅-C₃₀ carbocyclic group or a C₁-C₃₀ heterocyclic group,
R₁, R₁₁, R₁₂, R₂, R₂₉ₐ, R_{29b}, and Z₁ are each independently: hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₇-C₆₀ alkyl aryl group, a substituted or unsubstituted C₇-C₆₀ aryl alkyl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₂-C₆₀ alkyl heteroaryl group, a substituted or unsubstituted C₂-C₆₀ heteroaryl alkyl group, a substituted or unsubstituted C₁-C₆₀ heteroaryloxy group, a substituted or unsubstituted C₁-C₆₀ heteroarylthio group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), or - P(Q₈)(Q₉),
a1 and a2 are each independently an integer from 0 to 20,
two or more of a plurality of R₁ are optionally linked to each other to form a C₅-C₃₀ carbocyclic group that is unsubstituted or substituted with at least one R₁₀ₐ, or a C₁-C₃₀ heterocyclic group that is unsubstituted or substituted with at least one R₁₀ₐ,
two or more of a plurality of R₂ are optionally linked to each other to form a C₅-C₃₀ carbocyclic group that is unsubstituted or substituted with at least one R₁₀ₐ, or a C₁-C₃₀ heterocyclic group that is unsubstituted or substituted with at least one R₁₀ₐ,
R₁ and R₂ are optionally linked to each other to form a C₅-C₃₀ carbocyclic group that is unsubstituted or substituted with at least one R₁₀ₐ, or a C₁-C₃₀ heterocyclic group that is unsubstituted or substituted with at least one R₁₀ₐ,
R₁₀ₐ is as described in connection with R₂,
* and *' each indicates a binding site to M in Formula 1, and
at least one substituent of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₁-C₆₀ alkylthio group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₇-C₆₀ alkyl aryl group, the substituted C₇-C₆₀ aryl alkyl group, the substituted C₆-C₆₀ aryloxy group, the substituted C₆-C₆₀ arylthio group, the substituted C₁-C₆₀ heteroaryl group, the substituted C₂-C₆₀ alkyl heteroaryl group, the substituted C₂-C₆₀ heteroaryl alkyl group, the substituted C₁-C₆₀ heteroaryloxy group, the substituted C₁-C₆₀ heteroarylthio group, the substituted monovalent non-aromatic condensed polycyclic group, and the substituted monovalent non-aromatic condensed heteropolycyclic group is:
deuterium, -F, -Cl, -Br, -I, -SF₅, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, or a C₁-C₆₀ alkylthio group;
a C₁-C₆₀ alkyl group, a C₂-C₆₀ alkenyl group, a C₂-C₆₀ alkynyl group, a C₁-C₆₀ alkoxy group, or a C₁-C₆₀ alkylthio group, each substituted with at least one of deuterium, -F, -Cl, -Br, -I, -SF₅, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇-C₆₀ alkyl aryl group, a C₆-C₆₀ aryloxy group, a C₆-C₆₀ arylthio group, a C₁-C₆₀ heteroaryl group, a C₂-C₆₀ alkyl heteroaryl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), - Ge(Q₁₃)(Q₁₄)(Q₁₅), -B(Q₁₆)(Q₁₇), -P(=O)(Q₁₈)(Q₁₉), -P(Q₁₈)(Q₁₉), or a combination thereof;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇-C₆₀ alkyl aryl group, a C₆-C₆₀ aryloxy group, a C₆-C₆₀ arylthio group, a C₁-C₆₀ heteroaryl group, a C₂-C₆₀ alkyl heteroaryl group, a C₁-C₆₀ heteroaryloxy group, a C₁-C₆₀ heteroarylthio group, a monovalent non-aromatic condensed polycyclic group, or a monovalent non-aromatic condensed heteropolycyclic group, each unsubstituted or substituted with at least one of deuterium, -F, -Cl, -Br, -I, -SF₅, -CD₃, -CD₂H, -CDH₂, - CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₁-C₆₀ alkylthio group, C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇-C₆₀ alkyl aryl group, a C₇-C₆₀ aryl alkyl group, a C₆-C₆₀ aryloxy group, a C₆-C₆₀ arylthio group, a C₁-C₆₀ heteroaryl group, a C₂-C₆₀ alkyl heteroaryl group, a C₂-C₆₀ heteroaryl alkyl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -N(Q₂₁)(Q₂₂), - Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉), P(Q₂₈)(Q₂₉), or a combination thereof;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), - P(=O)(Q₃₈)(Q₃₉), or -P(Q₃₈)(Q₃₉); or
a combination thereof,
wherein Q₁ to Q₉, Q₁₁ to Q₁₉, Q₂₁ to Q₂₉, and Q₃₁ to Q₃₉ are each independently hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₇-C₆₀ alkyl aryl group, a substituted or unsubstituted C₇-C₆₀ aryl alkyl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₂-C₆₀ alkyl heteroaryl group, a substituted or unsubstituted C₂-C₆₀ heteroaryl alkyl group, a substituted or unsubstituted C₁-C₆₀ heteroaryloxy group, a substituted or unsubstituted C₁-C₆₀ heteroarylthio group, a substituted or unsubstituted monovalent non-aromatic condensed polycyclic group, or a substituted or unsubstituted monovalent non-aromatic condensed heteropolycyclic group.

2. The organometallic compound of claim 1, wherein
X₁ is N-{(T₁)_{b1}-(Z₁)_{c1}}, and
T₁ is a single bond, a C₁-C₁₀ alkylene group that is unsubstituted or substituted with at least one R₁₀ₐ, a C₅-C₂₀ carbocyclic group that is unsubstituted or substituted with at least one R₁₀ₐ, or a C₁-C₂₀ heterocyclic group that is unsubstituted or substituted with at least one R₁₀ₐ.

3. The organometallic compound of claims 1 or 2, wherein
A₁ is C bonded to an adjacent 5-membered ring, and A₂ is C bonded to M in Formula 1; or
A₃ is C bonded to an adjacent 5-membered ring, and A₂ is C bonded to M in Formula 1.

4. The organometallic compound of any of claims 1-3, wherein
ring CY₁ is a benzene group, a naphthalene group, an anthracene group, a phenanthrene group, a pyridine group, a pyrimidine group, a pyridazine group, a pyrazine group, a quinoline group, an isoquinoline group, a quinoxaline group, a quinazoline group, a phenanthridine group, a phenanthroline group, a benzoquinoline group, or a benzoisoquinoline group; and/or
wherein ring CY₂ is a benzene group, a naphthalene group, an anthracene group, a phenanthrene group, a pyridine group, a pyrimidine group, a pyridazine group, a pyrazine group, a quinoline group, an isoquinoline group, a quinoxaline group, a quinazoline group, a phenanthridine group, a phenanthroline group, a benzoquinoline group, a benzoisoquinoline group, a benzene group to which a cyclohexane group is condensed, a benzene group to which an adamantane group is condensed, a benzene group to which a norbornane group is condensed, a pyridine group to which a cyclohexane group is condensed, a pyridine group to which an adamantane group is condensed, a pyridine group to which a norbornane group is condensed, a pyrrole group, a cyclopentadiene group, a silole group, a thiophene group, a furan group, a selenophene group, an indole group, an indene group, a benzosilole group, a benzothiophene group, a benzofuran group, a benzoselenophene group, a carbazole group, a fluorene group, a dibenzosilole group, a dibenzothiophene group, a dibenzofuran group, a dibenzoselenophene group, an azaindole group, an azaindene group, an azabenzosilole group, an azabenzothiophene group, an azabenzofuran group, an azabenzoselenophene group, an azacarbazole group, an azafluorene group, an azadibenzosilole group, an azadibenzothiophene group, an azadibenzofuran group, or an azadibenzoselenophene group.

5. The organometallic compound of any of claims 1-4, wherein
R₁, R₁₁, R₁₂, R₂, R₂₉ₐ, R_{29b}, and Z₁ are each independently:
hydrogen, deuterium, -F, or a cyano group;
a C₁-C₂₀ alkyl group, a C₃-C₁₀ cycloalkyl group, a phenyl group, a naphthyl group, a pyridinyl group, a furanyl group, a thiophenyl group, a benzofuranyl group, a benzothiophenyl group, a dibenzofuranyl group, or a dibenzothiophenyl group, each unsubstituted or substituted with at least one of deuterium, -F, a cyano group, a C₁-C₂₀ alkyl group, a deuterated C₁-C₂₀ alkyl group, a fluorinated C₁-C₂₀ alkyl group, a C₃-C₁₀ cycloalkyl group, a deuterated C₃-C₁₀ cycloalkyl group, a fluorinated C₃-C₁₀ cycloalkyl group, a (C₁-C₂₀ alkyl)C₃-C₁₀ cycloalkyl group, a phenyl group, a deuterated a phenyl group, a fluorinated a phenyl group, a (C₁-C₂₀ alkyl)phenyl group, a naphthyl group, a pyridinyl group, a furanyl group, a thiophenyl group, a benzofuranyl group, a benzothiophenyl group, a dibenzofuranyl group, a dibenzothiophenyl group, - Si(Q33)(Q34)(Q35), -Ge(Q33)(Q34)(Q35), or a combination thereof; or
-Si(Q₃)(Q₄)(Q₅), or -Ge(Q₃)(Q₄)(Q₅).

6. The organometallic compound of any of claims 1-5, wherein a2 is not 0 and R₂ is not hydrogen; and/or
comprising deuterium, a fluoro group, a deuterated C₁-C₂₀ alkyl group, a fluorinated C₁-C₂₀ alkyl group, -Si(Q₃)(Q₄)(Q₅), -Ge(Q₃)(Q₄)(Q₅), or a combination thereof.

7. The organometallic compound of any of claims 1-6, wherein
X₁ is N-{(T₁)_{b1}-(Z₁)_{c1}}, and
X₁ satisfies at least one of Condition A and Condition B, or X₁ satisfies Condition C:
Condition A
T₁ is a C₅-C₃₀ carbocyclic group that is unsubstituted or substituted with at least one R₁₀ₐ, or a C₁-C₃₀ heterocyclic group that is unsubstituted or substituted with at least one R₁₀ₐ;
Condition B
Z₁ is a substituted or unsubstituted C₆-C₆₀ aryl group, a substituted or unsubstituted C₁-C₆₀ heteroaryl group, a substituted or unsubstituted monovalent non-aromatic condensed polycyclic group, or a substituted or unsubstituted monovalent non-aromatic condensed heteropolycyclic group;
Condition C
T₁ is a single bond, or a C₁-C₂₀ alkylene group that is unsubstituted or substituted with at least one R₁₀ₐ, and
Z₁ and R₁₀ₐ are each independently hydrogen, deuterium, -F, a cyano group, a C₁-C₂₀ alkyl group, a deuterated C₁-C₂₀ alkyl group, a fluorinated C₁-C₂₀ alkyl group, -Si(Q₃₃)(Q₃₄)(Q₃₅), -Ge(Q₃₃)(Q₃₄)(Q₃₅), or a combination thereof.

8. The organometallic compound of any of claims 1-7, wherein the organometallic compound satisfies at least one of Condition 1 to Condition 3:
Condition 1
a1 is not 0, and
R₁ comprises at least one of deuterium, a fluoro group, a cyano group, a deuterated C₁-C₂₀ alkyl group, a fluorinated C₁-C₂₀ alkyl group, a phenyl group, a deuterated phenyl group, a fluorinated phenyl group, a (C₁-C₂₀ alkyl)phenyl group, - Si(Q₃)(Q₄)(Q₅), -Ge(Q₃)(Q₄)(Q₅), or a combination thereof;
Condition 2
a2 is not 0, and
R₂ comprises at least one of deuterium, a fluoro group, a cyano group, a deuterated C₁-C₂₀ alkyl group, a fluorinated C₁-C₂₀ alkyl group, a phenyl group, a deuterated phenyl group, a fluorinated phenyl group, a (C₁-C₂₀ alkyl)phenyl group, - Si(Q₃)(Q₄)(Q₅), -Ge(Q₃)(Q₄)(Q₅), or a combination thereof;
Condition 3
X₁ is N-{(T₁)_{b1}-(Z₁)_{c1}}, and
a group represented by *-(T₁)_{b1}-(Z₁)_{c1} comprises at least one of deuterium, a fluoro group, a cyano group, a deuterated C₁-C₂₀ alkyl group, a fluorinated C₁-C₂₀ alkyl group, a phenyl group, a deuterated phenyl group, a fluorinated phenyl group, a (C₁-C₂₀ alkyl)phenyl group, -Si(Q₃)(Q₄)(Q₅), -Ge(Q₃)(Q₄)(Q₅), or a combination thereof.

9. The organometallic compound of any of claims 1-8, wherein
a group represented by 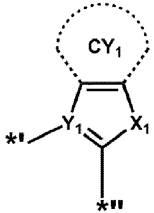
in Formula 2-1 is a group represented by one of Formulae CY1-1 to CY1-20: 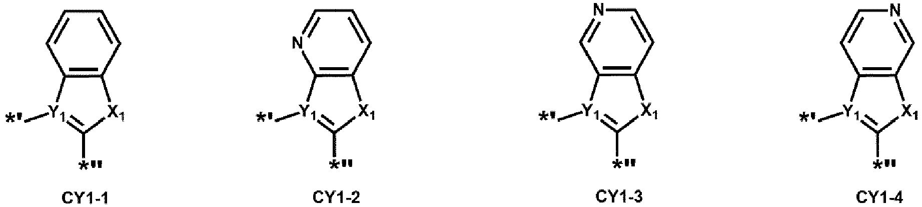 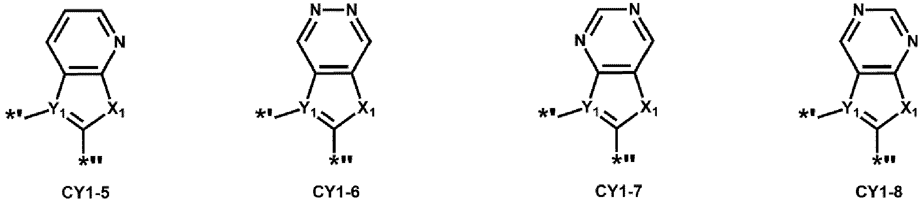 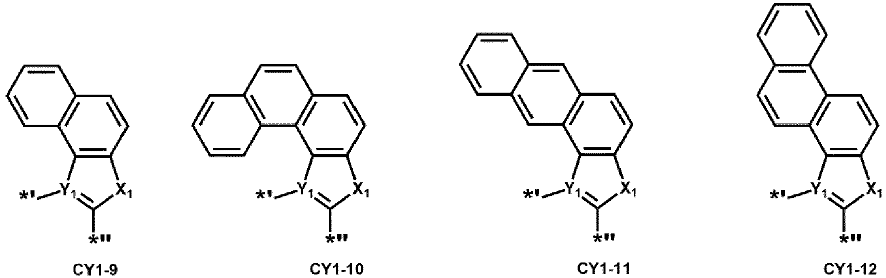 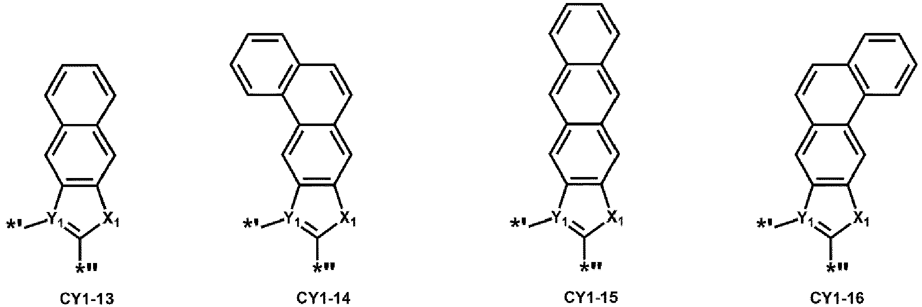 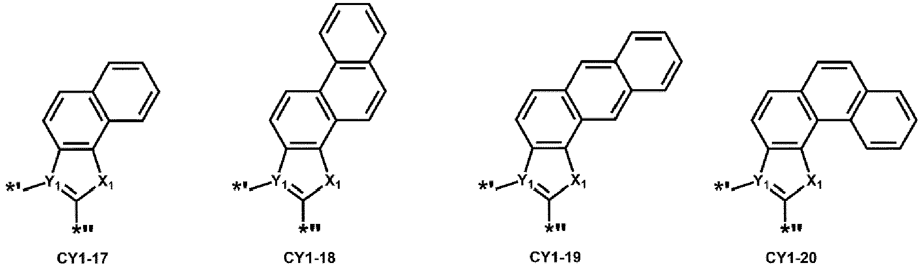
wherein, in Formulae CY1-1 to CY1-20,
Y₁ and X₁ are each as described in claim 1,
*' indicates a binding site to M in Formula 1, and
*" indicates a binding site to one of A₁ to A₄ of Formula 2-1; and/or
wherein
a group represented by 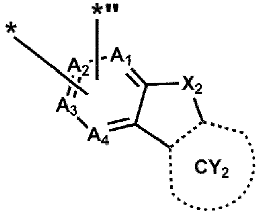 is a group represented by one of Formulae CY2(1) to CY2(6): 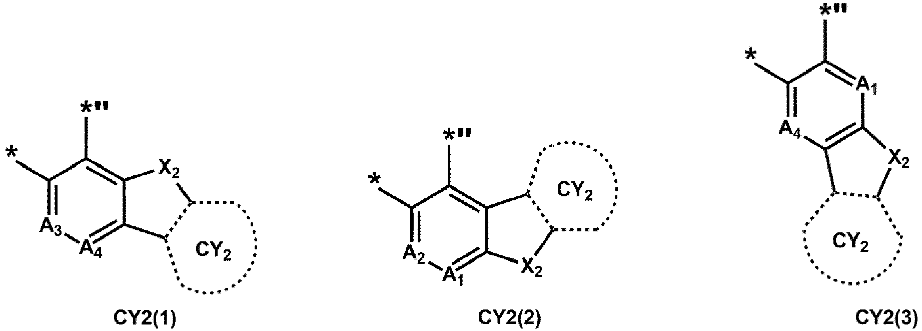 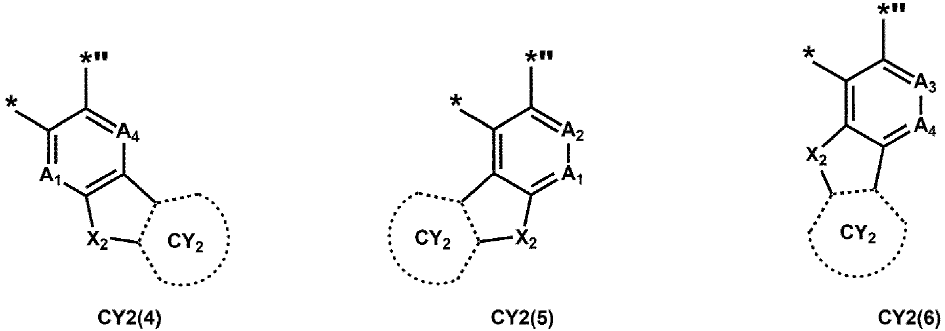
wherein, in Formulae CY2(1) to CY2(6),
X₂ and ring CY₂ are as described in claim 1,
A₁ to A₄ are each independently C or N,
*" is a binding site to a neighboring 5-membered ring, and
* is a binding site to M in Formula 1.

10. The organometallic compound of any of claims 1-9, wherein a group represented by 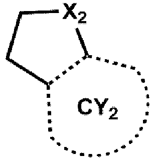 is a group represented by one of Formulae CY2-1 to CY2-20: 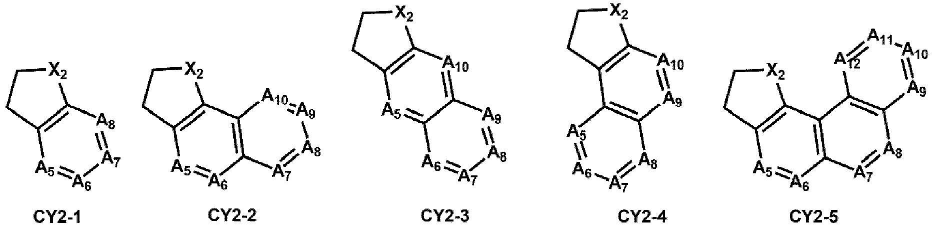 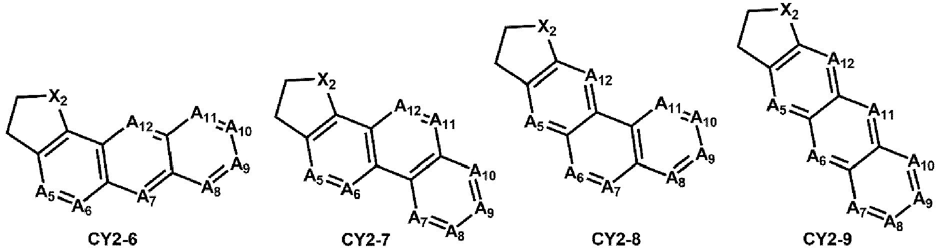 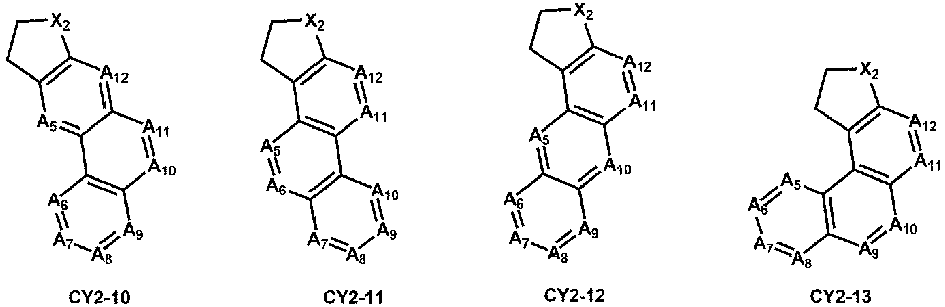 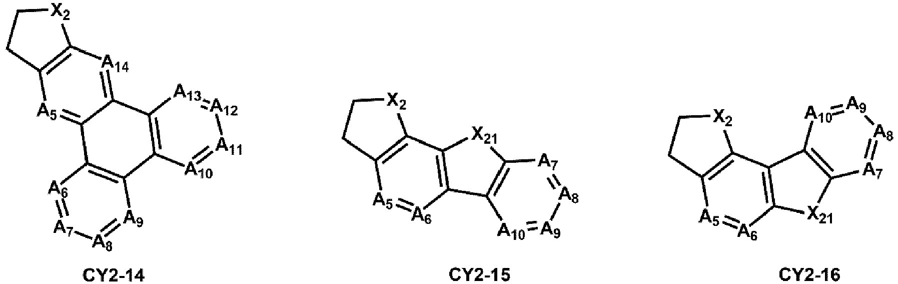 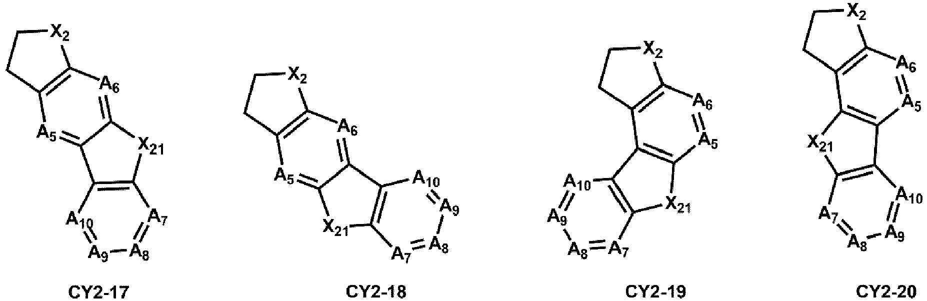 wherein, in Formulae CY2-1 to CY2-20,
X₂ is as described in claim 1,
X₂₁ is O, S, Se, N(R_{29c}), C(R_{29c})(R_{29d}), Si(R_{29c})(R_{29d}), or Ge(R_{29c})(R_{29d}),
R_{29c} and R_{29d} are each as described in connection with R₂₉ₐ in claim 1, and
A₅ to A₁₄ are each independently C or N.

11. The organometallic compound of any of claims 1-10, wherein a group represented by 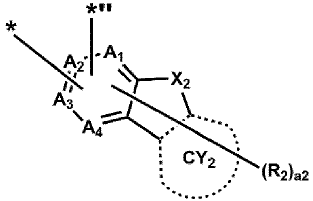 is a group represented by one of Formulae CY2A to CY2D: 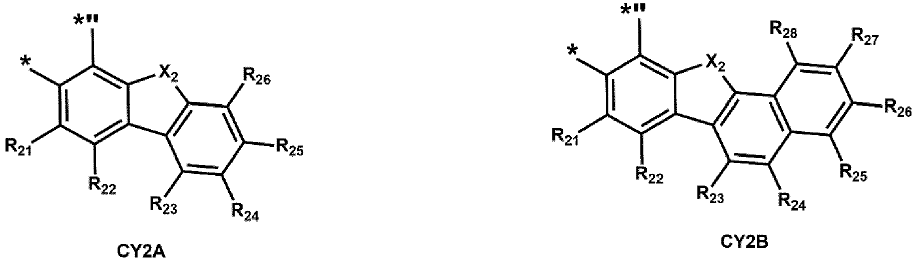 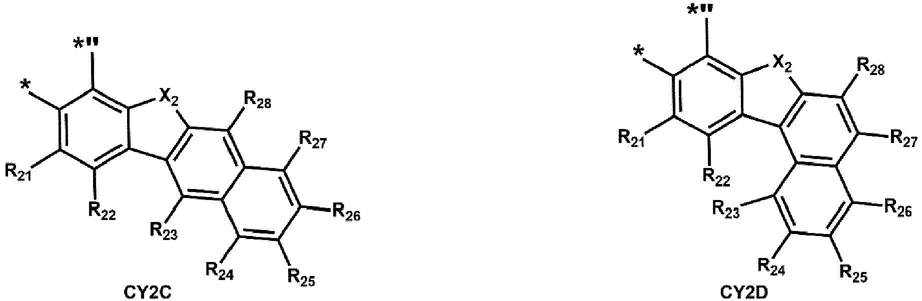 wherein, in Formulae CY2A to CY2D,
X₂ is as described in claim 1,
R₂₁ to R₂₈ are each as described in connection with R₂ in claim 1,
*" is a binding site to a neighboring 5-membered ring, and
* is a binding site to M in Formula 1.

12. An organic light-emitting device, comprising:
a first electrode,
a second electrode, and
an organic layer located between the first electrode and the second electrode,
wherein the organic layer comprises an emission layer, and
wherein the organic layer further comprises at least one of the organometallic compound of any of claims 1-11;
preferably wherein
the first electrode is an anode,
the second electrode is a cathode,
the organic layer further comprises a hole transport region located between the first electrode and the emission layer, and an electron transport region located between the emission layer and the second electrode, wherein
the hole transport region comprises a hole injection layer, a hole transport layer, an electron-blocking layer, a buffer layer, or a combination thereof, and
the electron transport region comprises a hole-blocking layer, an electron transport layer, an electron injection layer, or a combination thereof.

13. The organic light-emitting device of claim 12, wherein the emission layer comprises the at least one organometallic compound;
preferably wherein the emission layer emits a green light.

14. The organic light-emitting device of claim 13, wherein
the emission layer further comprises a host, and
an amount of the host in the emission layer is greater than an amount of the organometallic compound in the emission layer.

15. An electronic apparatus, comprising the organic light-emitting device of any of claims 12-14.

## Patentansprüche

1. Organometallische Verbindung dargestellt durch Formel 1:
Formel 1 M(L₁)₃
wobei in Formel 1
M Iridium ist, und
L₁ ein Ligand dargestellt durch Formel 2-1 oder ein Ligand dargestellt durch Formel 2-2 ist, 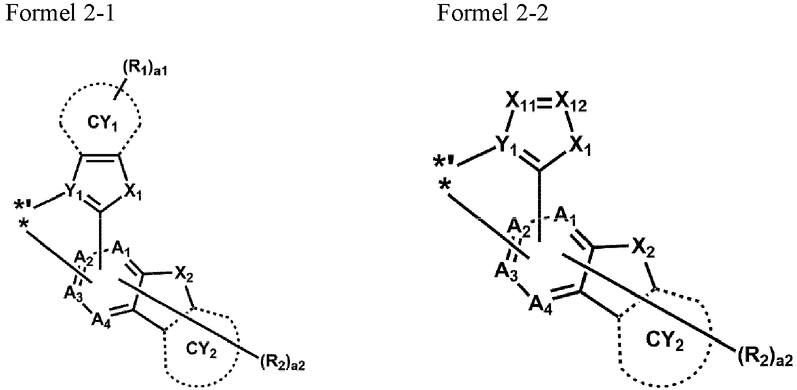
wobei in Formel 2-1 und 2-2
Y₁ N ist,
X₁ C(R₁₁) oder N ist,
X₁₂ C(R₁₂) oder N ist,
X₁ O, S oder N-{(T₁)_{b1}-(Z₁)_{c1}} ist,
T₁ eine Einfachbindung, eine C₁-C₂₀-Alkylengruppe, die unsubstituiert oder substituiert mit zumindest einem R₁₀ₐ ist, eine carbocyclische C₅-C₃₀-Gruppe, die unsubstituiert oder substituiert mit zumindest einem R₁₀ₐ ist, oder eine heterocyclische C₁-C₃₀-Gruppe, die unsubstituiert oder substituiert mit zumindest einem R₁₀ₐ ist, ist,
b 1 eine ganze Zahl von 1 bis 5 ist,
c1 eine ganze Zahl von 0 bis 20 ist,
A₁ bis A₄ jeweils unabhängig C oder N sind, wobei eines von A₁ bis A₄ C ist, das an einen angrenzenden 5-gliedrigen Ring gebunden ist, und ein anderes von A₁ bis A₄ C ist, das an M in Formel 1 gebunden ist,
X₂ O, S, Se, Si(R₂₉ₐ)(R_{29b}) oder Ge(R₂₉ₐ)(R_{29b}) ist,
Ring CY₁ und Ring CY₂ jeweils unabhängig eine carbocyclische C₅-C₃₀-Gruppe oder eine heterocyclische C₁-C₃₀-Gruppe sind,
R₁, R₁₁, R₁₂, R₂, R₂₉ₐ, R_{29b} und Z₁ jeweils unabhängig wie folgt sind: Wasserstoff, Deuterium, -F, -Cl, -Br, -I, -SF₅, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine substituierte oder unsubstituierte C₁-C₆₀-Alkylgruppe, eine substituierte oder unsubstituierte C₂-C₆₀-Alkenylgruppe, eine substituierte oder unsubstituierte C₂-C₆₀-Alkinylgruppe, eine substituierte oder unsubstituierte C₁-C₆₀-Alkoxygruppe, eine substituierte oder unsubstituierte C₁-C₆₀-Alkylthiogruppe, eine substituierte oder unsubstituierte C₃-C₁₀-Cycloalkylgruppe, eine substituierte oder unsubstituierte C₁-C₁₀-Heterocycloalkylgruppe, eine substituierte oder unsubstituierte C₃-C₁₀-Cycloalkenylgruppe, eine substituierte oder unsubstituierte C₁-C₁₀-Heterocycloalkenylgruppe, eine substituierte oder unsubstituierte C₆-C₆₀-Arylgruppe, eine substituierte oder unsubstituierte C₇-C₆₀-Alkylarylgruppe, eine substituierte oder unsubstituierte C₇-C₆₀-Arylalkylgruppe, eine substituierte oder unsubstituierte C₆-C₆₀-Aryloxygruppe, eine substituierte oder unsubstituierte C₆-C₆₀-Arylthiogruppe, eine substituierte oder unsubstituierte C₁-C₆₀-Heteroarylgruppe, eine substituierte oder unsubstituierte C₂-C₆₀-Alkylheteroarylgruppe, eine substituierte oder unsubstituierte C₂-C₆₀-Heteroarylalkylgruppe, eine substituierte oder unsubstituierte C₁-C₆₀-Heteroaryloxygruppe, eine substituierte oder unsubstituierte C₁-C₆₀-Heteroarylthiogruppe, eine substituierte oder unsubstituierte einwertige nichtaromatische kondensierte polycyclische Gruppe, eine substituierte oder unsubstituierte einwertige nichtaromatische kondensierte heteropolycyclische Gruppe, - N(Q₁)(Q₂), -Si(Q₃)(Q₄)(Q₅), -Ge(Q₃)(Q₄)(Q₅), -B(Q₆)(Q₇), -P(=O)(Q₈)(Q₉) oder -P(Q₈)(Q₉),
a1 und a2 jeweils unabhängig eine ganze Zahl von 0 bis 20 sind,
zwei oder mehr aus einer Vielzahl von R₁ optional miteinander verknüpft sind, um eine carbocyclische C₅-C₃₀-Gruppe, die unsubstituiert oder substituiert mit zumindest einem R₁₀ₐ ist, oder eine heterocyclische C₁-C₃₀-Gruppe, die unsubstituiert oder substituiert mit zumindest einem R₁₀ₐ ist, zu bilden,
zwei oder mehr aus einer Vielzahl von R₂ optional miteinander verknüpft sind, um eine carbocyclische C₅-C₃₀-Gruppe, die unsubstituiert oder substituiert mit zumindest einem R₁₀ₐ ist, oder eine heterocyclische C₁-C₃₀-Gruppe, die unsubstituiert oder substituiert mit zumindest einem R₁₀ₐ ist, zu bilden,
R₁ und R₂ optional miteinander verknüpft sind, um eine carbocyclische C₅-C₃₀-Gruppe, die unsubstituiert oder substituiert mit zumindest einem R₁₀ₐ ist, oder eine heterocyclische C₁-C₃₀-Gruppe, die unsubstituiert oder substituiert mit zumindest einem R₁₀ₐ ist, zu bilden,
R₁₀ₐ wie in Verbindung mit R₂ beschrieben ist,
* und *' jeweils eine Bindungsstelle zu M in Formel 1 angeben, und
zumindest ein Substituent der substituierten C₁-C₆₀-Alkylgruppe, der substituierten C₂-C₆₀-Alkenylgruppe, der substituierten C₂-C₆₀-Alkinylgruppe, der substituierten C₁-C₆₀-Alkoxygruppe, der substituierten C₁-C₆₀-Alkylthiogruppe, der substituierten C₃-C₁₀-Cycloalkylgruppe, der substituierten C₁-C₁₀-Heterocycloalkylgruppe, der substituierten C₃-C₁₀-Cycloalkenylgruppe, der substituierten C₁-C₁₀-Heterocycloalkenylgruppe, der substituierten C₆-C₆₀-Arylgruppe, der substituierten C₇-C₆₀-Alkylarylgruppe, der substituierten C₇-C₆₀-Arylalkylgruppe, der substituierten C₆-C₆₀-Aryloxygruppe, der substituierten C₆-C₆₀-Arylthiogruppe, der substituierten C₁-C₆₀-Heteroarylgruppe, der substituierten C₂-C₆₀-Alkylheteroarylgruppe, der substituierten C₂-C₆₀-Heteroarylalkylgruppe, der substituierten C₁-C₆₀-Heteroaryloxygruppe, der substituierten C₁-C₆₀-Heteroarylthiogruppe, der substituierten einwertigen nichtaromatischen kondensierten polycyclischen Gruppe und der substituierten einwertigen nichtaromatischen kondensierten heteropolycyclischen Gruppe wie folgt ist:
Deuterium, -F, -Cl, -Br, -I, -SF₅, -CD₃, -CD₂H, -CDH₂, -CF₃, -CF₂H, -CFH₂, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine C₁-C₆₀-Alkylgruppe, eine C₂-C₆₀-Alkenylgruppe, eine C₂-C₆₀-Alkinylgruppe, eine C₁-C₆₀-Alkoxygruppe oder eine C₁-C₆₀-Alkylthiogruppe,
eine C₁-C₆₀-Alkylgruppe, eine C₂-C₆₀-Alkenylgruppe, eine C₂-C₆₀-Alkinylgruppe, eine C₁-C₆₀-Alkoxygruppe oder eine C₁-C₆₀-Alkylthiogruppe, jeweils substituiert mit zumindest einem von Deuterium, -F, -Cl, -Br, -I, -SF₅, -CD₃, -CD₂H, -CDH₂, -CF₃, -CF₂H, -CFH₂, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₇-C₆₀-Alkylarylgruppe, einer C₆-C₆₀-Aryloxygruppe, einer C₆-C₆₀-Arylthiogruppe, einer C₁-C₆₀-Heteroarylgruppe, einer C₂-C₆₀-Alkylheteroarylgruppe, einer C₁-C₆₀-Heteroaryloxygruppe, einer C₁-C₆₀-Heteroarylthiogruppe, einer einwertigen nichtaromatischen kondensierten polycyclischen Gruppe, einer einwertigen nichtaromatischen kondensierten heteropolycyclischen Gruppe, -N(Q₁₁)(Q₁₂), -Si(Q₁₃)(Q₁₄)(Q₁₅), - Ge(Q₁₃)(Q₁₄)(Q₁₅), -B(Q₁₆)(Q₁₇), -P(=O)(Q₁₈)(Q₁₉), -P(Q₁₈)(Q₁₉) oder einer Kombination davon,
eine C₃-C₁₀-Cycloalkylgruppe, eine C₁-C₁₀-Heterocycloalkylgruppe, eine C₃-C₁₀-Cycloalkenylgruppe, eine C₁-C₁₀-Heterocycloalkenylgruppe, eine C₆-C₆₀-Arylgruppe, eine C₇-C₆₀-Alkylarylgruppe, eine C₆-C₆₀-Aryloxygruppe, eine C₆-C₆₀-Arylthiogruppe, eine C₁-C₆₀-Heteroarylgruppe, eine C₂-C₆₀-Alkylheteroarylgruppe, eine C₁-C₆₀-Heteroaryloxygruppe, eine C₁-C₆₀-Heteroarylthiogruppe, eine einwertige nichtaromatische kondensierte polycyclische Gruppe oder eine einwertige nichtaromatische kondensierte heteropolycyclische Gruppe, jeweils unsubstituiert oder substituiert mit zumindest einem von Deuterium, -F, -Cl, -Br, -I, -SF₅, -CD₃, - CD₂H, -CDH₂, -CF₃, -CF₂H, -CFH₂, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₆₀-Alkylgruppe, einer C₂-C₆₀-Alkenylgruppe, einer C₂-C₆₀-Alkinylgruppe, einer C₁-C₆₀-Alkoxygruppe, einer C₁-C₆₀-Alkylthiogruppe, C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₇-C₆₀-Alkylarylgruppe, einer C₇-C₆₀-Arylalkylgruppe, einer C₆-C₆₀-Aryloxygruppe, einer C₆-C₆₀-Arylthiogruppe, einer C₁-C₆₀-Heteroarylgruppe, einer C₂-C₆₀-Alkylheteroarylgruppe, einer C₂-C₆₀-Heteroarylalkylgruppe, einer C₁-C₆₀-Heteroaryloxygruppe, einer C₁-C₆₀-Heteroarylthiogruppe, einer einwertigen nichtaromatischen kondensierten polycyclischen Gruppe, einer einwertigen nichtaromatischen kondensierten heteropolycyclischen Gruppe, -N(Q₂₁)(Q₂₂), - Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉), P(Q₂₈)(Q₂₉) oder einer Kombination davon,
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), -P(=O)(Q₃₈)(Q₃₉) oder -P(Q₃₈)(Q₃₉); oder
eine Kombination davon,
wobei Q₁ bis Q₉, Q₁₁ bis Q₁₉, Q₂₁ bis Q₂₉ und Q₃₁ bis Q₃₉ jeweils unabhängig Wasserstoff, Deuterium, -F, -Cl, -Br, -I, -SF₅, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine substituierte oder unsubstituierte C₁-C₆₀-Alkylgruppe, eine substituierte oder unsubstituierte C₂-C₆₀-Alkenylgruppe, eine substituierte oder unsubstituierte C₂-C₆₀-Alkinylgruppe, eine substituierte oder unsubstituierte C₁-C₆₀-Alkoxygruppe, eine substituierte oder unsubstituierte C₁-C₆₀-Alkylthiogruppe, eine substituierte oder unsubstituierte C₃-C₁₀-Cycloalkylgruppe, eine substituierte oder unsubstituierte C₁-C₁₀-Heterocycloalkylgruppe, eine substituierte oder unsubstituierte C₃-C₁₀-Cycloalkenylgruppe, eine substituierte oder unsubstituierte C₁-C₁₀-Heterocycloalkenylgruppe, eine substituierte oder unsubstituierte C₆-C₆₀-Arylgruppe, eine substituierte oder unsubstituierte C₇-C₆₀-Alkylarylgruppe, eine substituierte oder unsubstituierte C₇-C₆₀-Arylalkylgruppe, eine substituierte oder unsubstituierte C₆-C₆₀-Aryloxygruppe, eine substituierte oder unsubstituierte C₆-C₆₀-Arylthiogruppe, eine substituierte oder unsubstituierte C₁-C₆₀-Heteroarylgruppe, eine substituierte oder unsubstituierte C₂-C₆₀-Alkylheteroarylgruppe, eine substituierte oder unsubstituierte C₂-C₆₀-Heteroarylalkylgruppe, eine substituierte oder unsubstituierte C₁-C₆₀-Heteroaryloxygruppe, eine substituierte oder unsubstituierte C₁-C₆₀-Heteroarylthiogruppe, eine substituierte oder unsubstituierte einwertige nichtaromatische kondensierte polycyclische Gruppe oder eine substituierte oder unsubstituierte einwertige nichtaromatische kondensierte heteropolycyclische Gruppe sind.

2. Organometallische Verbindung nach Anspruch 1, wobei
X₁ N-{(T₁)_{b1}-(Z₁)_{c1}} ist, und
T₁ eine Einfachbindung, eine C₁-C₁₀-Alkylengruppe, die unsubstituiert oder substituiert mit zumindest einem R₁₀ₐ ist, eine carbocyclische C₅-C₂₀-Gruppe, die unsubstituiert oder substituiert mit zumindest einem R₁₀ₐ ist, oder eine heterocyclische C₁-C₂₀-Gruppe, die unsubstituiert oder substituiert mit zumindest einem R₁₀ₐ ist, ist.

3. Organometallische Verbindung nach Anspruch 1 oder 2, wobei
A₁ C gebunden an einen angrenzenden 5-gliedrigen Ring ist, und A₂ C gebunden an M in Formel 1 ist; oder
A₃ C gebunden an einen angrenzenden 5-gliedrigen Ring ist, und A₂ C gebunden an M in Formel 1 ist.

4. Organometallische Verbindung nach einem der Ansprüche 1-3, wobei
Ring CY₁ eine Benzolgruppe, eine Naphthalingruppe, eine Anthracengruppe, eine Phenanthrengruppe, eine Pyridingruppe, eine Pyrimidingruppe, eine Pyridazingruppe, eine Pyrazingruppe, eine Chinolingruppe, eine Isochinolingruppe, eine Chinoxalingruppe, eine Chinazolingruppe, eine Phenanthridingruppe, eine Phenanthrolingruppe, eine Benzochinolingruppe oder eine Benzoisochinolingruppe ist, und/oder
wobei Ring CY₂ eine Benzolgruppe, eine Naphthalingruppe, eine Anthracengruppe, eine Phenanthrengruppe, eine Pyridingruppe, eine Pyrimidingruppe, eine Pyridazingruppe, eine Pyrazingruppe, eine Chinolingruppe, eine Isochinolingruppe, eine Chinoxalingruppe, eine Chinazolingruppe, eine Phenanthridingruppe, eine Phenanthrolingruppe, eine Benzochinolingruppe, eine Benzoisochinolingruppe, eine Benzolgruppe, an die eine Cyclohexangruppe kondensiert ist, eine Benzolgruppe, an die eine Adamantangruppe kondensiert ist, eine Benzolgruppe, an die eine Norbomangruppe kondensiert ist, eine Pyridingruppe, an die eine Cyclohexangruppe kondensiert ist, eine Pyridingruppe, an die eine Adamantangruppe kondensiert ist, eine Pyridingruppe, an die eine Norbomangruppe kondensiert ist, eine Pyrrolgruppe, eine Cyclopentadiengruppe, eine Silolgruppe, eine Thiophengruppe, eine Furangruppe, eine Selenophengruppe, eine Indolgruppe, eine Indengruppe, eine Benzosilolgruppe, eine Benzothiophengruppe, eine Benzofurangruppe, eine Benzoselenophengruppe, eine Carbazolgruppe, eine Fluorengruppe, eine Dibenzosilolgruppe, eine Dibenzothiophengruppe, eine Dibenzofurangruppe, eine Dibenzoselenophengruppe, eine Azaindolgruppe, eine Azaindengruppe, eine Azabenzosilolgruppe, eine Azabenzothiophengruppe, eine Azabenzofurangruppe, eine Azabenzoselenophengruppe, eine Azacarbazolgruppe, eine Azafluorengruppe, eine Azadibenzosilolgruppe, eine Azadibenzothiophengruppe, eine Azadibenzofurangruppe oder eine Azadibenzoselenophengruppe ist.

5. Organometallische Verbindung nach einem der Ansprüche 1-4, wobei R₁, R₁₁, R₁₂, R₂, R₂₉ₐ, R_{29b} und Z₁ jeweils unabhängig wie folgt sind:
Wasserstoff, Deuterium, -F oder eine Cyanogruppe,
eine C₁-C₂₀-Alkylgruppe, eine C₃-C₁₀-Cycloalkylgruppe, eine Phenylgruppe, eine Naphthylgruppe, eine Pyridinylgruppe, eine Furanylgruppe, eine Thiophenylgruppe, eine Benzofuranylgruppe, eine Benzothiophenylgruppe, eine Dibenzofuranylgruppe oder eine Dibenzothiophenylgruppe, jeweils unsubstituiert oder substituiert mit zumindest einem von Deuterium, -F, einer Cyanogruppe, einer C₁-C₂₀-Alkylgruppe, einer deuterierten C₁-C₂₀-Alkylgruppe, einer fluorierten C₁-C₂₀-Alkylgruppe, einer C₃-C₁₀-Cycloalkylgruppe, einer deuterierten C₃-C₁₀-Cycloalkylgruppe, einer fluorierten C₃-C₁₀-Cycloalkylgruppe, einer (C₁-C₂₀-Alkyl)C₃-C₁₀-Cycloalkylgruppe, einer Phenylgruppe, einer deuterierten Phenylgruppe, einer fluorierten Phenylgruppe, einer (C₁-C₂₀-Alkyl)phenylgruppe, einer Naphthylgruppe, einer Pyridinylgruppe, einer Furanylgruppe, einer Thiophenylgruppe, einer Benzofuranylgruppe, einer Benzothiophenylgruppe, einer Dibenzofuranylgruppe, einer Dibenzothiophenylgruppe, - Si(Q₃₃)(Q₃₄)(Q₃₅), -Ge(Q₃₃)(Q₃₄)(Q₃₅) oder einer Kombination davon, oder
-Si(Q₃)(Q₄)(Q₅) oder -Ge(Q₃)(Q₄)(Q₅).

6. Organometallische Verbindung nach einem der Ansprüche 1-5, wobei a2 nicht 0 ist und R₂ nicht Wasserstoff ist, und/oder
umfassend Deuterium, eine Fluorgruppe, eine deuterierte C₁-C₂₀-Alkylgruppe, eine fluorierte C₁-C₂₀-Alkylgruppe, -Si(Q₃)(Q₄)(Q₅), -Ge(Q₃)(Q₄)(Q₅) oder eine Kombination davon.

7. Organometallische Verbindung nach einem der Ansprüche 1-6, wobei
X₁ N-{(T₁)_{b1}-(Z₁)_{c1}} ist, und
X₁ zumindest eine von Bedingung A und Bedingung B erfüllt, oder X₁ Bedingung C erfüllt:
Bedingung A
T₁ ist eine carbocyclische C₅-C₃₀-Gruppe, die unsubstituiert oder substituiert mit zumindest einem R₁₀ₐ ist, oder eine heterocyclische C₁-C₃₀-Gruppe, die unsubstituiert oder substituiert mit zumindest einem R₁₀ₐ ist,
Bedingung B
Z₁ ist eine substituierte oder unsubstituierte C₆-C₆₀-Arylgruppe, eine substituierte oder unsubstituierte C₁-C₆₀-Heteroarylgruppe, eine substituierte oder unsubstituierte einwertige nichtaromatische kondensierte polycyclische Gruppe oder eine substituierte oder unsubstituierte einwertige nichtaromatische kondensierte heteropolycyclische Gruppe,
Bedingung C
T₁ ist eine Einfachbindung oder eine C₁-C₂₀-Alkylengruppe, die unsubstituiert oder substituiert mit zumindest einem R₁₀ₐ ist, und
Z₁ und R₁₀ₐ sind jeweils unabhängig Wasserstoff, Deuterium, -F, eine Cyanogruppe, eine C₁-C₂₀-Alkylgruppe, eine deuterierte C₁-C₂₀-Alkylgruppe, eine fluorierte C₁-C₂₀-Alkylgruppe, - Si(Q₃₃)(Q₃₄)(Q₃₅), -Ge(Q₃₃)(Q₃₄)(Q₃₅) oder eine Kombination davon.

8. Organometallische Verbindung nach einem der Ansprüche 1-7, wobei die organometallische Verbindung zumindest eine von Bedingung 1 bis Bedingung 3 erfüllt:
Bedingung 1
a1 ist nicht 0, und
R₁ umfasst zumindest eines von Deuterium, einer Fluorgruppe, einer Cyanogruppe, einer deuterierten C₁-C₂₀-Alkylgruppe, einer fluorierten C₁-C₂₀-Alkylgruppe, einer Phenylgruppe, einer deuterierten Phenylgruppe, einer fluorierten Phenylgruppe, einer (C₁-C₂₀-Alkyl)phenylgruppe, - Si(Q₃)(Q₄)(Q₅), -Ge(Q₃)(Q₄)(Q₅) oder einer Kombination davon,
Bedingung 2
a2 ist nicht 0, und
R₂ umfasst zumindest eines von Deuterium, einer Fluorgruppe, einer Cyanogruppe, einer deuterierten C₁-C₂₀-Alkylgruppe, einer fluorierten C₁-C₂₀-Alkylgruppe, einer Phenylgruppe, einer deuterierten Phenylgruppe, einer fluorierten Phenylgruppe, einer (C₁-C₂₀-Alkyl)phenylgruppe, - Si(Q₃)(Q₄)(Q₅), -Ge(Q₃)(Q₄)(Q₅) oder einer Kombination davon,
Bedingung 3
X₁ ist N-{(T₁)_{b1}-(Z₁)_{c1}}, und
eine Gruppe dargestellt durch *-(T₁)_{b1}-(Z₁)_{c1} umfasst zumindest eines von Deuterium, einer Fluorgruppe, einer Cyanogruppe, einer deuterierten C₁-C₂₀-Alkylgruppe, einer fluorierten C₁-C₂₀-Alkylgruppe, einer Phenylgruppe, einer deuterierten Phenylgruppe, einer fluorierten Phenylgruppe, einer (C₁-C₂₀-Alkyl)phenylgruppe, -Si(Q₃)(Q₄)(Q₅), -Ge(Q₃)(Q₄)(Q₅) oder einer Kombination davon.

9. Organometallische Verbindung nach einem der Ansprüche 1-8, wobei
eine Gruppe dargestellt durch 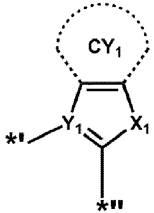
in Formel 2-1 eine Gruppe dargestellt durch eine der Formeln CY1-1 bis CY1-20 ist: 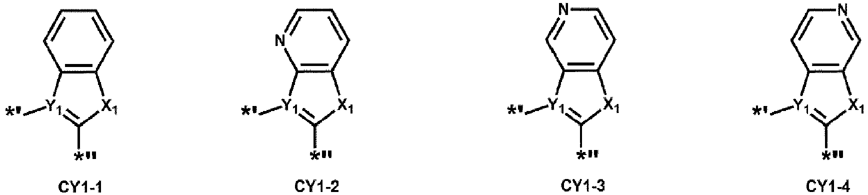 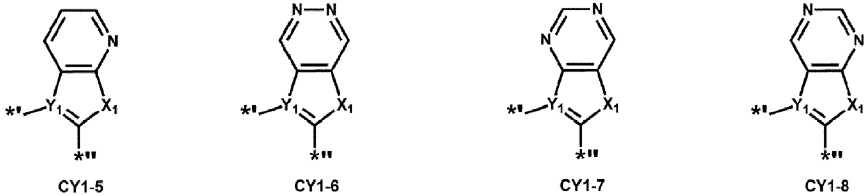 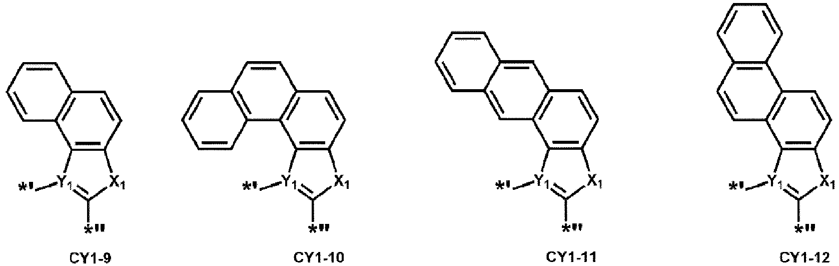 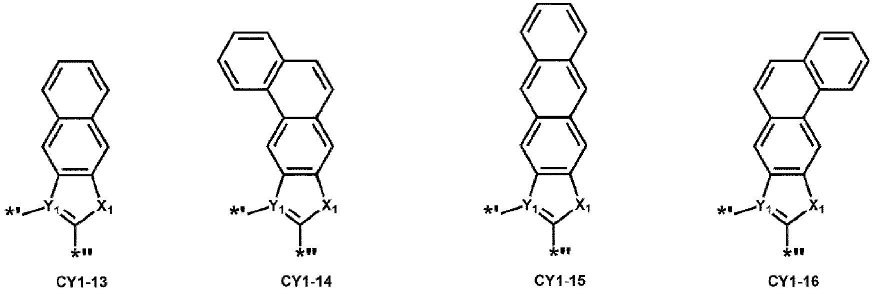 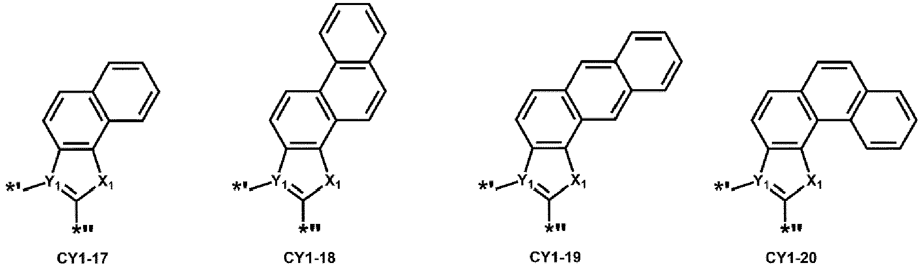
wobei in Formel CY1-1 bis CY1-20,
Y₁ und X₁ jeweils wie in Anspruch 1 beschrieben sind,
*' eine Bindungsstelle zu M in Formel 1 angibt, und
*" eine Bindungsstelle zu einem von A₁ bis A₄ von Formel 2-1 angibt, und/oder
wobei
eine Gruppe dargestellt durch 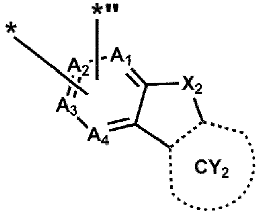
eine Gruppe dargestellt durch eine der Formeln CY2(1) bis CY2(6) ist: 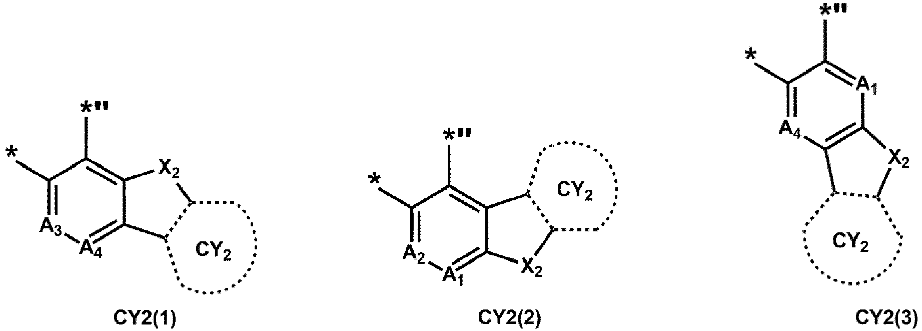 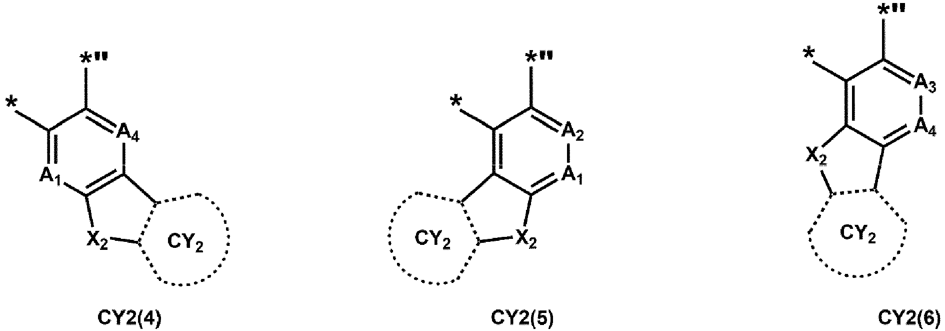
wobei in Formel CY2(1) bis CY2(6),
X₂ und Ring CY₂ wie in Anspruch 1 beschrieben sind,
A₁ bis A₄ jeweils unabhängig C oder N sind,
*" eine Bindungsstelle zu einem benachbarten 5-gliedrigen Ring ist, und
* eine Bindungsstelle zu M in Formel 1 ist.

10. Organometallische Verbindung nach einem der Ansprüche 1-9, wobei eine Gruppe dargestellt durch 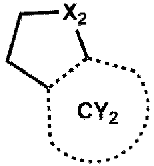
eine Gruppe dargestellt durch eine der Formeln CY2-1 bis CY2-20 ist: 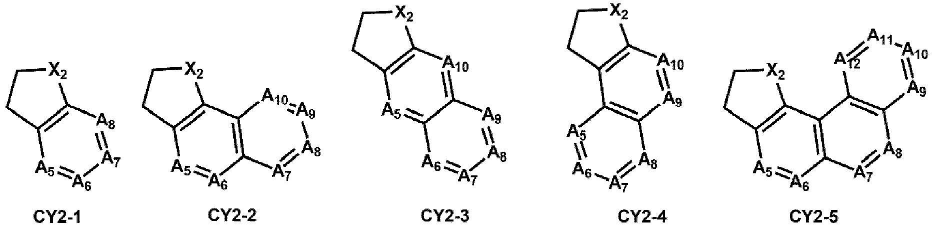 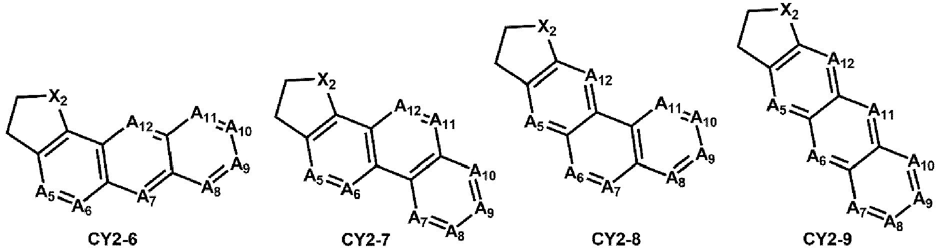 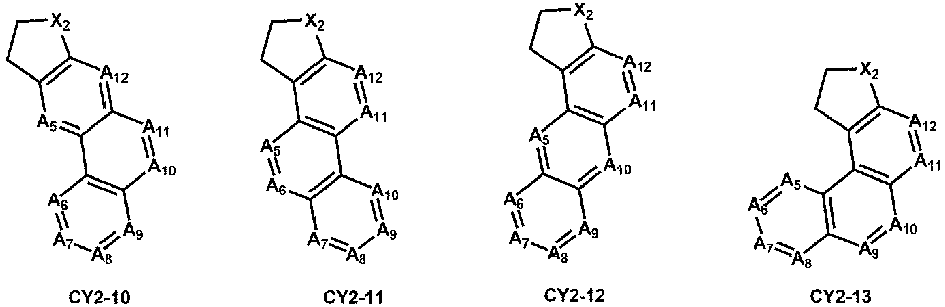 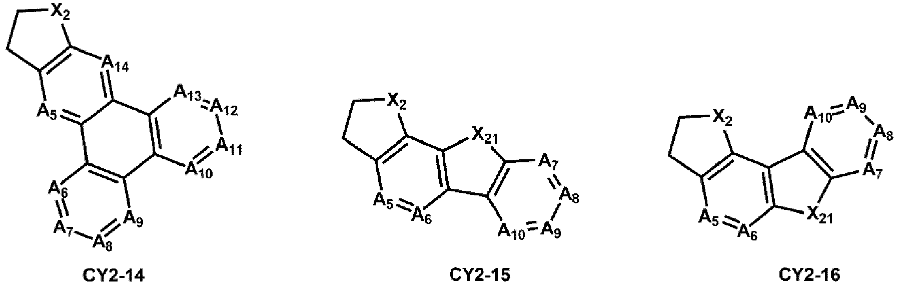 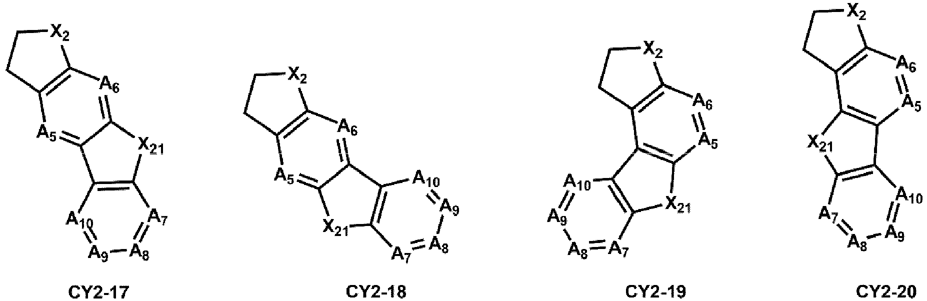
wobei in den Formeln CY2-1 bis CY2-20,
X₂ wie in Anspruch 1 beschrieben ist,
X₂₁ O, S, Se, N(R_{29c}), C(R_{29c})(R_{29d}), Si(R_{29c})(R_{29d}) oder Ge(R_{29c})(R_{29d}) ist,
R_{29c} und R_{29d} jeweils wie in Verbindung mit R₂₉ₐ in Anspruch 1 beschrieben sind, und
A₅ bis A₁₄ jeweils unabhängig C oder N sind.

11. Organometallische Verbindung nach einem der Ansprüche 1-10, wobei eine Gruppe dargestellt durch 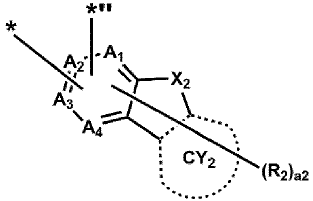
eine Gruppe dargestellt durch eine der Formeln CY2A bis CY2D ist: 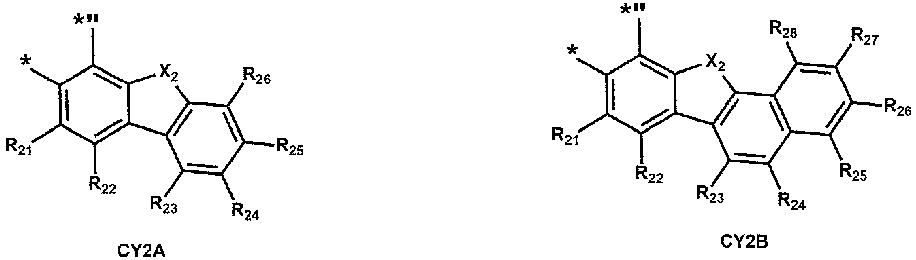 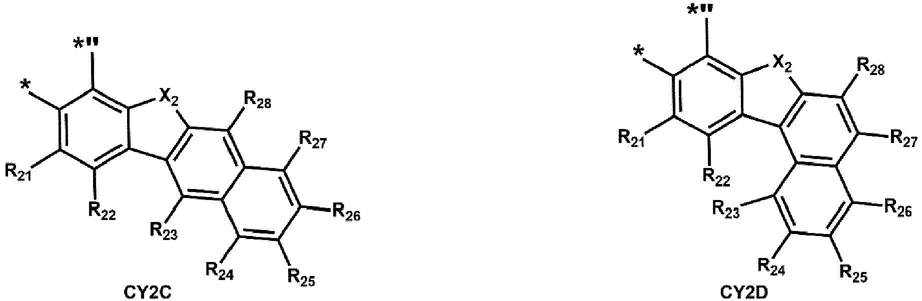
wobei in Formel CY2A bis CY2D,
X₂ wie in Anspruch 1 beschrieben ist,
R₂₁ bis R₂₈ jeweils wie in Verbindung mit R₂ in Anspruch 1 beschrieben sind,
*" eine Bindungsstelle zu einem benachbarten 5-gliedrigen Ring ist, und
* eine Bindungsstelle zu M in Formel 1 ist.

12. Organische lichtemittierende Vorrichtung, umfassend:
eine erste Elektrode,
eine zweite Elektrode, und
eine organische Schicht, die sich zwischen der ersten Elektrode und der zweiten Elektrode befindet,
wobei die organische Schicht eine Emissionsschicht umfasst, und
wobei die organische Schicht ferner zumindest eine von der organometallischen Verbindung nach einem der Ansprüche 1-11 umfasst;
wobei bevorzugt
die erste Elektrode eine Anode ist,
die zweite Elektrode eine Kathode ist,
die organische Schicht ferner eine Lochtransportregion, die sich zwischen der ersten Elektrode und der Emissionsschicht befindet, und eine Elektronentransportregion, die sich zwischen der Emissionsschicht und der zweiten Elektrode befindet, umfasst, wobei
die Lochtransportregion eine Lochinjektionsschicht, eine Lochtransportschicht, eine Elektronenblockierschicht, eine Pufferschicht oder eine Kombination davon umfasst, und
die Elektronentransportregion eine Lochblockierschicht, eine Elektronentransportschicht, eine Elektroneninjektionsschicht oder eine Kombination davon umfasst.

13. Organische lichtemittierende Vorrichtung nach Anspruch 12, wobei die Emissionsschicht die zumindest eine organometallische Verbindung umfasst;
wobei bevorzugt die Emissionsschicht ein grünes Licht emittiert.

14. Organische lichtemittierende Vorrichtung nach Anspruch 13, wobei
die Emissionsschicht ferner einen Wirt umfasst, und
eine Menge des Wirts in der Emissionsschicht größer als eine Menge der organometallischen Verbindung in der Emissionsschicht ist.

15. Elektronische Einrichtung, umfassend die organische lichtemittierende Vorrichtung nach einem der Ansprüche 12-14.

## Revendications

1. Composé organométallique représenté par la Formule 1 :
Formule 1 M(L₁)₃
dans lequel, dans la formule 1,
M est l'iridium, et
L₁ est un ligand représenté par la Formule 2-1 ou un ligand représenté par la Formule 2-2 : 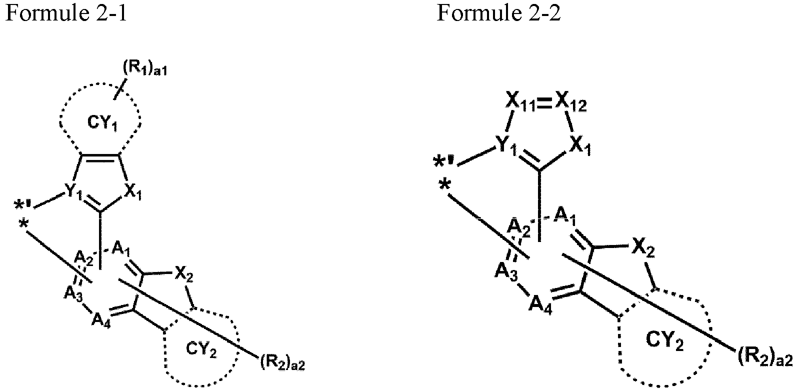
dans lequel, dans les formules 2-1 et 2-2,
Y₁ est N,
X₁₁ est C(R₁₁) ou N,
X₁₂ est C(R₁₂) ou N,
X₁ est O, S, ou N-{(T₁)_{b1}-(Z₁)_{c1}},
T₁ est une liaison simple, un groupe alkylène en C₁ à C₂₀ non substitué ou substitué par au moins un R₁₀ₐ, un groupe carbocyclique en C₅ à C₃₀ non substitué ou substitué par au moins un R₁₀ₐ, ou un groupe hétérocyclique en C₁ à C₃₀ non substitué ou substitué par au moins un R₁₀ₐ,
b1 est un nombre entier de 1 à 5,
c1 est un nombre entier de 0 à 20,
A₁ à A₄ sont chacun indépendamment C ou N, où l'un de A₁ à A₄ est C lié à un cycle adjacent à 5 chaînons, et un autre de A₁ à A₄ est C lié à M dans la formule 1,
X₂ est O, S, Se, Si(R₂₉ₐ)(R_{29b}), ou Ge(R₂₉ₐ)(R_{29b}),
le cycle CY₁ et le cycle CY₂ sont chacun indépendamment un groupe carbocyclique en C₅ à C₃₀ ou un groupe hétérocyclique en C₁ à C₃₀,
R₁, R₁₁, R₁₂, R₂, R₂₉ₐ, R_{29b}, et Z₁ sont chacun indépendamment : hydrogène, deutérium, -F, -Cl, -Br, -I, -SF₅, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀ substitué ou non substitué, un groupe alcényle en C₂ à C₆₀ substitué ou non substitué, un groupe alcynyle en C₂ à C₆₀ substitué ou non substitué, un groupe alcoxy en C₁ à C₆₀ substitué ou non substitué, un groupe alkylthio en C₁ à C₆₀ substitué ou non substitué, un groupe cycloalkyle en C₃ à C₁₀ substitué ou non substitué, un groupe hétérocycloalkyle en C₁ à C₁₀ substitué ou non substitué, un groupe cycloalcényle en C₃ à C₁₀ substitué ou non substitué, un groupe hétérocycloalcényle en C₁ à C₁₀ substitué ou non substitué, un groupe aryle en C₆ à C₆₀ substitué ou non substitué, un groupe alkylaryle en C₇ à C₆₀ substitué ou non substitué, un groupe arylalkyl en C₇ à C₆₀ substitué ou non substitué, un groupe aryloxy en C₆ à C₆₀ substitué ou non substitué, un groupe arylthio en C₆ à C₆₀ substitué ou non substitué, un groupe hétéroaryle en C₁ à C₆₀ substitué ou non substitué, un groupe alkylhétéroaryl en C₂ à C₆₀ substitué ou non substitué, un groupe hétéroarylalkyle en C₂ à C₆₀ substitué ou non substitué, un groupe hétéroaryloxy en C₁ à C₆₀ substitué ou non substitué, un groupe hétéroarylthio en C₁ à C₆₀ substitué ou non substitué, un groupe polycyclique condensé non-aromatique monovalent substitué ou non substitué, un groupe hétéropolycyclique condensé non-aromatique monovalent substitué ou non substitué, -N(Q₁)(Q₂), -Si(Q₃)(Q₄)(Q₅), -Ge(Q₃)(Q₄)(Q₅), -B(Q₆)(Q₇), - P(=O)(Q₈)(Q₉), ou -P(Q₈)(Q₉),
a1 et a2 sont chacun indépendamment un nombre entier de 0 à 20,
deux ou plus d'une pluralité de R₁ sont éventuellement liés l'un à l'autre pour former un groupe carbocyclique en C₅ à C₃₀ substitué ou non substitué par au moins un R₁₀ₐ ou un groupe hétérocyclique en C₁ à C₃₀ substitué ou non substitué par au moins un R₁₀ₐ,
deux ou plus d'une pluralité de R₂ sont éventuellement liés l'un à l'autre pour former un groupe carbocyclique en C₅ à C₃₀ substitué ou non substitué par au moins un R₁₀ₐ ou un groupe hétérocyclique en C₁ à C₃₀ substitué ou non substitué par au moins un R₁₀ₐ,
R₁ et R₂ sont éventuellement liés l'un à l'autre pour former un groupe carbocyclique en C₅ à C₃₀ substitué ou non substitué par au moins un R₁₀ₐ ou un groupe hétérocyclique en C₁ à C₃₀ substitué ou non substitué par au moins un R₁₀ₐ,
R₁₀ₐ est le même que celui décrit en relation avec R₂,
* et *' indiquent chacun un site de liaison à M dans la Formule 1, et
au moins un substituant du groupe alkyle en C₁ à C₆₀ substitué, du groupe alcényle en C₂ à C₆₀ substitué, du groupe alcynyle en C₂ à C₆₀ substitué, du groupe alkoxy en C₁ à C₆₀ substitué, du groupe alkylthio en C₁ à C₆₀ substitué, du groupe cycloalkyle en C₃ à C₁₀ substitué, du groupe hétérocycloalkyle en C₁ à C₁₀ substitué, du groupe cycloalcényle en C₃ à C₁₀ substitué, du groupe hétérocycloalcényle en C₁ à C₁₀ substitué, du groupe aryle en C₆ à C₆₀ substitué, du groupe alkylaryle en C₇ à C₆₀ substitué, du groupe arylalkyle en C₇ à C₆₀ substitué, du groupe aryloxy en C₆ à C₆₀ substitué, du groupe arylthio en C₆ à C₆₀ substitué, du groupe hétéroaryle en C₁ à C₆₀ substitué, du groupe alkylhétéroaryle en C₂ à C₆₀ substitué, du groupe hétéroarylalkyle en C₂ à C₆₀ substitué, du groupe hétéroaryloxy en C₁ à C₆₀ substitué, du groupe hétéroarylthio C₁ à C₆₀ substitué, du groupe polycyclique condensé non aromatique monovalent substitué et du groupe hétéropolycyclique condensé non aromatique monovalent substitué est :
deutérium, -F, -Cl, -Br, -I, -SF₅, -CD₃, -CD₂H, -CDH₂, -CF₃, -CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀, un groupe alcényle en C₂ à C₆₀, un groupe alcynyle en C₂ à C₆₀, un groupe alcoxy en C₁ à C₆₀ ou un groupe alkylthio en C₁ à C₆₀ ;
un groupe alkyle en C₁ à C₆₀, un groupe alcényle en C₂ à C₆₀, un groupe alcynyle en C₂ à C₆₀, un groupe alcoxy en C₁ à C₆₀, ou un groupe alkylthio en C₁ à C₆₀, chacun substitué par au moins l'un choisi parmi le deutérium -F, -Cl, -Br, -I, -SF₅, -CD₃, -CD₂H, -CDH₂, -CF₃, -CF₂H, - CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe alkylaryle en C₇ à C₆₀, un groupe aryloxy en C₆ à C₆₀, un groupe arylthio en C₆ à C₆₀, un groupe hétéroaryle en C₁ à C₆₀, un groupe alkylhétéroaryle en C₂ à C₆₀, un groupe hétéroaryloxy C₁ à C₆₀, un groupe hétéroarylthio en C₁ à C₆₀, un groupe polycyclique condensé non aromatique monovalent, un groupe hétéropolycyclique condensé non aromatique monovalent, -N(Q₁₁)(Q₁₂), - Si(Q₁₃)(Q₁₄)(Q₁₅), -Ge(Q₁₃)(Q₁₄)(Q₁₅), -B(Q₁₆)(Q₁₇), -P(=O)(Q₁₈)(Q₁₉), -P(Q₁₈)(Q₁₉), ou une combinaison de ceux-ci ;
un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe alkylaryle en C₇ à C₆₀, un groupe aryloxy en C₆ à C₆₀, un groupe arylthio en C₆ à C₆₀, un groupe hétéroaryle en C₁ à C₆₀, un groupe alkylhétéroaryle en C_{2 à} C₆₀, un groupe hétéroaryloxy en C₁ à C₆₀, un groupe hétéroarylthio en C₁ à C₆₀, un groupe polycyclique condensé non aromatique monovalent, ou un groupe hétéropolycyclique condensé non aromatique monovalent, chacun -non-substitué ou substitué par au moins l'un choisi parmi le deutérium, -F, -Cl, -Br, -I, - SF₅, -CD₃, -CD₂H, -CDH₂, -CF₃, -CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀, un groupe alcényle en C₂ à C₆₀, un groupe alcynyle en C₂ à C₆₀, un groupe alcoxy en C₁ à C₆₀, un groupe alkylthio en C₁ à C₆₀, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe alkylaryle en C₇ à C₆₀, un groupe arylalkyle en C₇ à C₆₀, un groupe aryloxy en C₆ à C₆₀, un groupe arylthio en C₆ à C₆₀, un groupe hétéroaryle en C₁ à C₆₀, un groupe alkylhétéroaryle en C₂ à C₆₀, un groupe hétéroarylalkyle en C₂ à C₆₀, un groupe hétéroaryloxy C₁ à C₆₀, un groupe hétéroarylthio en C₁ à C₆₀, un groupe polycyclique condensé non aromatique monovalent, un groupe hétéropolycyclique condensé non aromatique monovalent, -N(Q₂₁)(Q₂₂), - Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉), P(Q₂₈)(Q₂₉), ou une combinaison de ceux-ci ;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), -P(=O)(Q₃₈)(Q₃₉), ou -P(Q₃₈)(Q₃₉) ; ou
une combinaison de ceux-ci,
dans lequel Q₁ à Q₉, Q₁₁ à Q₁₉, Q₂₁ à Q₂₉, et Q₃₁ à Q₃₉ sont chacun indépendamment hydrogène, deutérium, -F, -Cl, -Br, -I, -SF₅, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀ substitué ou non substitué, un groupe alcényle en C₂ à C₆₀ substitué ou non substitué, un groupe alcynyle en C₂ à C₆₀ substitué ou non substitué, un groupe alcoxy en C₁ à C₆₀ substitué ou non substitué, un groupe alkylthio en C₁ à C₆₀ substitué ou non substitué, un groupe cycloalkyle en C₃ à C₁₀ substitué ou non substitué, un groupe hétérocycloalkyle en C₁ à C₁₀ substitué ou non substitué, un groupe cycloalcényle en C₃ à C₁₀ substitué ou non substitué, un groupe hétérocycloalcényle en C₁ à C₁₀ substitué ou non substitué, un groupe aryle en C₆ à C₆₀ substitué ou non substitué, un groupe alkylaryle en C₇ à C₆₀ substitué ou non substitué, un groupe arylalkyle en C₇ à C₆₀ substitué ou non substitué, un groupe aryloxy en C₆ à C₆₀ substitué ou non substitué, un groupe arylthio en C₆ à C₆₀ substitué ou non substitué, un groupe hétéroaryle en C₁ à C₆₀ substitué ou non substitué, un groupe alkylhétéroaryl en C₂ à C₆₀ substitué ou non substitué, un groupe hétéroarylalkyle en C₂ à C₆₀ substitué ou non substitué, un groupe hétéroaryloxy en C₁ à C₆₀ substitué ou non substitué, un groupe hétéroarylthio en C₁ à C₆₀ substitué ou non substitué, un groupe polycyclique condensé non-aromatique monovalent substitué ou non substitué, un groupe hétéropolycyclique condensé non-aromatique monovalent substitué ou non substitué.

2. Composé organométallique selon la revendication 1, dans lequel
X₁ est N-{(T₁)b₁-(Z₁)_{c1}}, et
T₁ est une liaison simple, un groupe alkylène en C₁ à C₁₀ non substitué ou substitué par au moins un R₁₀ₐ, un groupe carbocyclique en C₅ à C₂₀ non substitué ou substitué par au moins un R₁₀ₐ, ou un groupe hétérocyclique en C₁ à C₂₀ non substitué ou substitué par au moins un R₁₀ₐ.

3. Composé organométallique selon la revendication 1 ou 2, dans lequel
A₁ est C lié à un cycle adjacent à 5 chaînons, et A₂ est C lié à M dans la formule 1 ; ou
A₃ est C lié à un cycle adjacent à 5 chaînons, et A₂ est C lié à M dans la formule 1.

4. Composé organométallique selon l'une quelconque des revendications 1 à 3, dans lequel
le cycle CY₁ est un groupe benzène, un groupe naphtalène, un groupe anthracène, un groupe phénanthrène, un groupe pyridine, un groupe pyrimidine, un groupe pyridazine, un groupe pyrazine, un groupe quinoléine, un groupe isoquinoléine, un groupe quinoxaline, un groupe quinazoline, un groupe phénanthridine, un groupe phénanthroline, un groupe benzoquinoléine ou un groupe benzoisoquinoléine ; et/ou
dans lequel le cycle CY₂ est un groupe benzène, un groupe naphtalène, un groupe anthracène, un groupe phénanthrène, un groupe pyridine, un groupe pyrimidine, un groupe pyridazine, un groupe pyrazine, un groupe quinoléine, un groupe isoquinoléine, un groupe quinoxaline, un groupe quinazoline, un groupe phénanthridine, un groupe phénanthroline, un groupe benzoquinoléine, un groupe benzoisoquinoléine, un groupe benzène auquel un groupe cyclohexane est condensé, un groupe benzène auquel un groupe adamantane est condensé, un groupe benzène auquel un groupe norbornane est condensé, un groupe pyridine auquel un groupe cyclohexane est condensé, un groupe pyridine auquel un groupe adamantane est condensé, un groupe pyridine auquel un groupe norbornane est condensé, un groupe pyrrole, un groupe cyclopentadiène, un groupe silole, un groupe thiophène, un groupe furane, un groupe sélénophène, un groupe indole, un groupe indène, un groupe benzosilole, un groupe benzothiophène, un groupe benzofurane, un groupe benzosélénophène, un groupe carbazole, un groupe fluorène, un groupe dibenzosilole, un groupe dibenzothiophène, un groupe dibenzofurane, un groupe dibenzosélénophène, un groupe azaindole, un groupe azaindène, un groupe azabenzosilole, un groupe azabenzothiophène, un groupe azabenzofurane, un groupe azabenzosélénophène, un groupe azacarbazole, un groupe azafluorène, un groupe azadibenzosilole, un groupe azadibenzothiophène, un groupe azadibenzofurane ou un groupe azadibenzosélénophène.

5. Composé organométallique selon l'une quelconque des revendications 1 à 4, dans lequel R₁, R₁₁, R₁₂, R₂, R₂₉ₐ, R_{29b}, et Z₁ sont chacun indépendamment :
hydrogène, deutérium, -F ou un groupe cyano ;
un groupe alkyle en C₁ à C₂₀, un groupe cycloalkyle en C₃ à C₁₀, un groupe phényle, un groupe naphtyle, un groupe pyridinyle, un groupe furanyle, un groupe thiophényle, un groupe benzofuranyle, un groupe benzothiophényle, un groupe dibenzofuranyle ou un groupe dibenzothiophényle, chacun non substitué ou substitué par au moins un des éléments suivants : deutérium, -F, un groupe cyano, un groupe alkyle en en C₁ à C₂₀, un groupe alkyle en en C₁ à C₂₀ deutéré, un groupe alkyle en en C₁ à C₂₀ fluoré, un groupe cycloalkyle en en C₃ à C₁₀, un groupe cycloalkyle en C₃ à C₁₀ deutéré, un groupe cycloalkyle en C₃ à C₁₀ fluoré, un groupe (alkyle en C₁ à C₂₀)cycloalkyle en C₃ à C₁₀, un groupe phényle, un groupe phényle deutéré, un groupe phényle fluoré, un groupe (alkyle en C₁ à C₂₀)phényle, un groupe naphtyle, un groupe pyridinyle, un groupe furanyle, un groupe thiophényle, un groupe benzofuranyle, un groupe benzothiophényle, un groupe dibenzofuranyle, un groupe dibenzothiophényle, -Si(Q₃₃)(Q₃₄)(Q₃₅), -Ge(Q₃₃)(Q₃₄)(Q₃₅) ou une combinaison de ceux-ci ; ou
-Si(Q₃)(Q₄)(Q₅), or -Ge(Q₃)(Q₄)(Q₅).

6. Composé organométallique selon l'une quelconque des revendications 1 à 5, dans lequel a2 n'est pas 0 et R₂ n'est pas un hydrogène ; et/ou
comprenant du deutérium, un groupe fluoro, un groupe alkyle en C₁ à C₂₀ deutéré, un groupe alkyle en C₁ à C₂₀ fluoré, -Si(Q₃)(Q₄)(Q₅), -Ge(Q₃)(Q₄)(Q₅), ou une combinaison de ceux-ci.

7. Composé organométallique selon l'une quelconque des revendications 1 à 6, dans lequel
X₁ est N-{(T₁)_{b1}-(Z₁)_{c1}}, et
dans lequel X₁ satisfait au moins l'une de la Condition A et la Condition B, ou X₁ satisfait la Condition C :
Condition A
T₁ est un groupe carbocyclique en C₅ à C₃₀ non substitué ou substitué par au moins un R₁₀ₐ ou un groupe hétérocyclique en C₁ à C₃₀ non substitué ou substitué par au moins un R₁₀ₐ,
Condition B
Z₁ est un groupe aryle en C₆ à C₆₀ substitué ou non substitué, un groupe hétéroaryle en C₁ à C₆₀ substitué ou non substitué, un groupe polycyclique condensé non aromatique monovalent substitué ou non substitué, ou un groupe hétéropolycyclique condensé non aromatique monovalent substitué ou non substitué ;
Condition C
T₁ est une liaison simple ou un groupe alkylène en C₁ à C₂₀ qui est non substitué ou substitué par au moins un R₁₀ₐ, et
Z₁ et R₁₀ₐ sont chacun indépendamment hydrogène, deutérium, -F, un groupe cyano, un groupe alkyle en C₁ à C₂₀, un groupe alkyle en C₁ à C₂₀ deutéré, un groupe alkyle en C₁ à C₂₀ fluoré, -Si(Q₃₃)(Q₃₄)(Q₃₅), -Ge(Q₃₃)(Q₃₄)(Q₃₅) ou une combinaison de ceux-ci.

8. Composé organométallique selon l'une quelconque des revendications 1 à 7, dans lequel le composé organométallique satisfait au moins l'une de la Condition 1 à la Condition 3 :
Condition 1
a1 n'est pas 0, et
R₁ comprend au moins l'un parmi deutérium, un groupe fluoro, un groupe cyano, un groupe alkyle en C₁ à C₂₀ deutéré, un groupe alkyle en C₁ à C₂₀ fluoré, un groupe phényle, un groupe phényle deutéré, un groupe phényle fluoré, un groupe (alkyle en C₁ à C₂₀)phényle, - Si(Q₃)(Q₄)(Q₅), -Ge(Q₃)(Q₄)(Q₅), ou une combinaison de ceux-ci ;
Condition 2
a2 n'est pas 0, et
R₂ comprend au moins l'un parmi deutérium, un groupe fluoro, un groupe cyano, un groupe alkyle en C₁ à C₂₀ deutéré, un groupe alkyle en C₁ à C₂₀ fluoré, un groupe phényle, un groupe phényle deutéré, un groupe phényle fluoré, un groupe (alkyle en C₁ à C₂₀)phényle, - Si(Q₃)(Q₄)(Q₅), -Ge(Q₃)(Q₄)(Q₅), ou une combinaison de ceux-ci ;
Condition 3
X₁ est N-{(T₁)_{b1}-(Z₁)_{c1}}, et
un groupe représenté par *-(T₁)_{b1}-(Z₁)_{c1} comprend au moins un élément parmi le deutérium, un groupe fluoro, un groupe cyano, un groupe alkyle en C₁ à C₂₀ deutéré, un groupe alkyle en C₁ à C₂₀ fluoré, un groupe phényle, un groupe phényle deutéré, un groupe phényle fluoré, un groupe (alkyle en C₁ à C₂₀)phényle, -Si(Q₃)(Q₄)(Q₅), -Ge(Q₃)(Q₄)(Q₅), ou une combinaison de ceux-ci.

9. Composé organométallique selon l'une quelconque des revendications 1 à 8, dans lequel un groupe représenté par 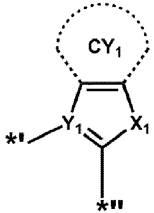
dans la Formule 2-1, est un groupe représenté par l'une des formules CY1-1 à CY1-20 : 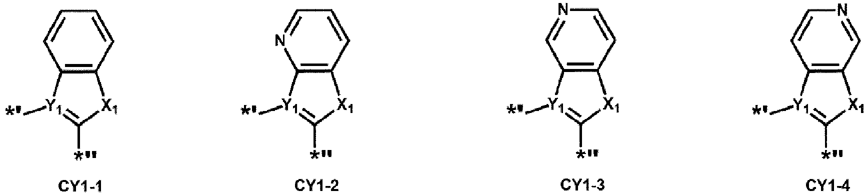 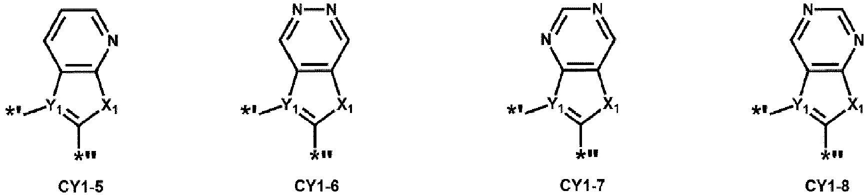 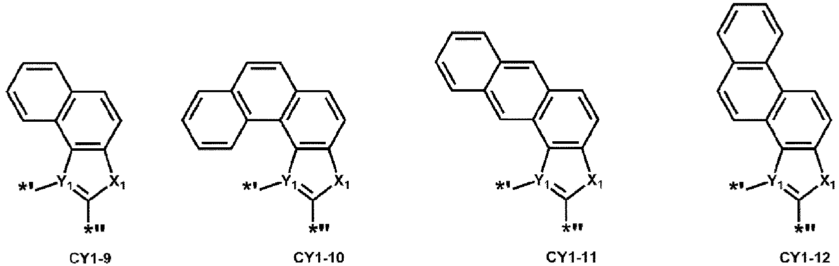 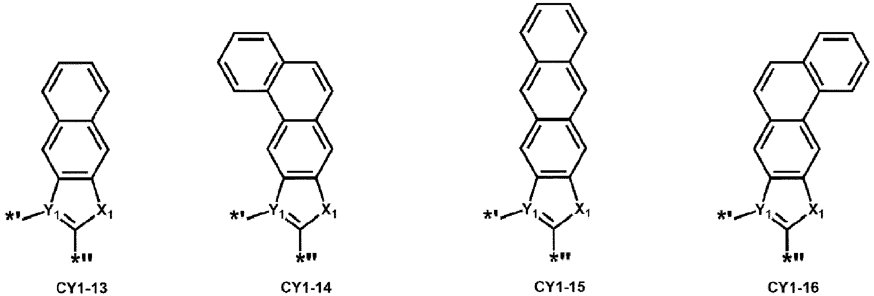 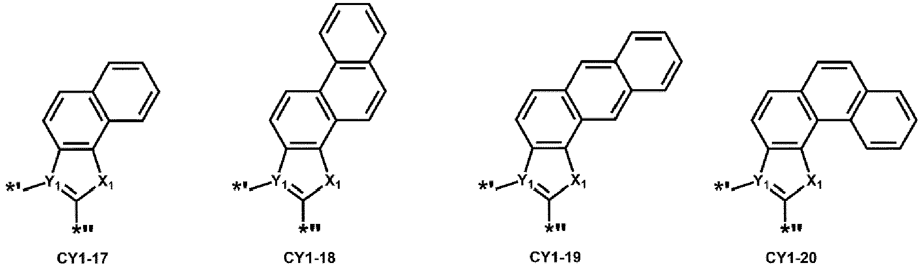
dans lequel dans les formules CY1-1 à CY1-20,
Y₁ et X₁ sont chacun tels que décrits selon la revendication 1,
*' indique un site de liaison à M selon la formule 1, et
*" indique un site de liaison à l'un de A₁ à A₄ de la formule 2-1 ; et/ou dans lequel
un groupe représenté par 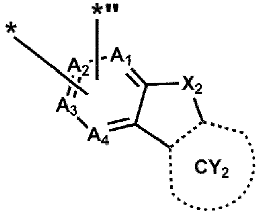
est un groupe représenté par l'une des formules CY2(1) à CY2(6) : 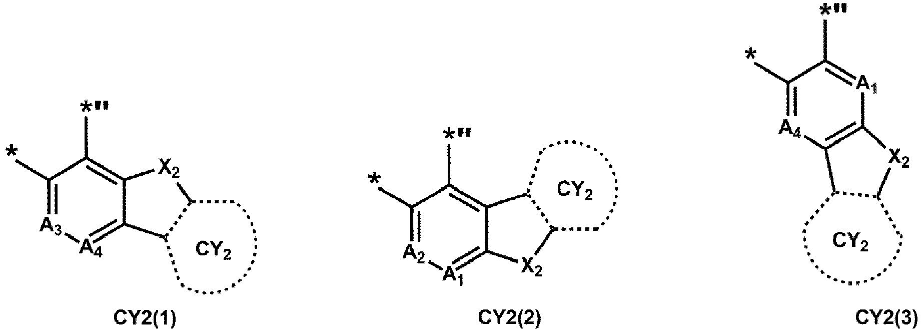 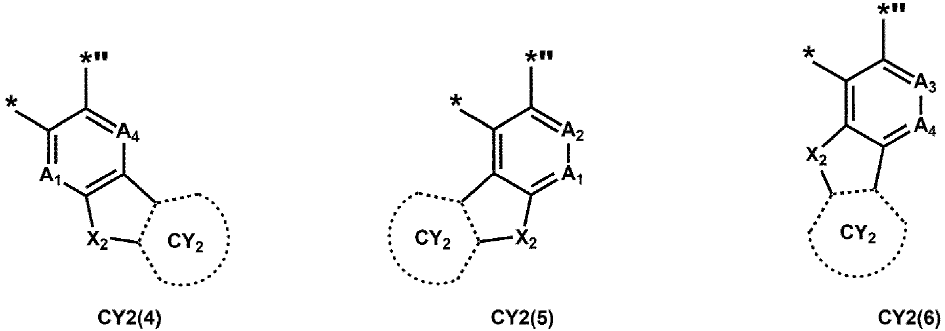
dans lequel, dans les formules CY2(1) à CY2(6),
X₂ et le cycle CY₂ sont tels que décrits selon la revendication 1,
A₁ à A₄ sont chacun indépendamment C ou N,
*" est un site de liaison à un cycle voisin à 5 chaînons, et
* indique un site de liaison à M selon la formule 1.

10. Composé organométallique selon l'une quelconque des revendications 1 à 9, dans lequel un groupe représenté par 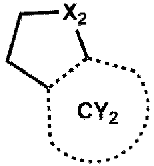
est un groupe représenté par l'une des formules CY2-1 à CY2-20 : 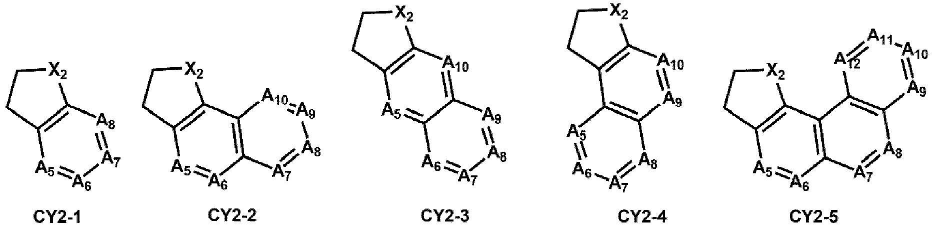 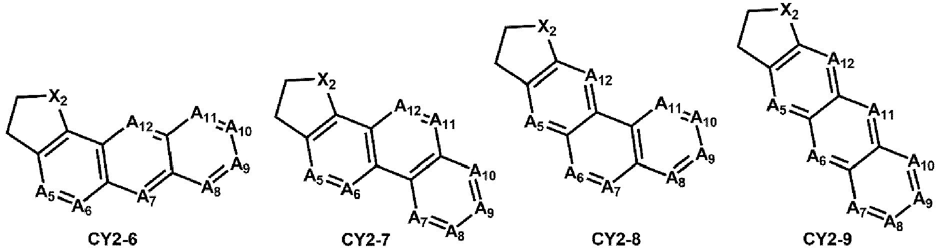 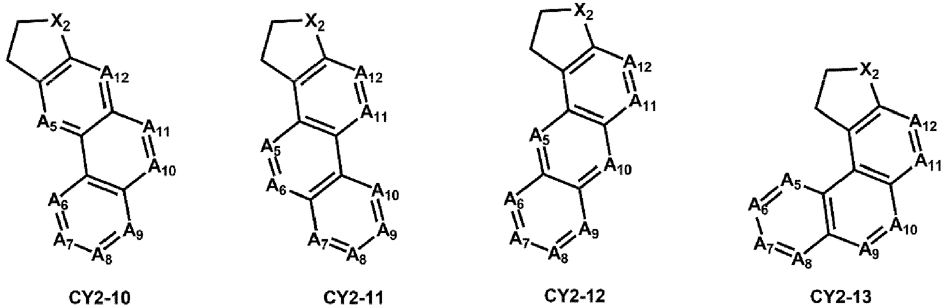 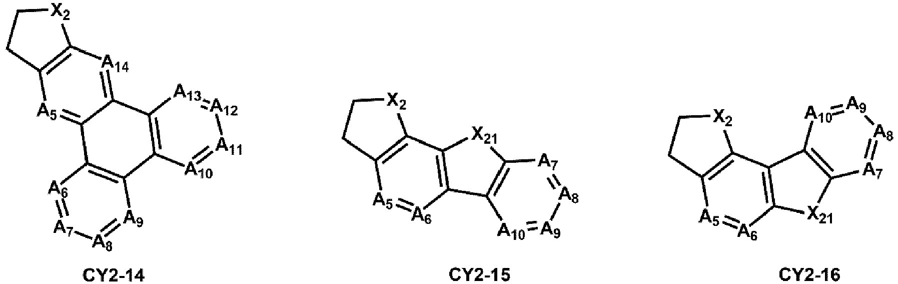 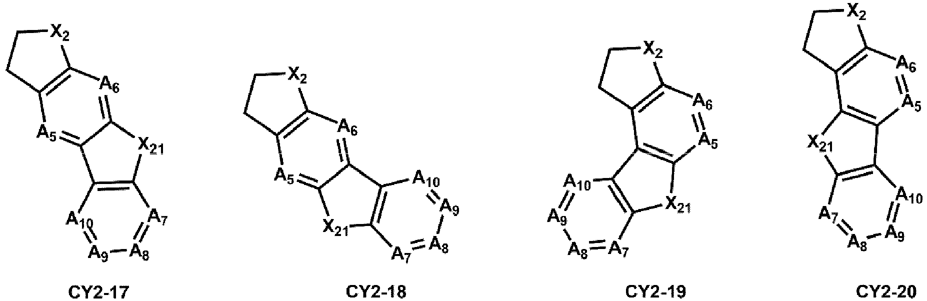
dans lequel, dans les formules CY2-1 à CY2-20,
X₂ est tel que décrit selon la revendication 1,
X₂₁ est O, S, Se, N(R_{29c}), C(R_{29c})(R_{29d}), Si(R_{29c})(R_{29d}), ou Ge(R_{29c})(R_{29d}),
R_{29c} et R_{29d} sont chacun tels que décrits en relation avec R₂₉ₐ selon la revendication 1, et
A₅ à A₁₄ sont chacun indépendamment C ou N.

11. Composé organométallique selon l'une quelconque des revendications 1 à 10, dans lequel un groupe représenté par 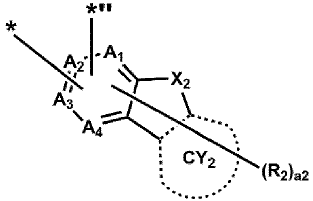
est un groupe représenté par l'une des Formules CY2A à CY2D : 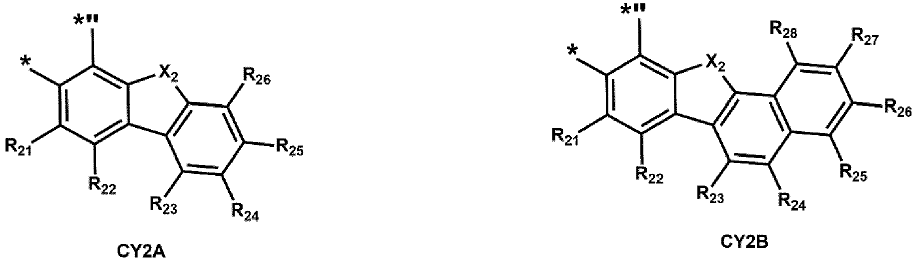 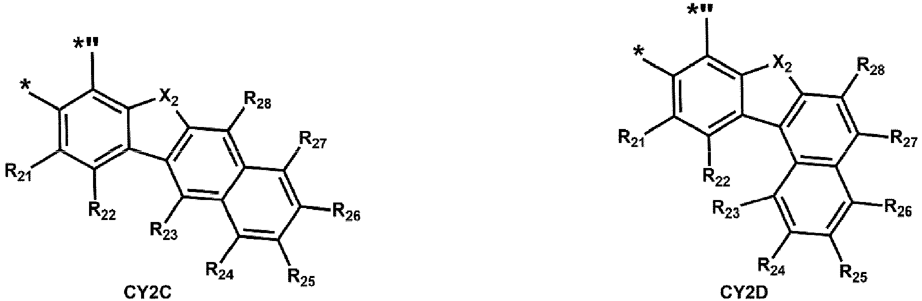
dans lequel dans les formules CY2A à CY2D,
X₂ est tel que décrit selon la revendication 1,
R₂₁ à R₂₈ sont chacun tels que décrits en relation avec R₂ selon la revendication 1,
*" est un site de liaison à un cycle voisin à 5 chaînons, et
* indique un site de liaison à M selon la formule 1.

12. Dispositif électroluminescent organique, comprenant :
une première électrode ;
une seconde électrode ; et
une couche organique disposée entre la première électrode et la seconde électrode,
dans lequel la couche organique comprend une couche d'émission, et
dans lequel la couche organique comprend en outre au moins un composé organométallique selon l'une quelconque des revendications 1 à 11 ;
de préférence dans lequel
la première électrode est une anode,
la seconde électrode est une cathode,
la couche organique comprend en outre une région de transport de trous disposée entre la première électrode et la couche d'émission et une région de transport d'électrons disposée entre la couche d'émission et la seconde électrode, dans lequel
la région de transport de trous comprend une couche d'injection de trous, une couche de transport de trous, une couche de blocage d'électrons, une couche tampon, ou une combinaison de celles-ci, et
la région de transport d'électrons comprend une couche de blocage de trous, une couche de transport d'électrons, une couche d'injection d'électrons ou une combinaison de celles-ci.

13. Dispositif électroluminescent organique selon la revendication 12, dans lequel la couche d'émission comprend l'au moins un composé organométallique,
de préférence dans lequel la couche d'émission émet une lumière verte.

14. Dispositif électroluminescent organique selon la revendication 13, dans lequel :
la couche d'émission comprend en outre un hôte, et
une quantité de l'hôte dans la couche d'émission est supérieure à une quantité du composé organométallique dans la couche d'émission.

15. Appareil électronique, comprenant le dispositif électroluminescent organique selon l'une quelconque des revendications 12 à 14.
